# EUROPEAN PATENT SPECIFICATION

(11) **EP 1 000 146 B1**
(45) Date of publication and mention of the grant of the patent: **30.08.2006**
(21) Application number: 98936586.1
(22) Date of filing: 31.07.1998
(51) Int. Cl.: C12N 15/12, C07K 14/47

(54) **5'ESTs FOR NON TISSUE SPECIFIC SECRETED PROTEINS**
5' ESTS FÜR PROTEINE, DIE NICHT GEWEBESPEZIFISCH SIND
EST 5' POUR PROTEINES SECRETEES SANS SPECIFICITE TISSULAIRE

(30) Priority: 01.08.1997 US 905135
(43) Date of publication of application: 17.05.2000
(73) Proprietor: Serono Genetics Institute S.A., 91030 Evry Cedex (FR)
(72) Inventor: DUMAS MILNE EDWARDS, Jean-Baptiste, F-75006 Paris (FR); DUCLERT, Aymeric, F-94100 Saint-Maur (FR); LACROIX, Bruno, F-69230 Saint-Genis Laval (FR)
(74) Representative: Brasnett, Adrian Hugh
(86) International application number: PCT/IB1998/001222
(87) International publication number: WO 1999/006548

(56) References cited:
- WO-A2-97/07198
- DATABASE EMBL [Online] CpG island; genomic MseI fragment, 18 October 1995 (1995-10-18) CROSS S.H. ET AL.: "Purification of CpG islands using a methylated DNA binding column" Database accession no. Z58035 XP002271717
- DATABASE EMBL [Online] zv93e08.s1 Soares_NhHMPu_S1 H.sapiens cDNA clone, 14 May 1997 (1997-05-14) HILLIER L. ET AL.: "WashU-Merck EST Project 1997" Database accession no. AA418573 XP002271718
- WU Q. AND MANIATIS T.: "A striking organization of a large family of human neural Cadherin-like cell adhesion genes" CELL, vol. 97, 11 June 1999 (1999-06-11), pages 779-790, XP002905101 ISSN: 0092-8674
- YOKOYAMA-KOBAYASHI M. ET AL: 'A signal sequence detection system using secreted protease activity as an indicator' GENE vol. 163, 1995, pages 193 - 196
- TAISHIRO K. ET AL: 'Signal sequence trap: a cloning strategy for secreted proteins and type I membrane proteins' SCIENCE vol. 261, 30 July 1993, pages 600 - 603
- VON HEIJNE G.: 'A new method for predicting signal sequence cleavage sites' NUCLEIC ACIDS RESEARCH vol. 14, no. 11, 1986, pages 4683 - 4690
- WEINER J.A. ET AL: 'Gamma Protocadherins are required for synaptic development in the spinal cord' PROCEEDINGS OF THE NATIONAL ACADEMY OF SCIENCES OF USA vol. 102, no. 1, 04 January 2005, pages 8 - 14

## Description

### Background of the Invention

The estimated 50,000-100,000 genes scattered along the human chromosomes offer tremendous promise for the understanding, diagnosis, and treatment of human diseases. In addition, probes capable of specifically hybridizing to loci distributed throughout the human genome find applications in the construction of high resolution chromosome maps and in the identification of individuals.

In the past, the characterization of even a single human gene was a painstaking process, requiring years of effort. Recent developments in the areas of cloning vectors, DNA sequencing, and computer technology have merged to greatly accelerate the rate at which human genes can be isolated, sequenced, mapped, and characterized. Cloning vectors such as yeast artificial chromosomes (YACs) and bacterial artificial chromosomes (BACs) are able to accept DNA inserts ranging from 300 to 1000 kilobases (kb) or 100-400 kb in length respectively, thereby facilitating the manipulation and ordering of DNA sequences distributed over great distances on the human chromosomes. Automated DNA sequencing machines permit the rapid sequencing of human genes. Bioinformatics software enables the comparison of nucleic acid and protein sequences, thereby assisting in the characterization of human gene products.

Currently, two different approaches are being pursued for identifying and characterizing the genes distributed along the human genome. In one approach, large fragments of genomic DNA are isolated, cloned, and sequenced. Potential open reading frames in these genomic sequences are identified using bioinformatics software. However, this approach entails sequencing large stretches of human DNA which do not encode proteins in order to find the protein encoding sequences scattered throughout the genome. In addition to requiring extensive sequencing, the bioinformatics software may mischaracterize the genomic sequences obtained. Thus, the software may produce false positives in which non-coding DNA is mischaracterized as coding DNA or false negatives in which coding DNA is mislabeled as non-coding DNA.

An alternative approach takes a more direct route to identifying and characterizing human genes. In this approach, complementary DNAs (cDNAs) are synthesized from isolated messenger RNAs (mRNAs) which encode human proteins. Using this approach, sequencing is only performed on DNA which is derived from protein coding portions of the genome. Often, only short stretches of the cDNAs are sequenced to obtain sequences called expressed sequence tags (ESTs). The ESTs may then be used to isolate or purify extended cDNAs which include sequences adjacent to the EST sequences. The extended cDNAs may contain all of the sequence of the EST which was used to obtain them or only a portion of the sequence of the EST which was used to obtain them. In addition, the extended cDNAs may contain the full coding sequence of the gene from which the EST was derived or, alternatively, the extended cDNAs may include portions of the coding sequence of the gene from which the EST was derived. It will be appreciated that there may be several extended cDNAs which include the EST sequence as a result of alternate splicing or the activity of alternative promoters.

In the past, these short EST sequences were often obtained from oligo-dT primed cDNA libraries. Accordingly, they mainly corresponded to the 3' untranslated region of the mRNA. In part, the prevalence of EST sequences derived from the 3' end of the mRNA is a result of the fact that typical techniques for obtaining cDNAs are not well suited for isolating cDNA sequences derived from the 5' ends of mRNAs. (Adams *et al., Nature* 377:3-174, 1996; Hillier *et al., Genome Res.* **6**:807-828, 1996).

In addition, in those reported instances where longer cDNA sequences have been obtained, the reported sequences typically correspond to coding sequences and do not include the full 5' untranslated region of the mRNA from which the cDNA is derived. Such incomplete sequences may not include the first exon of the mRNA, particularly in situations where the first exon is short. Furthermore, they may not include some exons, often short ones, which are located upstream of splicing sites. Thus, there is a need to obtain sequences derived from the 5' ends of mRNAs.

While many sequences derived from human chromosomes have practical applications, approaches based on the identification and characterization of those chromosomal sequences which encode a protein product are particularly relevant to diagnostic and therapeutic uses. Of the 50,000-100,000 protein coding genes, those genes encoding proteins which are secreted from the cell in which they are synthesized, as well as the secreted proteins themselves, are particularly valuable as potential therapeutic agents. Such proteins are often involved in cell to cell communication and may be responsible for producing a clinically relevant response in their target cells.

In fact, several secretory proteins, including tissue plasminogen activator, G-CSF, GM-CSF, erythropoietin, human growth hormone, insulin, interferon-α, interferon-β, interferon-γ, and interleukin-2, are currently in clinical use. These proteins are used to treat a wide range of conditions, including acute myocardial infarction, acute ischemic stroke, anemia, diabetes, growth hormone deficiency, hepatitis, kidney carcinoma, chemotherapy induced neutropenia and multiple sclerosis. For these reasons, extended cDNAs encoding secreted proteins or portions thereof represent a particularly valuable source of therapeutic agents. Thus, there is a need for the identification and characterization of secreted proteins and the nucleic acids encoding them.

In addition to being therapeutically useful themselves, secretory proteins include short peptides, called signal peptides, at their amino termini which direct their secretion. These signal peptides are encoded by the signal sequences located at the 5' ends of the coding sequences of genes encoding secreted proteins. Because these signal peptides will direct the extracellular secretion of any protein to which they are operably linked, the signal sequences may be exploited to direct the efficient secretion of any protein by operably linking the signal sequences to a gene encoding the protein for which secretion is desired. In addition, portions of signal sequences may also be used to direct the intracellular import of a peptide or protein of interest. This may prove beneficial in gene therapy strategies in which it is desired to deliver a particular gene product to cells other than the cell in which it is produced. Signal sequences encoding signal peptides also find application in simplifying protein purification techniques. In such applications, the extracellular secretion of the desired protein greatly facilitates purification by reducing the number of undesired proteins from which the desired protein must be selected. Thus, there exists a need to identify and characterize the 5' portions of the genes for secretory proteins which encode signal peptides.

Public information on the number of human genes for which the promoters and upstream regulatory regions have been identified and characterized is quite limited. In part, this may be due to the difficulty of isolating such regulatory sequences. Upstream regulatory sequences such as transcription factor binding sites are typically too short to be utilized as probes for isolating promoters from human genomic libraries. Recently, some approaches have been developed to isolate human promoters. One of them consists of making a CpG island library (Cross, *et al., Nature Genetics* **6**: 236-244, 1994). The second consists of isolating human genomic DNA sequences containing SpeI binding sites by the use of SpeI binding protein. (Mortlock *et al., Genome Res.* **6**:327-335, 1996). Both of these approaches have their limits due to a lack of specificity or of comprehensiveness.

The present 5' ESTs may be used to efficiently identify and isolate upstream regulatory regions which control the location, developmental stage, rate, and quantity of protein synthesis, as well as the stability of the mRNA. (Theil, *BioFactors* **4**:87-93, 1993). Once identified and characterized, these regulatory regions may be utilized in gene therapy or protein purification schemes to obtain the desired amount and locations of protein synthesis or to inhibit, reduce, or prevent the synthesis of undesirable gene products.

In addition, ESTs containing the 5' ends of secretory protein genes may include sequences useful as probes for chromosome mapping and the identification of individuals. Thus, there is a need to identify and characterize the sequences upstream of the 5' coding sequences of genes encoding secretory proteins.

### Summary of the Invention

The present invention relates to purified, isolated, or recombinant ESTs which include sequences derived from the authentic 5' ends of their corresponding mRNAs. The term "corresponding mRNA" refers to the mRNA which was the template for the cDNA synthesis which produced the 5' EST. These sequences will be referred to hereinafter as "5' ESTs." As used herein, the term "purified" does not require absolute purity; rather, it is intended as a relative definition. Individual 5' EST clones isolated from a cDNA library have been conventionally purified to electrophoretic homogeneity. The sequences obtained from these clones could not be obtained directly either from the library or from total human DNA. The cDNA clones are not naturally occurring as such, but rather are obtained via manipulation of a partially purified naturally occurring substance (messenger RNA). The conversion of mRNA into a cDNA library involves the creation of a synthetic substance (cDNA) and pure individual cDNA clones can be isolated from the synthetic library by clonal selection. Thus, creating a cDNA library from messenger RNA and subsequently isolating individual clones from that library results in an approximately 10⁴-10⁶ fold purification of the native message. Purification of starting material or natural material to at least one order of magnitude, preferably two or three orders, and more preferably four or five orders of magnitude is expressly contemplated.

As used herein, the term "isolated" requires that the material be removed from its original environment (e.g., the natural environment if it is naturally occurring). For example, a naturally-occurring polynucleotide present in a living animal is not isolated, but the same polynucleotide, separated from some or all of the coexisting materials in the natural system, is isolated.

As used herein, the term "recombinant" means that the 5' EST is adjacent to "backbone" nucleic acid to which it is not adjacent in its natural environment. Additionally, to be "enriched" the 5' ESTs will represent 5% or more of the number of nucleic acid inserts in a population of nucleic acid backbone molecules. Backbone molecules according to the present invention include nucleic acids such as expression vectors, self-replicating nucleic acids, viruses, integrating nucleic acids, and other vectors or nucleic acids used to maintain or manipulate a nucleic acid insert of interest. Preferably, the enriched 5' ESTs represent 15% or more of the number of nucleic acid inserts in the population of recombinant backbone molecules. More preferably, the enriched 5' ESTs represent 50% or more of the number of nucleic acid inserts in the population of recombinant backbone molecules. In a highly preferred embodiment, the enriched 5' ESTs represent 90% or more of the number of nucleic acid inserts in the population of recombinant backbone molecules.

"Stringent", moderate," and "low" hybridization conditions are as defined in Example 29.

Unless otherwise indicated, a "complementary" sequence is fully complementary.

Thus, 5' ESTs in cDNA libraries in which one or more 5' ESTs make up 5% or more of the number of nucleic acid inserts in the backbone molecules are "enriched recombinant 5' ESTs" as defined herein. Likewise, 5' ESTs in a population of plasmids in which one or more 5' EST of the present invention have been inserted such that they represent 5% or more of the number of inserts in the plasmid backbone are " enriched recombinant 5' ESTs" as defined herein. However, 5' ESTs in cDNA libraries in which 5' ESTs constitute less than 5% of the number of nucleic acid inserts in the population of backbone molecules, such as libraries in which backbone molecules having a 5' EST insert are extremely rare, are not "enriched recombinant 5' ESTs."

In particular, the present invention relates to 5' ESTs which are derived from genes encoding secreted proteins. As used herein, a "secreted" protein is one which, when expressed in a suitable host cell, is transported across or through a membrane, including transport as a result of signal peptides in its amino acid sequence. "Secreted" proteins include without limitation proteins secreted wholly (e.g. soluble proteins), or partially (e.g. receptors) from the cell in which they are expressed. "Secreted" proteins also include without limitation proteins which are transported across the membrane of the endoplasmic reticulum.

Such 5' ESTs include nucleic acid sequences, called signal sequences, which encode signal peptides which direct the extracellular secretion of the proteins encoded by the genes from which the 5' ESTs are derived. Generally, the signal peptides are located at the amino termini of secreted proteins.

Secreted proteins are translated by ribosomes associated with the "rough" endoplasmic reticulum. Generally, secreted proteins are co-translationally transferred to the membrane of the endoplasmic reticulum. Association of the ribosome with the endoplasmic reticulum during translation of secreted proteins is mediated by the signal peptide. The signal peptide is typically cleaved following its co-translational entry into the endoplasmic reticulum. After delivery to the endoplasmic reticulum, secreted proteins may proceed through the Golgi apparatus. In the Golgi apparatus, the proteins may undergo post-translational modification before entering secretory vesicles which transport them across the cell membrane.

The 5' ESTs of the present invention have several important applications. For example, they may be used to obtain and express cDNA clones which include the full protein coding sequences of the corresponding gene products, including the authentic translation start sites derived from the 5' ends of the coding sequences of the mRNAs from which the 5' ESTs are derived. These cDNAs will be referred to hereinafter as "full length cDNAs." These cDNAs may also include DNA derived from mRNA sequences upstream of the translation start site. The full length cDNA sequences may be used to express the proteins corresponding to the 5' ESTs. As discussed above secreted proteins are therapeutically important. Thus, the proteins expressed from the cDNAs may be useful in treating or controlling a variety of human conditions. The 5' ESTs may also be used to obtain the corresponding genomic DNA. The term "corresponding genomic DNA" refers to the genomic DNA which encodes the mRNA from which the 5' EST was derived.

Alternatively, the 5' ESTs may be used to obtain and express extended cDNAs encoding portions of the secreted protein. The portions may comprise the signal peptides of the secreted proteins or the mature proteins generated when the signal peptide is cleaved off The portions may also comprise polypeptides having at least 10 consecutive amino acids encoded by the extended cDNAs or full length cDNAs. Alternatively, the portions may comprise at least 15 consecutive amino acids encoded by the extended cDNAs or full length cDNAs. In some embodiments, the portions may comprise at least 25 consecutive amino acids encoded by the extended cDNAs or full length cDNAs. In other embodiments, the portions may comprise at least 40 amino acids encoded by the extended cDNAs or full length cDNAs.

Antibodies which specifically recognize the entire secreted proteins encoded by the extended cDNAs, full length cDNAs, or fragments thereof having at least 10 consecutive amino acids, at least 15 consecutive amino acids, at least 25 consecutive amino acids, or at least 40 consecutive amino acids may also be obtained as described below. Antibodies which specifically recognize the mature protein generated when the signal peptide is cleaved may also be obtained as described below. Similarly, antibodies which specifically recognize the signal peptides encoded by the extended cDNAs or full length cDNAs may also be obtained.

In some embodiments, the extended cDNAs obtained using the 5' ESTs include the signal sequence. In other embodiments, the extended cDNAs obtained using the 5' ESTs may include the full coding sequence for the mature protein (*i.e*. the protein generated when the signal polypeptide is cleaved off). In addition, the extended cDNAs obtained using the 5' ESTs may include regulatory regions upstream of the translation start site or downstream of the stop codon which control the amount, location, or developmental stage of gene expression.

As discussed above, secreted proteins are therapeutically important. Thus, the proteins expressed from the extended cDNAs or full length cDNAs obtained using the 5' ESTs may be useful in treating or controlling a variety of human conditions.

The 5' ESTs (or cDNAs or genomic DNAs obtained therefrom) may be used in forensic procedures to identify individuals or in diagnostic procedures to identify individuals having genetic diseases resulting from abnormal expression of the genes corresponding to the 5' ESTs. In addition, the present invention is useful for constructing a high resolution map of the human chromosomes.

The present invention also relates to secretion vectors capable of directing the secretion of a protein of interest. Such vectors may be used in gene therapy strategies in which it is desired to produce a gene product in one cell which is to be delivered to another location in the body. Secretion vectors may also facilitate the purification of desired proteins.

The present invention also relates to expression vectors capable of directing the expression of an inserted gene in a desired spatial or temporal manner or at a desired level. Such vectors may include sequences upstream of the 5' ESTs, such as promoters or upstream regulatory sequences.

Finally, the present invention may also be used for gene therapy to control or treat genetic diseases. Signal peptides may also be fused to heterologous proteins to direct their extracellular secretion.

Bacterial clones containing Bluescript plasmids having inserts containing the 5' ESTs of the present invention (SEQ ID NOs: 38-291 are presently stored at 80°C in 4% (v/v) glycerol in the inventor's laboratories under the designations listed next to the SEQ ID NOs in II). The inserts may be recovered from the deposited materials by growing the appropriate clones on a suitable medium. The Bluescript DNA can then be isolated using plasmid isolation procedures familiar to those skilled in the art such as alkaline lysis minipreps or large scale alkaline lysis plasmid isolation procedures. If desired the plasmid DNA may be further enriched by centrifugation on a cesium chloride gradient, size exclusion chromatography, or anion exchange chromatography. The plasmid DNA obtained using these procedures may then be manipulated using standard cloning techniques familiar to those skilled in the art. Alternatively, a PCR can be done with primers designed at both ends of the EST insertion. The PCR product which corresponds to the 5' EST can then be manipulated using standard cloning techniques familiar to those skilled in the art.

One aspect of the present invention is a signal peptide having the sequence of amino acids -30 to - 1 of SEQ ID No: 299.

Another aspect of the present invention is a signal sequence encoding a signal peptide having the sequence of amino acids -30 to -1 of SEQ ID No: 299. In one embodiment the signal sequence has the sequence of nucleotides 115 to 204 of SEQ ID No: 45.

Yet another aspect of the present invention is a purified and isolated nucleic acid encoding a human secreted polypeptide comprising the sequence of amino acids -30 to -1 of SEQ ID No: 299 at the 5'-end of the coding sequence. In a first embodiment, the nucleic acid comprises a signal sequence having the sequence of nucleotides 115 to 204 of SEQ ID No: 45. In this embodiment, the amino terminus of said polypeptide may be encoded by nucleotides 115 to 396 of SEQ ID No: 45. In a second embodiment, the signal sequence is fused in frame to the 5'-end of a sequence encoding a polypeptide that is heterologous to a polypeptide comprising amino acids 1 to 64 of SEQ ID No: 299.

A further embodiment of the present invention is an expression vector comprising a signal sequence encoding a signal peptide having the sequence of amino acids -30 to -1 of SEQ ID No: 299 operably linked to a promoter. The expression vector may be a secretion vector. In one embodiment the signal sequence has the sequence of nucleotides 115 to 204 of SEQ ID No: 45.

Another aspect of the invention is an expression vector comprising a purified and isolated nucleic acid encoding a human secreted polypeptide, operably linked to a promoter, wherein the isolated nucleic acid encodes the sequence of amino acids -30 to -1 of SEQ ID No: 299 at the 5'-end of the coding sequence. The expression vector may be a secretion vector. In a first embodiment, the nucleic acid comprises a signal sequence having the sequence of nucleotides 115 to 204 of SEQ ID No: 45. In this embodiment, the amino terminus of said polypeptide is encoded by nucleotides 115 to 396 of SEQ ID No: 45. In a second embodiment, the signal sequence is fused in frame to the 5'-end of a sequence encoding a polypeptide that is heterologous to a polypeptide comprising amino acids 1 to 64 of SEQ ID No: 299.

Still another aspect of the present invention is a polypeptide encoded by a purified and isolated nucleic acid encoding a human secreted polypeptide comprising the sequence of amino acids -30 to -1 of SEQ ID No: 299 at the 5'-end of the coding sequence. The polypeptide may comprise amino acids 1 to 64 of SEQ ID No: 299. In a first embodiment, the nucleic acid comprises a signal sequence having the sequence of nucleotides 115 to 204 of SEQ ID No: 45. In this embodiment, the amino terminus of said polypeptide may be encoded by nucleotides 115 to 396 of SEQ ID No: 45. In a second embodiment, the signal sequence is fused in frame to the 5'-end of a sequence encoding a polypeptide that is heterologous to a polypeptide comprising amino acids I to 64 of SEQ ID No: 299.

Another aspect of the present invention is a fusion protein encoded by a purified and isolated nucleic acid encoding a human secreted polypeptide comprising the sequence of amino acids -30 to -1 of SEQ ID No: 299 at the 5'-end of the coding sequence, wherein the signal sequence is fused in frame to the 5'-end of a sequence encoding a polypeptide that is heterologous to a polypeptide comprising amino acids 1 to 64 of SEQ ID No: 299.

A further aspect of the present invention is the use of a signal peptide having the sequence of amino acids -30 to -1 of SEQ ID No: 299 for directing the extracellular secretion of a polypeptide provided that said use is not applied to a human body or animal.

Another aspect of the present invention is use of a signal peptide having the sequence of amino acids -30 to -1 of SEQ ID No: 299 for simplifying protein purification of a desired polypeptide. The polypeptide may be encoded by a purified and isolated nucleic acid encoding a human secreted polypeptide comprising the sequence of amino acids -30 to -1 of SEQ ID No: 299 at the 5'-end of the coding sequence. The polypeptide may comprise amino acids I to 64 of SEQ ID No: 299. In a first embodiment, the nucleic acid comprises a signal sequence having the sequence of nucleotides 115 to 204 of SEQ ID No: 45. In this embodiment, the amino terminus of said polypeptide may be encoded by nucleotides 115 to 396 of SEQ ID No: 45. In a second embodiment, the signal sequence is fused in frame to the 5'-end of a sequence encoding a polypeptide that is heterologous to a polypeptide comprising amino acids 1 to 64 of SEQ ID No: 299.

A further aspect of the present invention is use of a vector comprising a purified and isolated nucleic acid encoding a human secreted polypeptide, operably linked to a promoter, wherein the isolated nucleic acid comprises the sequence of amino acids -30 to -1 of SEQ ID No: 299 at the 5'-end of the coding sequence for directing the extracellular secretion of a polypeptide provided that said use is not applied to a human or animal body. The expression vector may be a secretion vector. The polypeptide whose secretion is directed may be encoded by a purified and isolated nucleic acid encoding a human secreted polypeptide comprising the sequence of amino acids -30 to -1 of SEQ ID No: 299 at the 5'-end of the coding sequence. The polypeptide may comprise amino acids 1 to 64 of SEQ ID No: 299. In a first embodiment, the nucleic acid comprises a signal sequence having the sequence of nucleotides 115 to 204 of SEQ ID No: 45. In this embodiment, the amino terminus of said polypeptide may be encoded by nucleotides 115 to 396 of SEQ ID No: 45. In a second embodiment, the signal sequence is fused in frame to the 5'-end of a sequence encoding a polypeptide that is heterologous to a polypeptide comprising amino acids 1 to 64 of SEQ ID No: 299. In a first embodiment, the nucleic acid comprises a signal sequence having the sequence of nucleotides 115 to 204 of SEQ ID No: 45. In this embodiment, the amino terminus of said polypeptide is encoded by nucleotides 115 to 396 of SEQ ID No: 45. In a second embodiment, the signal sequence is fused in frame to the 5'-end of a sequence encoding a polypeptide that is heterologous to a polypeptide comprising amino acids 1 to 64 of SEQ ID No: 299.

Use of an expression vector comprising a purified and isolated nucleic acid encoding a human secreted polypeptide, operably linked to a promoter, wherein the isolated nucleic acid comprises the sequence of amino acids -30 to -1 of SEQ ID No: 299 at the 5'-end of the coding sequence for simplifying protein purification of a desired polypeptide. The expression vector may be a secretion vector. The polypeptide may comprise amino acids 1 to 64 of SEQ ID No: 299. In a first embodiment, the nucleic acid comprises a signal sequence having the sequence of nucleotides 115 to 204 of SEQ ID No: 45. In this embodiment, the amino terminus of said polypeptide may be encoded by nucleotides 115 to 396 of SEQ ID No: 45. In a second embodiment, the signal sequence is fused in frame to the 5'-end of a sequence encoding a polypeptide that is heterologous to a polypeptide comprising amino acids 1 to 64 of SEQ ID No: 299. In a first embodiment, the nucleic acid comprises a signal sequence having the sequence of nucleotides 115 to 204 of SEQ ID No: 45. In this embodiment, the amino terminus of said polypeptide is encoded by nucleotides 115 to 396 of SEQ ID No: 45. In a second embodiment, the signal sequence is fused in frame to the 5'-end of a sequence encoding a polypeptide that is heterologous to a polypeptide comprising amino acids 1 to 64 of SEQ ID No: 299.

### Brief Description of the Drawings

Figure 1 is a summary of a procedure for obtaining cDNAs which have been selected to include the 5' ends of the mRNAs from which they derived.
Figure 2 shows the distribution of Von Heijne scores for 5' ESTs in each of the categories described herein and the probability that these 5' ESTs encode a signal peptide.
Figure 3 summarizes a general method used to clone and sequence extended cDNAs containing sequences adjacent to 5' ESTs.
Figure 4 (description of promoters structure isolated from SignalTag 5' ESTs) provides a schematic description of promoters isolated and the way they are assembled with the corresponding 5' tags.

### Detailed Description

Table IV is an analysis of the 43 amino acids located at the N terminus of all human SwissProt proteins to determine the frequency of false positives and false negatives using the techniques for signal peptide identification described herein.

Table V shows the distribution of 5' ESTs in each category described herein and the number of 5' ESTs in each category having a given minimum Von Heijne's score.

Table VI shows the distribution of 5' ESTs in each category described herein with respect to the tissue from which the 5' ESTs of the corresponding mRNA were obtained.

Table VII describes the transcription factor binding sites present in each of these promoters.

### I. General Methods for Obtaining 5' ESTs derived from mRNAs with intact 5' ends

In order to obtain the 5' ESTs of the present invention, mRNAs with intact 5' ends must be obtained. Currently, there are two approaches for obtaining such mRNAs with intact 5' ends as described below: either chemical (1) or enzymatic (2).

### 1. Chemical Methods for Obtaining mRNAs having Intact 5' Ends

One of these approaches is a chemical modification method involving derivatization of the 5' ends of the mRNAs and selection of the derivatized mRNAs. The 5' ends of eukaryotic mRNAs possess a structure referred to as a "cap" which comprises a guanosine methylated at the 7 position. The cap is joined to the first transcribed base of the mRNA by a 5', 5'-triphosphate bond. In some instances, the 5' guanosine is methylated in both the 2 and 7 positions. Rarely, the 5' guanosine is trimethylated at the 2, 7 and 7 positions. In the chemical method for obtaining mRNAs having intact 5' ends, the 5' cap is specifically derivatized and coupled to a reactive group on an immobilizing substrate. This specific derivatization is based on the fact that only the ribose linked to the methylated guanosine at the 5' end of the mRNA and the ribose linked to the base at the 3' terminus of the mRNA, possess 2', 3'-cis diols.

Optionally, the 2', 3'-cis diol of the 3' terminal ribose may be chemically modified, substituted, converted, or eliminated, leaving only the ribose linked to the methylated guanosine at the 5' end of the mRNA with a 2', 3'-cis diol. A variety of techniques are available for eliminating the 2', 3'-cis diol on the 3' terminal ribose. For example, controlled alkaline hydrolysis may be used to generate mRNA fragments in which the 3' terminal ribose is a 3'-phosphate, 2'-phosphate or (2', 3')-cyclophosphate. Thereafter, the fragment which includes the original 3' ribose may be eliminated from the mixture through chromatography on an oligodT column. Alternatively, a base which lacks the 2', 3'-cis diol may be added to the 3' end of the mRNA using an RNA ligase such as T4 RNA ligase. Example 1 below describes a method for ligation of a nucleoside diphosphate to the 3' end of messenger RNA.

### EXAMPLE 1

### Ligation of the Nucleoside Diphosphate pCp to the 3' End of mRNA

One µg of RNA was incubated in a final reaction medium of 10 µl in the presence of 5U of T₄ phage RNA ligase in the buffer provided by the manufacturer (Gibco - BRL), 40 U of the RNase inhibitor RNasin (Promega) and, 2 µl of ³²pCp (Amersham #PB 10208). The incubation was performed at 37°C for 2 hours or overnight at 7-8°C.

Following modification or elimination of the 2', 3'-cis diol at the 3' ribose, the 2', 3'-cis diol present at the 5' end of the mRNA may be oxidized using reagents such as NaBH₄, NaBH₃CN, or sodium periodate, thereby converting the 2', 3'-cis diol to a dialdehyde. Example 2 describes the oxidation of the 2', 3'-cis diol at the 5' end of the mRNA with sodium periodate.

### EXAMPLE 2

### Oxidation of 2', 3'-cis diol at the 5' End of the mRNA with Sodium Periodate

0.1 OD unit of either a capped oligoribonucleotide of 47 nucleotides (including the cap) or an uncapped oligoribonucleotide of 46 nucleotides were treated as follows. The oligoribonucleotides were produced by *in vitro* transcription using the transcription kit "AmpliScribe T7" (Epicentre Technologies). As indicated below, the DNA template for the RNA transcript contained a single cytosine. To synthesize the uncapped RNA, all four NTPs were included in the *in vitro* transcription reaction. To obtain the capped RNA, GTP was replaced by an analogue of the cap, m7G(5')ppp(5')G. This compound, recognized by the polymerase, was incorporated into the 5' end of the nascent transcript during the initiation of transcription but was not incorporated during the extension step. Consequently, the resulting RNA contained a cap at its 5' end. The sequences of the oligoribonucleotides produced by the *in vitro* transcription reaction were:
+Cap:
   5'm7GpppGCAUCCUACUCCCAUCCAAUUCCACCCUAACUCCUCCCAUCUCCAC-3' (SEQ ID NO:1)
-Cap:
   5'-pppGCAUCCUACUCCCAUCCAAUUCCACCCUAACUCCUCCCAUCUCCAC-3' (SEQ ID NO:2)

The oligoribonucleotides were dissolved in 9 µl of acetate buffer (0.1 M sodium acetate, pH 5.2) and 3 µl of freshly prepared 0.1 M sodium periodate solution. The mixture was incubated for 1 hour in the dark at 4°C or room temperature. Thereafter, the reaction was stopped by adding 4 µl of 10% ethylene glycol. The product was ethanol precipitated, resuspended in at least 10 µl of water or appropriate buffer and dialyzed against water.

The resulting aldehyde groups may then be coupled to molecules having a reactive amine group, such as hydrazine, carbazide, thiocarbazide or semicarbazide groups, in order to facilitate enrichment of the 5' ends of the mRNAs. Molecules having reactive amine groups which are suitable for use in selecting mRNAs having intact 5' ends include avidin, proteins, antibodies, vitamins, ligands capable of specifically binding to receptor molecules, or oligonucleotides. Example 3 below describes the coupling of the resulting dialdehyde to biotin.

### EXAMPLE 3

### Coupling of the Dialdehyde at the 5' End of Transcripts with Biotin

The oxidation product obtained in Example 2 was dissolved in 50 µl of sodium acetate at a pH between 5 and 5.2 and 50 µl of freshly prepared 0.02 M solution of biotin hydrazide in a methoxyethanol/water mixture (1:1) of formula:

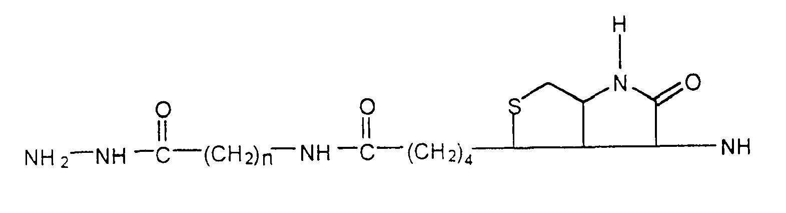

In the compound used in these experiments, n=5. However, it will be appreciated that other commercially available hydrazides may also be used, such as molecules of the above formula in which n varies from 0 to 5. The mixture was then incubated for 2 hours at 37°C, precipitated with ethanol and dialyzed against distilled water. Example 4 demonstrates the specificity of the biotinylation reaction.

### EXAMPLE 4

### Specificity of Biotinylation of Capped Transcripts

The specificity of the biotinylation for capped mRNAs was evaluated by gel electrophoresis of the following samples:
Sample 1. The 46 nucleotide uncapped *in vitro* transcript prepared as in Example 2 and labeled with ³²pCp as described in Example 1
Sample 2. The 46 nucleotide uncapped *in vitro* transcript prepared as in Example 2, labeled with ³²pCp as described in Example 1, treated with the oxidation reaction of Example 2, and subjected to the biotinylation conditions of Example 3.
Sample 3. The 47 nucleotide capped *in vitro* transcript prepared as in Example 2 and labeled with ³²pCp as described in Example 1.
Sample 4. The 47 nucleotide capped *in vitro* transcript prepared as in Example 2, labeled with ³²pCp as described in Example 1, treated with the oxidation reaction of Example 2, and subjected to the biotinylation conditions of Example 3.

Samples 1 and 2 had identical migration rates, demonstrating that the uncapped RNAs were not oxidized and biotinylated. Sample 3 migrated more slowly than Samples 1 and 2, while Sample 4 exhibited the slowest migration. The difference in migration of the RNAs in Samples 3 and 4 demonstrates that the capped RNAs were specifically biotinylated.

In some cases, mRNAs having intact 5' ends may be enriched by binding the molecule containing a reactive amine group to a suitable solid phase substrate such as the inside of the vessel containing the mRNAs, magnetic beads, chromatography matrices, or nylon or nitrocellulose membranes. For example, where the molecule having a reactive amine group is biotin, the solid phase substrate may be coupled to avidin or streptavidin. Alternatively, where the molecule having the reactive amine group is an antibody or receptor ligand, the solid phase substrate may be coupled to the cognate antigen or receptor. Finally, where the molecule having a reactive amine group comprises an oligonucleotide, the solid phase substrate may comprise a complementary oligonucleotide.

The mRNAs having intact 5' ends may be released from the solid phase following the enrichment procedure. For example, where the dialdehyde is coupled to biotin hydrazide and the solid phase comprises streptavidin, the mRNAs may be released from the solid phase by simply heating to 95 degrees Celsius in 2% SDS. In some methods, the molecule having a reactive amine group may also be cleaved from the mRNAs having intact 5' ends following enrichment. Example describes the capture of biotinylated mRNAs with streptavidin coated beads and the release of the biotinylated mRNAs from the beads following enrichment.

### EXAMPLE 5

### Capture and Release of Biotinylated mRNAs Using Streptavidin Coated Beads

The streptavidin coated magnetic beads were prepared according to the manufacturer's instructions (CPG Inc., USA). The biotinylated mRNAs were added to a hybridization buffer (1.5 M NaCl, pH 5 - 6). After incubating for 30 minutes, the unbound and nonbiotinylated material was removed. The beads were then washed several times in water with 1% SDS. The beads thus obtained were incubated for 15 minutes at 95°C in water containing 2% SDS.

Example 6 demonstrates the efficiency with which biotinylated mRNAs were recovered from the streptavidin coated beads.

### EXAMPLE 6

### Efficiency of Recovery of Biotinylated mRNAs

The efficiency of the recovery procedure was evaluated as follows. Capped RNAs were labeled with ³²pCp, oxidized, biotinylated and bound to streptavidin coated beads as described above. Subsequently, the bound RNAs were incubated for 5, 15 or 30 minutes at 95°C in the presence of 2% SDS.

The products of the reaction were analyzed by electrophoresis on 12% polyacrylamide gels under denaturing conditions (7 M urea). The gels were subjected to autoradiography. During this manipulation, the hydrazone bonds were not reduced.

Increasing amounts of nucleic acids were recovered as incubation times in 2% SDS increased, demonstrating that biotinylated mRNAs were efficiently recovered.

In an alternative method for obtaining mRNAs having intact 5' ends, an oligonucleotide which has been derivatized to contain a reactive amine group is specifically coupled to mRNAs having an intact cap. Preferably, the 3' end of the mRNA is blocked prior to the step in which the aldehyde groups are joined to the derivatized oligonucleotide, as described above, so as to prevent the derivatized oligonucleotide from being joined to the 3' end of the mRNA. For example, pCp may be attached to the 3' end of the mRNA using T4 RNA ligase as described in example 1. However, as discussed above, blocking the 3' end of the mRNA is an optional step. Derivatized oligonucleotides may be prepared as described in Example 7.

### EXAMPLE 7

### Derivatization of Oligonucleotides

An oligonucleotide phosphorylated at its 3' end was converted to a 3' hydrazide in 3' by treatment with an aqueous solution of hydrazine or of dihydrazide of the formula H₂N(R1)NH₂ at about 1 to 3 M, and at pH 4.5 at a temperature of 8°C overnight. This incubation was performed in the presence of a carbodiimide type agent soluble in water such as 1-ethyl-3-(3-dimethylaminopropyl)carbodiimide at a final concentration of 0.3 M.

The derivatized oligonucleotide was then separated from the other agents and products using a standard technique for isolating oligonucleotides.

As discussed above, the mRNAs to be enriched may be treated to eliminate the 3' OH groups which may be present thereon. This may be accomplished by enzymatic ligation of sequences lacking a 3' OH, such as pCp, as described in Example 1. Alternatively, the 3' OH groups may be eliminated by alkaline hydrolysis as described in Example 8 below.

### EXAMPLE 8

### Elimination of 3' OH Groups of mRNA Using Alkaline Hydrolysis

In a total volume of 100 µl of 0.1 N sodium hydroxide, 1.5 µg mRNA is incubated for 40 to 60 minutes at 4°C. The solution is neutralized with acetic acid and precipitated with ethanol.

Following the optional elimination of the 3' OH groups, the diol groups at the 5' ends of the mRNAs are oxidized as described below in Example 9.

### EXAMPLE 9

### Oxidation of Diols of mRNA

Up to 1 OD unit of RNA was dissolved in 9 µl of buffer (0.1 M sodium acetate, pH 6-7) or water and 3 µl of freshly prepared 0.1 M sodium periodate solution. The reaction was incubated for 1 h in the dark at 4°C or room temperature. Following the incubation, the reaction was stopped by adding 4 µl of 10% ethylene glycol. Thereafter the mixture was incubated at room temperature for 15 minutes. After ethanol precipitation, the product was resuspended in at least 10 µl of water or appropriate buffer and dialyzed against water.

Following oxidation of the diol groups at the 5' ends of the mRNAs, the derivatized oligonucleotide was joined to the resulting aldehydes as described in Example 10.

### EXAMPLE 10

### Ligature of Aldehydes of mRNA to Derivatized Oligonucleotides

The oxidized mRNA was dissolved in an acidic medium such as 50 µl of sodium acetate pH 4-6. Fifty µl of a solution of the derivatized oligonucleotide were added in order to obtain an mRNA:derivatized oligonucleotide ratio of 1:20. The mixture was reduced with a borohydride and incubated for 2 h at 37°C or overnight ( 14 h) at 10°C. The mixture was then ethanol precipitated, resuspended in 10 µl or more of water or appropriate buffer and dialyzed against distilled water. If desired, the resulting product may be analyzed using acrylamide gel electrophoresis, HPLC analysis, or other conventional techniques.

Following the attachment of the derivatized oligonucleotide to the mRNAs, a reverse transcription reaction may be performed as described in Example 11 below.

### EXAMPLE 11

### Reverse Transcription of mRNAs Ligatured to Derivatized Oligonucleotides

An oligodeoxyribonucleotide was derivatized as follows. Three OD units of an oligodeoxyribonucleotide of sequence 5'ATCAAGAATTCGCACGAGACCATTA3' (SEQ ID NO:3) having 5'-OH and 3'-P ends were dissolved in 70 µl of a 1.5 M hydroxybenzotriazote solution, pH 5.3, prepared in dimethylformamide/water (75:25) containing 2 µg of 1-ethyl-3-(3-dimethylaminopropyl)carbodiimide. The mixture was incubated for 2 h 30 min at 22°C and then precipitated twice in LiClO₄/acetone. The pellet was resuspended in 200 µl of 0.25 M hydrazine and incubated at 8°C from 3 to 14 h. Following the hydrazine reaction, the mixture was precipitated twice in LiClO₄/acetone.

The messenger RNAs to be reverse transcribed were extracted from blocks of placenta having sides of 2 cm which had been stored at -80°C. The total RNA was extracted using conventional acidic phenol techniques. Oligo-dT chromatography was used to purify the mRNAs. The integrity of the mRNAs was checked by Northern-blotting.,

The diol groups on 7 µg of the placental mRNAs were oxidized as described above in Example 9. The derivatized oligonucleotide was joined to the mRNAs as described in Example 10 above except that the precipitation step was replaced by an exclusion chromatography step to remove derivatized oligodeoxyribonucleotides which were not joined to mRNAs. Exclusion chromatography was performed as follows:

Ten ml of Ultrogel AcA34 (BioSepra#230151) gel, a mix of agarose and acrylamide, were equilibrated in 50 ml of a solution of 10 mM Tris pH 8.0, 300 mM NaCl, 1 mM EDTA, and 0.05% SDS. The mixture was allowed to sediment. The supernatant was eliminated and the gel was resuspended in 50 ml of buffer. This procedure was repeated 2 or 3 times.

A glass bead (diameter 3 mm) was introduced into a 2 ml disposable pipette (length 25 cm). The pipette was filled with the gel suspension until the height of the gel stabilized at 1 cm from the top of the pipette. The column was then equilibrated with 20 ml of equilibration buffer (10 mM Tris HCl pH 7.4, 20 mM NaCl).

Ten µl of the mRNA which had reacted with the derivatized oligonucleotide were mixed in 39 µl of 10 mM urea and 2 µl of blue-glycerol buffer, which had been prepared by dissolving 5 mg of bromophenol blue in 60% glycerol (v/v), and passing the mixture through a 0.45 µm diameter filter.

The column was then loaded with the mRNAs coupled to the oligonucleotide. As soon as the sample had penetrated, equilibration buffer was added. Hundred µl fractions were then collected. Derivatized oligonucleotide which had not been attached to mRNA appeared in fraction 16 and later fractions. Thus, fractions 3 to 15 were combined and precipitated with ethanol.

To determine whether the derivatized oligonucleotide was actually linked to mRNA, one tenth of the combined fractions were spotted twice on a nylon membrane and hybridized to a radioactive probe using conventional techniques. The ³²P labeled probe used in these hybridizations was an oligodeoxyribonucleotide of sequence 5'TAATGGTCTCGTGCGAATTCTTGAT3' (SEQ ID NO:4) anticomplementary to the derivatized oligonucleotide. A signal observed after autoradiography, indicated that the derivatized oligonucleotide had been truly joined to the mRNA.

The remaining nine tenth of the mRNAs which had reacted with the derivatized oligonucleotide was reverse transcribed as follows. A reverse transcription reaction was carried out with reverse transcriptase following the manufacturer's instructions and 50 pmol of nonamers with random sequence as primers.

To ensure that reverse transcription had been carried out through the cap structure, two types of experiments were performed.

In the first approach, after elimination of RNA of the cDNA:RNA heteroduplexes obtained from the reverse transcription reaction by an alkaline hydrolysis, a portion of the resulting single stranded cDNAs was spotted on a positively charged membrane and hybridized, using conventional methods, to a ³²P labeled probe having a sequence identical to that of the derivatized oligonucleotide. Control spots containing, 1 pmol, 100 fmol, 50 fmol, 10 fmol and 1 fmol of a control oligodeoxyribonucleotide of sequence identical to that of the derivatized oligonucleotide were included. The signal observed in the spots containing the cDNA indicated that approximately 15 fmol of the derivatized oligonucleotide had been reverse transcribed. These results demonstrate that the reverse transcription can be performed through the cap and, in particular, that reverse transcriptase crosses the 5'-P-P-P-5' bond of the cap of eukaryotic messenger RNAs.

In the second type of experiment, the single stranded cDNAs obtained from the above first strand synthesis were used as template for PCR reactions. Two types of reactions were carried out. First, specific amplification of the mRNAs for alpha globin, dehydrogenase, pp15 and elongation factor E4 were carried out using the following pairs of oligodeoxyribonucleotide primers.
alpha-globin
   GLO-S: 5'CCG ACA AGA CCA ACG TCA AGG CCG C3' (SEQ ID NO:5)
   GLO-As: 5'TCA CCA GCA GGC AGT GGC TTA GGA G 3' (SEQ ID NO:6)
dehydrogenase
   3 DH-S: 5'AGT GAT TCC TGC TAC TTT GGA TGG C3' (SEQ ID NO:7)
   3 DH-As: 5'GCT TGG TCT TGT TCT GGA GTT TAG A3' (SEQ ID NO:8)
pp15
   PP 15-S: 5'TCC AGA ATG GGA GAC AAG CCA ATT T3' (SEQ ID NO:9)
   PP 15-As: 5'AGG GAG GAG GAA ACA GCG TGA GTC C3' (SEQ ID NO:10)
Elongation factor E4
   EFA1-S: 5'ATG GGA AAG GAA AAG ACT CAT ATC A3' (SEQ ID NO:11)
   EF1A-As: 5'AGC AGC AAC AAT CAG GAC AGC ACA G3' (SEQ ID NO:12)

Second, non specific amplifications were also carried out with the antisense oligodeoxyribonucleotides of the pairs described above and with a primer derived from the sequence of the derivatized oligodeoxyribonucleotide (5'ATCAAGAATTCGCACGAGACCATTA3') (SEQ ID NO:13).

One twentieth of the following RT-PCR product samples were run on a 1.5% agarose gel and stained with ethidium bromide.
Sample 1: The products of a PCR reaction using the globin primers of SEQ ID NOs 5 and 6 in the presence of cDNA.
Sample 2: The products of a PCR reaction using the globin primers of SEQ ID NOs 5 and 6 in the absence of added cDNA.
Sample 3: The products of a PCR reaction using the dehydrogenase primers of SEQ ID NOs 7 and 8 in the presence of cDNA.
Sample 4: The products of a PCR reaction using the dehydrogenase primers of SEQ ID NOs 7 and 8 in the absence of added cDNA.
Sample 5: The products of a PCR reaction using the pp15 primers of SEQ ID NOs 9 and 10 in the presence of cDNA.
Sample 6: The products of a PCR reaction using the pp15 primers of SEQ ID NOs 9 and 10 in the absence of added cDNA.
Sample 7: The products of a PCR reaction using the EIF4 primers of SEQ ID NOs 11 and 12 in the presence of added cDNA.
Sample 8: The products of a PCR reaction using the EIF4 primers of SEQ ID NOs 11 and 12 in the absence of added cDNA.

A band of the size expected for the PCR product was observed only in samples 1, 3, 5 and 7, thus indicating the presence of the corresponding sequence in the cDNA population.

PCR reactions were also carried out with the antisense oligonucleotides of the globin and dehydrogenase primers (SEQ ID NOs 6 and 8) and an oligonucleotide whose sequence corresponds to that of the derivatized oligonucleotide. The presence of PCR products of the expected size in the samples equivalent to above samples 1 and 3 indicated that the derivatized oligonucleotide had been linked to mRNA

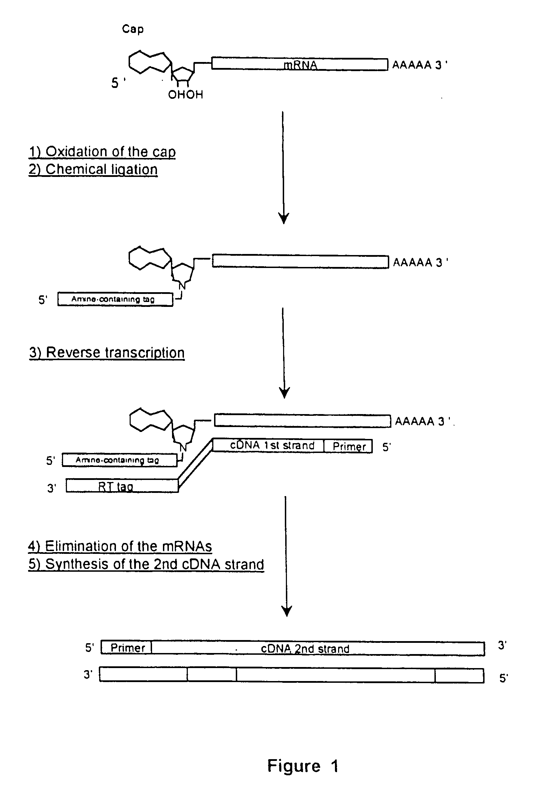

The above examples summarize the chemical procedure for enriching mRNAs for those having intact 5' ends as illustrated in Figure 1. Further detail regarding the chemical approaches for obtaining such mRNAs are disclosed in International Application No. WO96/34981, published November 7, 1996. Strategies based on the above chemical modifications to the 5' cap structure may be utilized to generate cDNAs selected to include the 5' ends of the mRNAs from which they derived. In one version of such procedures, the 5' ends of the mRNAs are modified as described above. Thereafter, a reverse transcription reaction is conducted to extend a primer complementary to the 5' end of the mRNA. Single stranded RNAs are eliminated to obtain a population of cDNA/mRNA heteroduplexes in which the mRNA includes an intact 5' end. The resulting heteroduplexes may be captured on a solid phase coated with a molecule capable of interacting with the molecule used to derivatize the 5' end of the mRNA. Thereafter, the strands of the heteroduplexes are separated to recover single stranded first cDNA strands which include the 5' end of the mRNA. Second strand cDNA synthesis may then proceed using conventional techniques. For example, the procedures disclosed in WO 96/34981 or in Carninci. *et al., Genomics* **37**:327-336, 1996 may be employed to select cDNAs which include the sequence derived from the 5' end of the coding sequence of the mRNA.

Following ligation of the oligonucleotide tag to the 5' cap of the mRNA, a reverse transcription reaction is conducted to extend a primer complementary to the mRNA to the 5' end of the mRNA. Following elimination of the RNA component of the resulting heteroduplex using standard techniques, second strand cDNA synthesis is conducted with a primer complementary to the oligonucleotide tag.

### 2. Enzyntatic Methods for Obtaining mRNAs having Intact 5' Ends

Other techniques for selecting cDNAs extending to the 5' end of the mRNA from which they are derived are fully enzymatic. Some versions of these techniques are disclosed in Dumas Milne Edwards J.B. (Doctoral Thesis of Paris VI University, Le clonage des ADNc complets: difficultes et perspectives nouvelles. Apports pour l'etude de la regulation de l'expression de la tryptophane hydroxylase de rat, 20 Dec. 1993), EP0 625572 and Kato *et al., Gene* **150**:243-250, 1994.

Briefly, in such approaches, isolated mRNA is treated with alkaline phosphatase to remove the phosphate groups present on the 5' ends of uncapped incomplete mRNAs. Following this procedure, the cap present on full length mRNAs is enzymatically removed with a decapping enzyme such as T4 polynucleotide kinase or tobacco acid pyrophosphatase. An oligonucleotide, which may be either a DNA oligonucleotide or a DNA-RNA hybrid oligonucleotide having RNA at its 3' end, is then ligated to the phosphate present at the 5' end of the decapped mRNA using T4 RNA ligase. The oligonucleotide may include a restriction site to facilitate cloning of the cDNAs following their synthesis. Example 12 below describes one enzymatic method based on the doctoral thesis of Dumas.

### EXAMPLE 12

### Enzymatic Approach for Obtaining 5' ESTs

Twenty micrograms of PolyA+ RNA were dephosphorylated using Calf Intestinal Phosphatase (Biolabs). After a phenol chloroform extraction, the cap structure of mRNA was hydrolysed using the Tobacco Acid Pyrophosphatase (purified as described by Shinshi *e*t *al.., Biochemistry* **15:** 2185-2190, 1976) and a hemi 5'DNA/RNA-3' oligonucleotide having an unphosphorylated 5' end, a stretch of adenosine ribophosphate at the 3' end, and an EcoRI site near the 5' end was ligated to the 5'P ends of mRNA using the T4 RNA ligase (Biolabs). Oligonucleotides suitable for use in this procedure are preferably 30 to 50 bases in length. Oligonucleotides having an unphosphorylated 5' end may be synthesized by adding a fluorochrome at the 5' end. The inclusion of a stretch of adenosine ribophosphates at the 3' end of the oligonucleotide increases ligation efficiency. It will be appreciated that the oligonucleotide may contain cloning sites other than EcoRI.

Following ligation of the oligonucleotide to the phosphate present at the 5' end of the decapped mRNA, first and second strand cDNA synthesis is carried out using conventional methods or those specified in EP0 625,572 and Kato *et al. supra,* and Dumas Milne Edwards, *supra,* the disclosures of which are incorporated herein by reference. The resulting cDNA may then be ligated into vectors such as those disclosed in Kato *et al., supra* or other nucleic acid vectors known to those skilled in the art using techniques such as those described in Sambrook *et al.,* Molecular Cloning: A Laboratory Manual 2d Ed., Cold Spring Harbor Laboratory Press, 1989.

### II. Obtention and Characterization of the 5' ESTs of the Present Invention

The 5' ESTs of the present invention were obtained using the aforementioned chemical and enzymatic approaches for enriching mRNAs for those having intact 5' ends as decribed below.

### 1. Obtention of 5' ESTS Using mRNAs with Intact 5' Ends

First, mRNAs were prepared as described in Example 13 below.

### EXAMPLE 13

### Preparation of mRNA With Intact 5' Ends

Total human RNAs or polyA⁺ RNAs derived from 29 different tissues were respectively purchased from LABIMO and CLONTECH and used to generate 44 cDNA libraries as follows. The purchased RNA had been isolated from cells or tissues using acid guanidium thiocyanate-phenol-chloroform extraction (Chomczyniski and Sacchi, *Analytical Biochemistry* **162**:156-159, 1987). PolyA⁺ RNA was isolated from total RNA (LABIMO) by two passes of oligo dT chromatography, as described by Aviv and Leder, *Proc. Natl. Acad. Sci. USA* **69**:1408-1412, 1972 in order to eliminate ribosomal RNA.

The quality and the integrity of the polyA+ RNAs were checked. Northern blots hybridized with a globin probe were used to confirm that the mRNAs were not degraded. Contamination of the polyA⁺ mRNAs by ribosomal sequences was checked using Northern blots and a probe derived from the sequence of the 28S rRNA. Preparations of mRNAs with less than 5% of rRNAs were used in library construction. To avoid constructing libraries with RNAs contaminated by exogenous sequences (prokaryotic or fungal), the presence of bacterial 16S ribosomal sequences or of two highly expressed fungal mRNAs was examined using PCR.

Following preparation of the mRNAs, the above described chemical and/or the enzymatic procedures for enriching mRNAs for thoses having intact 5' ends were employed to obtain 5' ESTs from various tissues. In both approaches, an oligonucleotide tag was attached to the 5' ends of the mRNAs. The oligonucleotide tag had an EcoRI site therein to facilitate later cloning procedures. To facilitate the processing of single stranded and double stranded cDNA obtained in the construction of the librairies, the same nucleotidic sequence was used to design the ligated oligonucleotide in both chemical and enzymatic approaches. Nevertheless, in the chemical procedure, the tag used was an oligodeoxyribonucleotide which was linked to the cap of the mRNA whereas in the enzymatic ligation, the tag was a chimeric hemi 5'DNAlRNA3' oligonucleotide which was ligated to the 5' end of decapped mRNA as described in example 12.

Following attachment of the oligonucleotide tag to the mRNA by either the chemical or enzymatic methods, the integrity of the mRNA was examined by performing a Northern blot with 200 to 500 ng of mRNA using a probe complementary to the oligonucleotide tag before performing the first strand synthesis as described in example 14.

### EXAMPLE 14

### cDNA Synthesis Using mRNA Templates Having Intact 5' Ends

For the mRNAs joined to oligonucleotide tags using both the chemical and enzymatic methods, first strand cDNA synthesis was performed using the Superscript II (Gibco BRL) or the Rnase H Minus M-MLV (Promega) reverse transcriptase with random nonamers as primers. In order to protect internal EcoRI sites in the cDNA from digestion at later steps in the procedure, methylated dCTP was used for first strand synthesis. After removal of RNA by an alkaline hydrolysis, the first strand of cDNA was precipitated using isopropanol in order to eliminate residual primers.

For both the chemical and the enzymatic methods, the second strand of the cDNA was synthesized with a Klenow fragment using a primer corresponding to the 5' end of the ligated oligonucleotide described in Example 12. Preferably, the primer is 20-25 bases in length. Methylated dCTP was also used for second strand synthesis in order to protect internal EcoRI sites in the cDNA from digestion during the cloning process.

Following cDNA synthesis, the cDNAs were cloned into pBlueScript as described in Example 15 below.

### EXAMPLE 15

### Cloning of cDNAsderived from mRNA with intact 5' ends into BlueScript

Following second strand synthesis, the ends of the cDNA were blunted with T4 DNA polymerase (Biolabs) and the cDNA was digested with EcoRI. Since methylated dCTP was used during cDNA synthesis, the EcoRI site present in the tag was the only hemi-methylated site, hence the only site susceptible to EcoRI digestion. The cDNA was then size fractionated using exclusion chromatography (AcA, Biosepra) and fractions corresponding to cDNAs of more than 150 bp were pooled and ethanol precipitated. The cDNA was directionally cloned into the SmaI and EcoRI ends of the phagemid pBlueScript vector (Stratagene). The ligation mixture was electroporated into bacteria and propagated under appropriate antibiotic selection.

Clones containing the oligonucleotide tag attached were then selected as described in Example 16 below.

### EXAMPLE 16

### Selection of Clones Having the Oligonucleotide Tag Attached Thereto

The plasmid DNAs containing 5' EST libraries made as described above were purified (Qiagen). A positive selection of the tagged clones was performed as follows. Briefly, in this selection procedure, the plasmid DNA was converted to single stranded DNA using gene II endonuclease of the phage F1 in combination with an exonuclease (Chang *et al., Gene* **127**:95-8, 1993) such as exonuclease III or T7 gene 6 exonuclease. The resulting single stranded DNA was then purified using paramagnetic beads as described by Fry *et al., Biotechniques,* **13:** 124-131, 1992. In this procedure, the single stranded DNA was hybridized with a biotinylated oligonucleotide having a sequence corresponding to the 3' end of the oligonucleotide described in Example 13, Preferably, the primer has a length of 20-25 bases. Clones including a sequence complementary to the biotinylated oligonucleotide were captured by incubation with streptavidin coated magnetic beads followed by magnetic selection. After capture of the positive clones, the plasmid DNA was released from the magnetic beads and converted into double stranded DNA using a DNA polymerase such as the ThermoSequenase obtained from Amersham Pharmacia Biotech. Alternatively, protocoles such as the one described in the Gene Trapper kit available from Gibco BRL may be used. The double stranded DNA was then electroporated into bacteria. The percentage of positive clones having the 5' tag oligonucleotide was estimated to typically rank between 90 and 98% using dot blot analysis.

Following electroporation, the libraries were ordered in 384-microtiter plates (MTP). A copy of the MTP was stored for future needs. Then the libraries were transferred into 96 MTP and sequenced as described below.

### EXAMPLE 17

### Sequencing of Inserts in Selected Clones

Plasmid inserts were first amplified by PCR on PE 9600 thermocyclers (Perkin-Elmer, Applied Biosystems Division, Foster City, CA), using standard SETA-A and SETA-B primers (Genset SA), AmpliTaqGold (Perkin-Elmer), dNTPs (Boehringer), buffer and cycling conditions as recommended by the Perkin-Elmer Corporation.

PCR products were then sequenced using automatic ABI Prism 377 sequencers (Perkin Elmer). Sequencing reactions were performed using PE 9600 thermocyclers with standard dye-primer chemistry and ThermoSequenase (Amersham Pharmacia Biotech). The primers used were either T7 or 21M13 (available from Genset SA) as appropriate. The primers were labeled with the JOE, FAM, ROX and TAMRA dyes. The dNTPs and ddNTPs used in the sequencing reactions were purchased from Boehringer. Sequencing buffer, reagent concentrations and cycling conditions were as recommended by Amersham.

Following the sequencing reaction, the samples were precipitated with ethanol, resuspended in formamide loading buffer, and loaded on a standard 4% acrylamide gel. Electrophoresis was performed for 2.5 hours at 3000V on an ABI 377 sequencer, and the sequence data were collected and analyzed using the ABI Prism DNA Sequencing Analysis Software, version 2.1.2.

### 2. Computer analysis of the Obtained 5' ESTs: Construction of NetGene and SignalTag databases

The sequence data from the 44 cDNA libraries made as described above were transferred to a proprietary database, where quality control and validation steps were performed. A proprietary base-caller, working using a Unix system, automatically flagged suspect peaks, taking into account the shape of the peaks, the inter-peak resolution, and the noise level. The proprietary base-caller also performed an automatic trimming. Any stretch of 25 or fewer bases having more than 4 suspect peaks was considered unreliable and was discarded. Sequences corresponding to cloning vector or ligation oligonucleotides were automatically removed from the EST sequences. However, the resulting EST sequences may contain 1 to 5 bases belonging to the above mentioned sequences at their 5' end. If needed, these can easily be removed on a case to case basis.

Following sequencing as described above, the sequences of the 5' ESTs were entered in NetGene^{™}, a proprietary database called for storage and manipulation as described below. It will be appreciated by those skilled in the art that the data could be stored and manipulated on any medium which can be read and accessed by a computer. Computer readable media include magnetically, optically, or electronically readable media. For example, the computer readable media may be a hard disc, a floppy disc, a magnetic tape, CD-ROM, RAM, or ROM as well as other types of other media known to those skilled in the art.

In addition, the sequence data may be stored and manipulated in a variety of data processor programs in a diversity of formats. For instance, the sequence data may be stored as text in a word processing file, such as Microsoft WORD or WORDPERFECT or as an ASCII file in a variety of database programs familiar to those of skill in the art, such as DB2, SYBASE, or ORACLE.

The computer readable media on which the sequence information is stored may be in a personal computer, a network, a server or other computer systems known to those skilled in the art. The computer or other system preferably includes the storage media described above, and a processor for accessing and manipulating the sequence data. Once the sequence data has been stored, it may be manipulated and searched to locate those stored sequences which contain a desired nucleic acid sequence or which encode a protein having a particular functional domain. For example, the stored sequence information may be compared to other known sequences to identify homologies, motifs implicated in biological function, or structural motifs.

Programs which may be used to search or compare the stored sequences include the MacPattern (EMBL), BLAST, and BLAST2 program series (NCBI), basic local alignment search tool programs for nucleotide (BLASTN) and peptide (BLASTX) comparisons (Altschul *et al, J. Mol. Biol.* **215**: 403, 1990) and FASTA (Pearson and Lipman, *Proc. Natl. Acad. Sci. USA* **85**: 2444, 1988). The BLAST programs then extend the alignments on the basis of defined match and mismatch criteria.

Motifs which may be detected using the above programs and those described in Example 28 include sequences encoding leucine zippers, helix-turn-helix motifs, glycosylation sites, ubiquitination sites, alpha helices, and beta sheets, signal sequences encoding signal peptides which direct the secretion of the encoded proteins, sequences implicated in transcription regulation such as homeoboxes, acidic stretches, enzymatic active sites, substrate binding sites, and enzymatic cleavage sites.

Before searching the cDNAs in the NetGene^{™} database for sequence motifs of interest, cDNAs derived from mRNAs which were not of interest were identified and eliminated from further consideration as described in Example 18 below.

### EXAMPLE 18

### Elimination of Undesired Sequences from Further Consideration

5' ESTs in the NetGene^{™} database which were derived from undesired sequences such as transfer RNAs, ribosomal RNAs, mitochondrial RNAs, prokaryotic RNAs, fungal RNAs, Alu sequences, L1 sequences, or repeat sequences were identified using the FASTA and BLASTN programs with the parameters listed in Table I.

To eliminate 5' ESTs encoding tRNAs from further consideration, the 5' EST sequences were compared to the sequences of 1190 known tRNAs obtained from EMBL release 38, of which 100 were human. The comparison was performed using FASTA on both strands of the 5' ESTs. Sequences having more than 80% homology over more than 60 nucleotides were identified as tRNA. Of the 144 841 sequences screened, 26 were identified as tRNAs and eliminated from further consideration.

To eliminate 5' ESTs encoding rRNAs from further consideration, the 5' EST sequences were compared to the sequences of 2497 known rRNAs obtained from EMBL release 38, of which 73 were human. The comparison was performed using BLASTN on both strands of the 5' ESTs with the parameter S=108. Sequences having more than 80% homology over stretches longer than 40 nucleotides were identified as rRNAs. Of the 144,341 sequences screened, 3,312 were identified as rRNAs and eliminated from further consideration.

To eliminate 5' ESTs encoding mtRNAs from further consideration, the 5' EST sequences were compared to the sequences of the two known mitochondrial genomes for which the entire genomic sequences are available and all sequences transcribed from these mitochondrial genomes including tRNAs, rRNAs, and mRNAs for a total of 38 sequences. The comparison was performed using BLASTN on both strands of the 5' ESTs with the parameter S=108. Sequences having more than 80% homology over stretches longer than 40 nucleotides were identified as mtRNAs. Of the 144,341 sequences screened, 6,110 were identified as mtRNAs and eliminated from further consideration.

Sequences which might have resulted from exogenous contaminants were eliminated from further consideration by comparing the 5' EST sequences to release 46 of the EMBL bacterial and fungal divisions using BLASTN with the parameter S=144. All sequences having more than 90% homology over at least 40 nucleotides were identified as exogenous contaminants. Of the 42 cDNA libraries examined, the average percentages of prokaryotic and fungal sequences contained therein were 0.2% and 0.5% respectively. Among these sequences, only one could be identified as a sequence specific to fungi. The others were either fungal or prokaryotic sequences having homologies with vertebrate sequences or including repeat sequences which had not been masked during the electronic comparison.

In addition, the 5' ESTs were compared to 6093 Alu sequences and 1115 L1 sequences to mask 5' ESTs containing such repeat sequences. 5' ESTs including THE and MER repeats, SSTR sequences or satellite, micro-satellite, or telomeric repeats were also eliminated from further consideration. On average, 11.5% of the sequences in the libraries contained repeat sequences. Of this 11.5%, 7% contained Alu repeats, 3.3% contained L1 repeats and the remaining 1.2% were derived from the other screened types of repetitive sequences. These percentages are consistent with those found in cDNA libraries prepared by other groups. For example, the cDNA libraries of Adams *et al*. contained between 0% and 7.4% Alu repeats depending on the source of the RNA which was used to prepare the cDNA library *(Adams et al., Nature* **377**:174, 1996).

The sequences of those 5' ESTs remaining after the elimination of undesirable sequences were compared with the sequences of known human mRNAs to determine the accuracy of the sequencing procedures described above.

### EXAMPLE 19

### Measurement of Sequencing Accuracy by Comparison to Known Sequences

To further determine the accuracy of the sequencing procedure described above, the sequences of 5' ESTs derived from known sequences were identified and compared to the original known sequences. First, a FASTA analysis with overhangs shorter than 5 bp on both ends was conducted on the 5' ESTs to identify those matching an entry in the public human mRNA database. The 6655 5' ESTs which matched a known human mRNA were then realigned with their cognate mRNA and dynamic programming was used to include substitutions, insertions, and deletions in the list of "errors" which would be recognized. Errors occurring in the last 10 bases of the 5' EST sequences were ignored to avoid the inclusion of spurious cloning sites in the analysis of sequencing accuracy.

This analysis revealed that the sequences incorporated in the NetGene^{™} database had an accuracy of more than 99.5%.

To determine the efficiency with which the above selection procedures select cDNAs which include the 5' ends of their corresponding mRNAs, the following analysis was performed.

### EXAMPLE 20

### Determination of Efficiency of 5' EST Selection

To determine the efficiency at which the above selection procedures isolated 5' ESTs which included sequences close to the 5' end of the mRNAs from which they derived, the sequences of the ends of the 5' ESTs derived from the elongation factor 1 subunit α and ferritin heavy chain genes were compared to the known cDNA sequences of these genes. Since the transcription start sites of both genes are well characterized, they may be used to determine the percentage of derived 5' ESTs which included the authentic transcription start sites.

For both genes, more than 95% of the obtained 5' ESTs actually included sequences close to or upstream of the 5' end of the corresponding mRNAs.

To extend the analysis of the reliability of the procedures for isolating 5' ESTs from ESTs in the NetGene^{™} database, a similar analysis was conducted using a database composed of human mRNA sequences extracted from GenBank database release 97 for comparison. The 5' ends of more than 85% of 5' ESTs derived from mRNAs included in the GeneBank database were located close to the 5' ends of the known sequence. As some of the mRNA sequences available in the GenBank database are deduced from genomic sequences, a 5' end matching with these sequences will be counted as an internal match. Thus, the method used here underestimates the yield of ESTs including the authentic 5' ends of their corresponding mRNAs.

The EST libraries made above included multiple 5' ESTs derived from the same mRNA. The sequences of such 5' ESTs were compared to one another and the longest 5' ESTs for each mRNA were identified. Overlapping cDNAs were assembled into continuous sequences (contigs). The resulting continuous sequences were then compared to public databases to gauge their similarity to known sequences, as described in Example 21 below.

### EXAMPLE 21

### Clustering of the 5' ESTs and Calculation of Novelty Indices for cDNA Libraries

For each sequenced EST library, the sequences were clustered by the 5' end. Each sequence in the library was compared to the others with BLASTN2 (direct strand, parameters S=107). ESTs with High Scoring Segment Pairs (HSPs) at least 25 bp long, having 95% identical bases and beginning closer than 10 bp from each EST 5' end were grouped. The longest sequence found in the cluster was used as representative of the group. A global clustering between libraries was then performed leading to the definition of super-contigs.

To assess the yield of new sequences within the EST libraries, a novelty rate (NR) was defined as: NR= 100 X (Number of new unique sequences found in the library/Total number of sequences from the library). Typically, novelty rating ranged between 10% and 41% depending on the tissue from which the EST library was obtained. For most of the libraries, the random sequencing of 5' EST libraries was pursued until the novelty rate reached 20%.

Following characterization as described above, the collection of 5' ESTs in NetGene^{™} was screened to identify those 5' ESTs bearing potential signal sequences as described in Example 22 below.

### EXAMPLE 22

### Identification of Potential Signal Sequences in 5' ESTs

The 5' ESTs in the NetGene^{™} database were screened to identify those having an uninterrupted open reading frame (ORF) longer than 45 nucleotides beginning with an ATG codon and extending to the end of the EST. Approximately half of the cDNA sequences in NetGene^{™} contained such an ORF. The ORFs of these 5' ESTs were then searched to identify potential signal motifs using slight modifications of the procedures disclosed in Von Heijne, *Nucleic Acids Res.* **14**:4683-4690, 1986. Those 5' EST sequences encoding a stretch of at least 15 amino acid long with a score of at least 3.5 in the Von Heijne signal peptide identification matrix were considered to possess a signal sequence. Those 5' ESTs which matched a known human mRNA or EST sequence and had a 5' end more than 20 nucleotides downstream of the known 5' end were excluded from further analysis. The remaining cDNAs having signal sequences therein were included in a database called SignalTag™.

To confirm the accuracy of the above method for identifying signal sequences, the analysis of Example 23 was performed.

### EXAMPLE 23

### Confirmation of Accuracy of Identification of Potential Signal Sequences in 5' ESTs

The accuracy of the above procedure for identifying signal sequences encoding signal peptides was evaluated by applying the method to the 43 amino acids located at the N terminus of all human SwissProt proteins. The computed Von Hcijne score for each protein was compared with the known characterization of the protein as being a secreted protein or a non-secreted protein. In this manner, the number of non-secreted proteins having a score higher than 3.5 (false positives) and the number of secreted proteins having a score lower than 3.5 (false negatives) could be calculated.

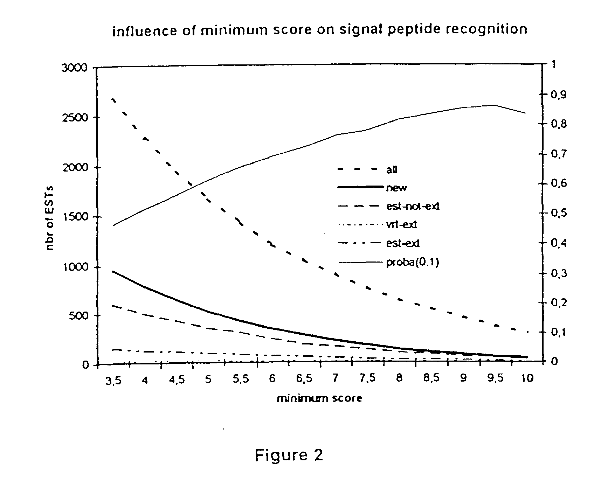

Using the results of the above analysis, the probability that a peptide encoded by the 5' region of the mRNA is in fact a genuine signal peptide based on its Von Heijne's score was calculated based on either the assumption that 10 % of human proteins are secreted or the assumption that 20 % of human proteins are secreted. The results of this analysis are shown in Figure 2 and in table IV.

Using the above method of identification of secretory proteins, 5' ESTs of the following polypeptides known to be secreted were obtained: human glucagon, gamma interferon induced monokine precursor, secreted cyclophilin-like protein, human pleiotropin, and human biotinidase precursor. Thus, the above method successfully identified those 5' ESTs which encode a signal peptide.

To confirm that the signal peptide encoded by the 5' ESTs actually functions as a signal peptide, the signal sequences from the 5' ESTs may be cloned into a vector designed for the identification of signal peptides. Such vectors are designed to confer the ability to grow in selective medium only to host cells containing a vector with an operably linked signal sequence. For example, to confirm that a 5' EST encodes a genuine signal peptide, the signal sequence of the 5' EST may be inserted upstream and in frame with a non-secreted form of the yeast invertase gene in signal peptide selection vectors such as those described in U.S. Patent No. 5,536,637. Growth of host cells containing signal sequence selection vectors with the correctly inserted 5' EST signal sequence confirms that the 5' EST encodes a genuine signal peptide.

Alternatively, the presence of a signal peptide may be confirmed by cloning the extended cDNAs obtained using the ESTs into expression vectors such as pXT1 (as described below in example 30), or by constructing promoter-signal sequence-reporter gene vectors which encode fusion proteins between the signal peptide and an assayable reporter protein. After introduction of these vectors into a suitable host cell, such as COS cells or NIH 3T3 cells, the growth medium may be harvested and analyzed for the presence of the secreted protein. The medium from these cells is compared to the medium from control cells containing vectors lacking the signal sequence or extended cDNA insert to identify vectors which encode a functional signal peptide or an authentic secreted protein.

Those 5' ESTs which encoded a signal peptide, as determined by the method of Example 22 above, were further grouped into four categories based on their homology to known sequences as described in Example 24 below.

### EXAMPLE 24

### Categorization of 5' ESTs Encoding a Signal Peptide

Those 5' ESTs having a sequence not matching any known vertebrate sequence nor any publicly available EST sequence were designated "new." Of the sequences in the SignalTag™ database, 947 of the 5' ESTs having a Von Heijne's score of at least 3.5 fell into this category.

Those 5' ESTs having a sequence not matching any vertebrate sequence but matching a publicly known EST were designated "EST-ext", provided that the known EST sequence was extended by at least 40 nucleotides in the 5' direction. Of the sequences in the SignalTag™ database, 150 of the 5' ESTs having a Von Heijne's score of at least 3.5 fell into this category.

Those ESTs not matching any vertebrate sequence but matching a publicly known EST without extending the known EST by at least 40 nucleotides in the 5' direction were designated "EST." Of the sequences in the SignalTag™ database, 599 of the 5' ESTs having a Von Heijne's score of at least 3.5 fell into this category.

Those 5' ESTs matching a human mRNA sequence but extending the known sequence by at least 40 nucleotides in the 5' direction were designated "VERT-ext." Of the sequences in the SignalTag™ database, 23 of the 5' ESTs having a Von Heijne's score of at least 3.5 fell into this category. Included in this category was a 5' EST which extended the known sequence of the human translocase mRNA by more than 200 bases in the 5' direction. A 5' EST which extended the sequence of a human tumor suppressor gene in the 5' direction was also identified.

Table V shows the distribution of 5' ESTs in each category and the number of 5' ESTs in each category having a given minimum von Heijne's score.

### 3. Evaluation of Spatial and Temporal Expression of mRNAs Corresponding to the 5'ESTs or Extended cDNAs

Each of the 5' ESTs was also categorized based on the tissue from which its corresponding mRNA was obtained, as described below in Example 25.

### EXAMPLE 25

### Categorization of Expression Patterns

Table VI shows the distribution of 5' ESTs in each of the above defined category with respect to the tissue from which the 5'ESTs of the corresponding mRNA were obtained.

Table II provides the sequence identification numbers of 5' EST sequences derived from different tissues, the categories in which these sequences fall, and the von Heijne's score of the signal peptides which they encode. The 5' EST sequences and the amino acid sequences they encode are provided in the appended sequence listings. Table III provides the sequence ID numbers of the 5' ESTs and the sequences of the signal peptides which they encode. The sequences of the 5' ESTs and the polypeptides they encode are provided in the sequence listing appended hereto.

The sequences of DNA SEQ ID NOs: 38-291 can readily be screened for any errors therein and any sequence ambiguities can be resolved by resequencing a fragment containing such errors or amibiguities on both strands. Such fragments may be obtained from the plasmids stored in the inventors' laboratory or can be isolated using the techniques described herein. Resolution of any such ambiguities or errors may be facilitated by using primers which hybridize to sequences located close to the ambiguous or erroneous sequences. For example, the primers may hybridize to sequences within 50-75 bases of the amibiguity or error. Upon resolution of an error or ambiguity, the corresponding corrections can be made in the protein sequences encoded by the DNA containing the error or amibiguity.

In addition to categorizing the 5' ESTs with respect to their tissue of origin, the spatial and temporal expression patterns of the mRNAs corresponding to the 5' ESTs, as well as their expression levels, may be determined as described in Example 26 below. Characterization of the spatial and temporal expression patterns and expression levels of these mRNAs is useful for constructing expression vectors capable of producing a desired level of gene product in a desired spatial or temporal manner, as will be discussed in more detail below.

Furthermore, 5' ESTs whose corresponding mRNAs are associated with disease states may also be identified. For example, a particular disease may result from the lack of expression, over expression, or under expression of an mRNA corresponding to a 5' EST. By comparing mRNA expression patterns and quantities in samples taken from healthy individuals with those from individuals suffering from a particular disease, 5' ESTs responsible for the disease may be identified.

It will be appreciated that the results of the above characterization procedures for 5' ESTs also apply to extended cDNAs (obtainable as described below) which contain sequences adjacent to the 5' ESTs. It will also be appreciated that if desired, characterization may be delayed until extended cDNAs have been obtained rather than characterizing the ESTs themselves.

### EXAMPLE 26

### Evaluation of Expression Levels and Patterns of mRNAs Corresponding to 5' ESTs or Extended cDNAs

Expression levels and patterns of mRNAs corresponding to 5' ESTs or extended cDNAs (obtainable as described below in example 27) may be analyzed by solution hybridization with long probes as described in International Patent Application No. WO 97/05277. Briefly, a 5' EST, extended cDNA, or fragment thereof corresponding to the gene encoding the mRNA to be characterized is inserted at a cloning site immediately downstream of a bacteriophage (T3, T7 or SP6) RNA polymerase promoter to produce antisense RNA Preferably, the 5' EST or extended cDNA has 100 or more nucleotides. The plasmid is linearized and transcribed in the presence of ribonucleotides comprising modified ribonucleotides (*i.e.* biotin-UTP and DIG-UTP). An excess of this doubly labeled RNA is hybridized in solution with mRNA isolated from cells or tissues of interest. The hybridizations are performed under standard stringent conditions (40-50°C for 16 hours in an 80% formamide, 0.4 M NaCl buffer, pH 7-8). The unhybridized probe is removed by digestion with ribonucleases specific for single-stranded RNA (*i.e.* RNases CL3, T1, Phy M, U2 or A). The presence of the biotin-UTP modification enables capture of the hybrid on a microtitration plate coated with streptavidin. The presence of the DIG modification enables the hybrid to be detected and quantified by ELISA using an anti-DIG antibody coupled to alkaline phosphatase.

The 5' ESTs, extended cDNAs, or fragments thereof may also be tagged with nucleotide sequences for the serial analysis of gene expression (SAGE) as disclosed in UK Patent Application No. 2 305 241 A. In this method, cDNAs are prepared from a cell, tissue, organism or other source of nucleic acid for which gene expression patterns must be determined. The resulting cDNAs are separated into two pools. The cDNAs in each pool are cleaved with a first restriction endonuclease, called an anchoring enzyme, having a recognition site which is likely to be present at least once in most cDNAs. The fragments which contain the 5' or 3' most region of the cleaved cDNA are isolated by binding to a capture medium such as streptavidin coated beads. A first oligonucleotide linker having a first sequence for hybridization of an amplification primer and an internal restriction site for a so-called tagging endonuclease is ligated to the digested cDNAs in the first pool. Digestion with the second endonuclease produces short tag fragments from the cDNAs.

A second oligonucleotide having a second sequence for hybridization of an amplification primer and an internal restriction site is ligated to the digested cDNAs in the second pool. The cDNA fragments in the second pool are also digested with the tagging endonuclease to generate short tag fragments derived from the cDNAs in the second pool. The tags resulting from digestion of the first and second pools with the anchoring enzyme and the tagging endonuclease are ligated to one another to produce so-called ditags. In some embodiments, the ditags are concatamerized to produce ligation products containing from 2 to 200 ditags. The tag sequences are then determined and compared to the sequences of the 5' ESTs or extended cDNAs to determine which 5' ESTs or extended cDNAs are expressed in the cell, tissue, organism, or other source of nucleic acids from which the tags were derived. In this way, the expression pattern of the 5' ESTs or extended cDNAs in the cell, tissue, organism, or other source of nucleic acids is obtained.

Quantitative analysis of gene expression may also be performed using arrays. As used herein, the term array means a one dimensional, two dimensional, or multidimensional arrangement of full length cDNAs (*i.e*. extended cDNAs which include the coding sequence for the signal peptide, the coding sequence for the mature protein, and a stop codon), extended cDNAs, 5' ESTs or fragments thereof of sufficient length to permit specific detection of gene expression. Preferably, the fragments are at least 15 nucleotides in length. More preferably, the fragments are at least 100 nucleotide long. More preferably, the fragments are more than 100 nucleotides in length. In some embodiments, the fragments may be more than 500 nucleotide long.

For example, quantitative analysis of gene expression may be performed with full length cDNAs as defined below, extended cDNAs, 5' ESTs, or fragments thereof in a complementary DNA microarray as described by Schena *et al. (Science* **270**:467-470, 1995; *Proc. Natl. Acad Sci. U.S.A.* **93**:10614-10619, 1996). Full length cDNAs, extended cDNAs, 5' ESTs or fragments thereof are amplified by PCR and arrayed from 96-well microtiter plates onto silylated microscope slides using high-speed robotics. Printed arrays are incubated in a humid chamber to allow rehydration of the array elements and rinsed, once in 0.2% SDS for 1 min, twice in water for 1 min and once for 5 min in sodium borohydride solution. The arrays are submerged in water for 2 min at 95°C, transferred into 0.2% SDS for 1 min, rinsed twice with water, air dried and stored in the dark at 25°C.

Cell or tissue mRNA is isolated or commercially obtained and probes are prepared by a single round of reverse transcription. Probes are hybridized to 1 cm² microarrays under a 14 x 14 mm glass coverslip for 6-12 hours at 60°C. Arrays are washed for 5 min at 25°C in low stringency wash buffer (1 x SSC/0.2% SDS), then for 10 min at room temperature in high stringency wash buffer (0.1 x SSC/0.2% SDS). Arrays are scanned in 0.1 x SSC using a fluorescence laser scanning device fitted with a custom filter set. Accurate differential expression measurements are obtained by taking the average of the ratios of two independent hybridizations.

Quantitative analysis of the expression of genes may also be performed with full length cDNAs, extended cDNAs, 5' ESTs, or fragments thereof in complementary DNA arrays as described by Pietu *et al. (Genome Research* **6**:492-503, 1996). The full length cDNAs, extended cDNAs, 5' ESTs or fragments thereof are PCR amplified and spotted on membranes. Then, mRNAs originating from various tissues or cells are labeled with radioactive nucleotides. After hybridization and washing in controlled conditions, the hybridized mRNAs are detected by phospho-imaging or autoradiography. Duplicate experiments are performed and a quantitative analysis of differentially expressed mRNAs is then performed.

Alternatively, expression analysis of the 5' ESTs or extended cDNAs can be done through high density nucleotide arrays as described by Lockhart *et al.* (*Nature Biotechnology* **14**: 1675-1680, 1996) and Sosnowsky *et al. (Proc. Natl. Acad. Sci.* **94**:1119-1123, 1997). Oligonucleotides of 15-50 nucleotides corresponding to sequences of the 5' ESTs or extended cDNAs are synthesized directly on the chip (Lockhart *et al., supra*) or synthesized and then addressed to the chip (Sosnowsky *et al., supra*)*.* Preferably, the oligonucleotides are about 20 nucleotides in length.

cDNA probes labeled with an appropriate compound, such as biotin, digoxigenin or fluorescent dye, are synthesized from the appropriate mRNA population and then randomly fragmented to an average size of 50 to 100 nucleotides. The said probes are then hybridized to the chip. After washing as described in Lockhart *et al, supra* and application of different electric fields (Sonowsky et *al, supra.),* the dyes or labeling compounds are detected and quantified. Duplicate hybridizations are performed. Comparative analysis of the intensity of the signal originating from cDNA probes on the same target oligonucleotide in different cDNA samples indicates a differential expression of the mRNA corresponding to the 5' EST or extended cDNA from which the oligonucleotide sequence has been designed.

### III. Use of 5' ESTs to Clone Extended cDNAs and to Clone the Corresponding Genomic DNAs

Once 5' ESTs which include the 5' end of the corresponding mRNAs have been selected using the procedures described above, they can be utilized to isolate extended cDNAs which contain sequences adjacent to the 5' ESTs. The extended cDNAs may include the entire coding sequence of the protein encoded by the corresponding mRNA, including the authentic translation start site, the signal sequence, and the sequence encoding the mature protein remaining after cleavage of the signal peptide. Such extended cDNAs are referred to herein as "full length cDNAs." Alternatively, the extended cDNAs may include only the sequence encoding the mature protein remaining after cleavage of the signal peptide, or only the sequence encoding the signal peptide.

Example 27 below describes a general method for obtaining extended cDNAs using 5' ESTs. Example 28 below provides experimental results, using the method explained in example 27, describing several extended cDNAs including the entire coding sequence and authentic 5' end of the corresponding mRNA for several secreted proteins.

The methods of Examples 27, 28, and 29 can also be used to obtain extended cDNAs which encode less than the entire coding sequence of the secreted proteins encoded by the genes corresponding to the 5' ESTs. In some embodiments, the extended cDNAs isolated using these methods encode at least 10 amino acids of one of the proteins encoded by the sequences of SEQ ID NOs: 38-291. In further embodiments, the extended cDNAs encode at least 20 amino acids of the proteins encoded by the sequences of SEQ ID NOs: 38-291. In further embodiments, the extended cDNAs encode at least 30 amino amino acids of the sequences of SEQ ID NOs: 38-291. In a preferred embodiment, the extended cDNAs encode a full length protein sequence, which includes the protein coding sequences of SEQ ID NOs: 38-291.

### EXAMPLE 27

### General Method for Using 5'ESTs to Clone and Sequence cDNAs which Include the Entire Coding Region and the Authentic 5' End of the Corresponding mRNA

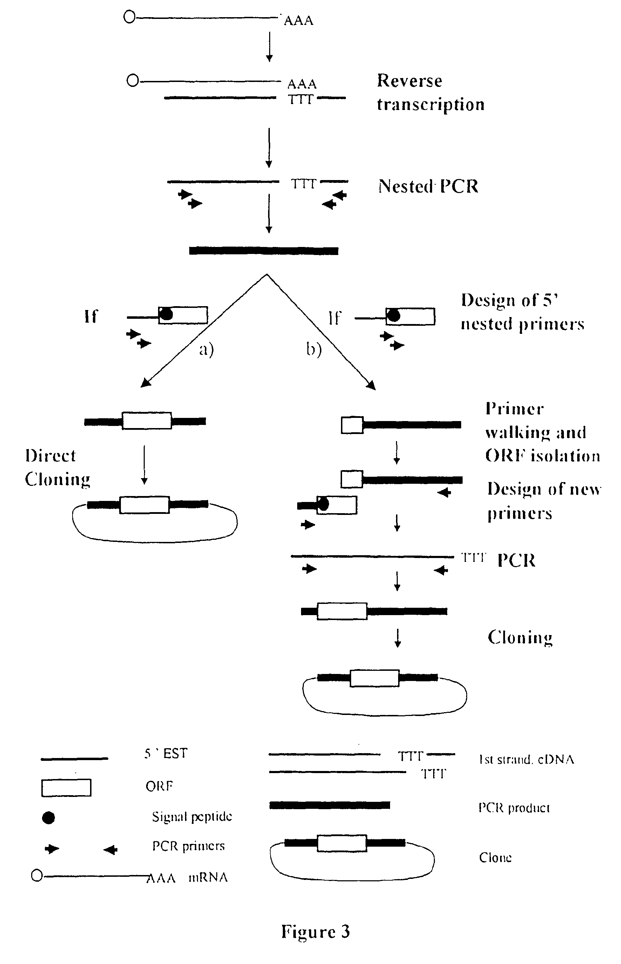

The following general method has been used to quickly and efficiently isolate extended cDNAs having the authentic 5' ends of their corresponding mRNAs as well as the full protein coding sequence and including sequence adjacent to the sequences of the 5' ESTs used to obtain them. This method may be applied to obtain extended cDNAs for any 5' EST in the NetGene^{™} database, including those 5' ESTs encoding polypeptides belonging to secreted proteins. The method is summarized in figure 3.

### 1. Obtention of Extended cDNAs

### a) First strand synthesis

The method takes advantage of the known 5' sequence of the mRNA. A reverse transcription reaction is conducted on purified mRNA with a poly 14dT primer containing a 49 nucleotide sequence at its 5' end allowing the addition of a known sequence at the end of the cDNA which corresponds to the 3' end of the mRNA. For example, the primer may have the following sequence: 5'-ATC GTT GAG ACT CGT ACC AGC AGA GTC ACG AGA GAG ACT ACA CGG TAC TGG TTT TTT TTT TTT TTVN -3' (SEQ ID NO: 14). Those skilled in the art will appreciate that other sequences may also be added to the poly dT sequence and used to prime the first strand synthesis. Using this primer and a reverse transcriptase such as the Superscript II (Gibco BRL) or Rnase H Minus M-MLV (Promega) enzyme, a reverse transcript anchored at the 3' polyA site of the RNAs is generated.

After removal of the mRNA hybridized to the first cDNA strand by alkaline hydrolysis, the products of the alkaline hydrolysis and the residual poly dT primer are eliminated with an exclusion column such as an AcA34 (Biosepra) matrix as explained in Example 11.

### b) Second strand synthesis

A pair of nested primers on each end is designed based on the known 5' sequence from the 5' EST and the known 3' end added by the poly dT primer used in the first strand synthesis. Softwares used to design primers are either based on GC content and melting temperatures of oligonucleotides, such as OSP (Illier and Green, *PCR Meth.-Appl.* **1**: 124-128, 1991), or based on the octamer frequency disparity method (Griffais *et al., Nucleic Acids Res.* **19**: 3887-3891, 1991) such as PC-Rare (http://bioinformatics.weizmann.ac.il/software/PC-Rare/doc/manuel.html).

Preferably, the nested primers at the 5' end are separated from one another by four to nine bases. The 5' primer sequences may be selected to have melting temperatures and specificities suitable for use in PCR.

Preferably, the nested primers at the 3' end are separated from one another by four to nine bases. For example, the nested 3' primers may have the following sequences: (5'- CCA GCA GAG TCA CGA GAG AGA CTA CAC GG -3'(SEQ ID NO: 15), and 5'- CAC GAG AGA GAC TAC ACG GTA CTG G -3' (SEQ ID NO:16). These primers were selected because they have melting temperatures and specificities compatible with their use in PCR. However, those skilled in the art will appreciate that other sequences may also be used as primers.

The first PCR run of 25 cycles is performed using the Advantage Tth Polymerase Mix (Clontech) and the outer primer from each of the nested pairs. A second 20 cycle PCR using the same enzyme and the inner primer from each of the nested pairs is then performed on 1/2500 of the first PCR product. Thereafter, the primers and nucleotides are removed.

### 2. Sequencing of Full Length Extended cDNAs or Fragments Thereof

Due to the lack of position constraints on the design of 5' nested primers compatible for PCR use using the OSP software, amplicons of two types are obtained. Preferably, the second 5' primer is located upstream of the translation initiation codon thus yielding a nested PCR product containing the whole coding sequence. Such a full length extended cDNA undergoes a direct cloning procedure as described in section a. However, in some cases, the second 5' primer is located downstream of the translation initiation codon, thereby yielding a PCR product containing only part of the ORF. Such incomplete PCR products are submitted to a modified procedure described in section b.

### a) Nested PCR products containing complete ORFs

When the resulting nested PCR product contains the complete coding sequence, as predicted from the 5'EST sequence, it is cloned in an appropriate vector such as pED6dpc2, as described in section 3.

### b) Nested PCR products containing incomplete ORFs

When the amplicon does not contain the complete coding sequence, intermediate steps are necessary to obtain both the complete coding sequence and a PCR product containing the full coding sequence. The complete coding sequence can be assembled from several partial sequences determined directly from different PCR products as described in the following section.

Once the full coding sequence has been completely determined, new primers compatible for PCR use are designed to obtain amplicons containing the whole coding region. However, in such cases, 3' primers compatible for PCR use are located inside the 3' UTR of the corresponding mRNA, thus yielding amplicons which lack part of this region, *i.e.* the polyA tract and sometimes the polyadenylation signal, as illustrated in figure 3. Such full length extended cDNAs are then cloned into an appropriate vector as described in section 3.

### c) Sequencing extended cDNAs

Sequencing of extended cDNAs is performed using a Die Terminator approach with the AmpliTaq DNA polymerase FS kit available from Perkin Elmer.

In order to sequence PCR fragments, primer walking is performed using software such as OSP to choose primers and automated computer software such as ASMG (Sutton *et al., Genome Science Technol.* **1**: 9-19, 1995) to construct contigs of walking sequences including the initial 5' tag using minimum overlaps of 32 nucleotides. Preferably, primer walking is performed until the sequences of full length cDNAs are obtained.

Completion of the sequencing of a given extended cDNA fragment is assessed as follows. Since sequences located after a polyA tract are difficult to determine precisely in the case of uncloned products, sequencing and primer walking processes for PCR products are interrupted when a polyA tract is identified in extended cDNAs obtained as described in case b. The sequence length is compared to the size of the nested PCR product obtained as described above. Due to the limited accuracy of the determination of the PCR product size by gel electrophoresis, a sequence is considered complete if the size of the obtained sequence is at least 70 % the size of the first nested PCR product. If the length of the sequence determined from the computer analysis is not at least 70 % of the length of the nested PCR product, these PCR products are cloned and the sequence of the insertion is determined. When Northern blot data are available, the size of the mRNA detected for a given PCR product is used to finally assess that the sequence is complete. Sequences which do not fulfill the above criteria are discarded and will undergo a new isolation procedure.

Sequence data of all extended cDNAs are then transferred to a proprietary database, where quality controls and validation steps are carried out as described in example 15.

### 3. Cloning of Full Length Extended cDNAs

The PCR product containing the full coding sequence is then cloned in an appropriate vector. For example, the extended cDNAs can be cloned into the expression vector pED6dpc2 (DiscoverEase, Genetics Institute, Cambridge, MA) as follows. pED6dpc2 vector DNA is prepared with blunt ends by performing an EcoRI digestion followed by a fill in reaction. The blunt ended vector is dephosphorylated. After removal of PCR primers and ethanol precipitation, the PCR product containing the full coding sequence or the extended cDNA obtained as described above is phosphorylated with a kinase subsequently removed by phenol-Sevag extraction and precipitation. The double stranded extended cDNA is then ligated to the vector and the resulting expression plasmid introduced into appropriate host cells.

Since the PCR products obtained as described above are blunt ended molecules that can be cloned in either direction, the orientation of several clones for each PCR product is determined. Then, 4 to 10 clones are ordered in microtiter plates and subjected to a PCR reaction using a first primer located in the vector close to the cloning site and a second primer located in the portion of the extended cDNA corresponding to the 3' end of the mRNA. This second primer may be the antisense primer used in anchored PCR in the case of direct cloning (case a) or the antisense primer located inside the 3'UTR in the case of indirect cloning (case b). Clones in which the start codon of the extended cDNA is operably linked to the promoter in the vector so as to permit expression of the protein encoded by the extended cDNA are conserved and sequenced. In addition to the ends of cDNA inserts, approximately 50 bp of vector DNA on each side of the cDNA insert are also secluenced.

The cloned PCR products are then entirely sequenced according to the aforementioned procedure. In this case, contigation of long fragments is then performed on walking sequences that have already contigated for uncloned PCR products during primer walking. Sequencing of cloned amplicons is complete when the resulting contigs include the whole coding region as well as overlapping sequences with vector DNA on both ends.

### 4. Computer analysis of Full Length Extended cDNA

Sequences of all full length extended cDNAs are then submitted to further analysis as described below. Before searching the extended full length cDNAs for sequences of interest, extended cDNAs which are not of interest (vector RNAs, transfer RNAs, ribosomal RNAs, mitochondrial RNAs, prokaryotic RNAs and fungal RNAs) are discarded using methods essentially similar to those described for 5'ESTs in Example 18.

### a) Identification of structural features

Structural features, e.g. polyA tail and polyadenylation signal, of the sequences of full length extended cDNAs are subsequently determined as follows.

A polyA tail is defined as a homopolymeric stretch of at least 11 A with at most one alternative base within it. The polyA tail search is restricted to the last 100 nt of the sequence and limited to stretches of 11 consecutive A's because sequencing reactions are often not readable after such a polyA stretch. Stretches having more than 90% homology over 8 nucleotides are identified as polyA tails using BLAST2N.

To search for a polyadenylation signal, the polyA tail is clipped from the full-length sequence. The 50 bp preceding the polyA tail are first searched for the canonic polyadenylation AAUAAA signal and, if the canonic signal is not detected, for the alternative AUUAAA signal (Sheets *et al., Nuc. Acids Res.* **18**: 5799-5805, 1990). If neither of these consensus polyadenylation signals is found, the canonic motif is searched again allowing one mismatch to account for possible sequencing errors. More than 85 % of identified polyadenylation signals of either type actually ends 10 to 30 bp from the polyA tail. Alternative AUUAAA signals represents approximately 15 % of the total number of identified polyadenylation signals.

### b) Identification of functional features

Functional features, e.g. ORFs and signal sequences, of the sequences of full length extended cDNAs were subsequently determined as follows.

The 3 upper strand frames of extended cDNAs are searched for ORFs defined as the maximum length fragments beginning with a translation intiation codon and ending with a stop codon. ORFs encoding at least 20 amino acids are preferred.

Each found ORF is then scanned for the presence of a signal peptide in the first 50 amino-acids or, where appropriate, within shorter regions down to 20 amino acids or less in the ORF, using the matrix method of von Heijne (*Nuc. Acids Res.* **14**: 4683-4690, 1986) as described in Example 22.

### c) Homology to either nucleotidic or proteic sequences

Categorization of full-length sequences may be achieved using procedures essentially similar to those described for 5'ESTs in Example 24.

Extended cDNAs prepared as described above may be subsequently engineered to obtain nucleic acids which include desired portions of the extended cDNA using conventional techniques such as subcloning, PCR, or *in vitro* oligonucleotide synthesis. For example, nucleic acids which include only the full coding sequences *(i.e.* the sequences encoding the signal peptide and the mature protein remaining after the signal peptide is cleaved off) may be obtained using techniques known to those skilled in the art. Alternatively, conventional techniques may be applied to obtain nucleic acids which contain only the coding sequences for the mature protein remaining after the signal peptide is cleaved off or nucleic acids which contain only the coding sequences for the signal peptides.

Similarly, nucleic acids containing any other desired portion of the coding sequences for the secreted protein may be obtained. For example, the nucleic acid may contain at least 10 consecutive bases of an extended cDNA such as one of the extended cDNAs described below. In another embodiment, the nucleic acid may contain at least 15 consecutive bases of an extended cDNA such as one of the extended cDNAs described below. Alternatively, the nucleic acid may contain at least 20 consecutive bases of an extended cDNA such as one of the extended cDNAs described below. In another embodiment, the nucleic acid may contain at least 25 consecutive bases of an extended cDNA such as one of the extended cDNAs described below. In yet another embodiment, the nucleic acid may contain at least 40 consecutive bases of an extended cDNA such as one of the extended cDNAs described below.

Once an extended cDNA has been obtained, it can be sequenced to determine the amino acid sequence it encodes. Once the encoded amino acid sequence has been determined, one can create and identify any of the many conceivable cDNAs that will encode that protein by simply using the degeneracy of the genetic code. For example, allelic variants or other homologous nucleic acids can be identified as described below. Alternatively, nucleic acids encoding the desired amino acid sequence can be synthesized *in vitro.*

In a preferred embodiment, the coding sequence may be selected using the known codon or codon pair preferences for the host organism in which the cDNA is to be expressed.

The extended cDNAs derived from the 5' ESTS of the present invention were obtained as described in Example 28 below.

### EXAMPLE 28

### Characterization of cloned extended cDNAs obtained using 5' ESTs

The procedure described in Example 27 above was used to obtain the extended cDNAs derived from the 5' ESTs of the present invention in a variety of tissues. The following list provides a few examples of thus obtained extended cDNAs.

Using this approach, the full length cDNA of SEQ ID NO:17 (internal identification number 48-19-3-G1-FL1) was obtained. This cDNA falls into the "EST-ext" category described above and encodes the signal peptide MKKVLLLITAILAVAVG (SEQ ID NO: 18) having a von Heijne score of 8.2.

The full length cDNA of SEQ ID NO:19 (internal identification number 58-34-2-E7-FL2) was also obtained using this procedure. This cDNA falls into the "EST-ext" category described above and encodes the signal peptide MWWFQQGLSFLPSALVIWTSA (SEQ ID NO:20) having a von Heijne score of 5.5.

Another full length cDNA obtained using the procedure described above has the sequence of SEQ ID NO:21 (internal identification number 51-27-1-E8-FL1). This cDNA, falls into the "EST-ext" category described above and encodes the signal peptide MVLTTLPSANSANSPVNMPTTGPNSLSYASSALSPCLT (SEQ ID NO:22) having a von Heijne score of 5.9.

The above procedure was also used to obtain a full length cDNA having the sequence of SEQ ID NO:23 (internal identification number 76-4-1-G5-FL1). This cDNA falls into the "EST-ext" category described above and encodes the signal peptide ILSTVTALTFAXA (SEQ ID NO:24) having a von Heijne score of 5.5

The full length cDNA of SEQ ID NO:25 (internal identification number 51-3-3-B10-FL3) was also obtained using this procedure. This cDNA falls into the "new" category described above and encodes a signal peptide LVLTLCTLPLAVA (SEQ ID NO:26) having a von Heijne score of 10.1.

The full length cDNA of SEQ ID NO:27 (internal identification number 58-35-2-F10-FL2) was also obtained using this procedure. This cDNA falls into the "new" category described above and encodes a signal peptide LWLLFFLVTAIHA (SEQ ID NO:28) having a von Heijne score of 10.7.

Bacterial clones containing plasmids containing the full length cDNAs described above are presently stored in the inventor's laboratories under the internal identification numbers provided above. The inserts may be recovered from the stored materials by growing an aliquot of the appropriate bacterial clone in the appropriate medium. The plasmid DNA can then be isolated using plasmid isolation procedures familiar to those skilled in the art such as alkaline lysis minipreps or large scale alkaline lysis plasmid isolation procedures. If desired the plasmid DNA may be further enriched by centrifugation on a cesium chloride gradient, size exclusion chromatography, or anion exchange chromatography. The plasmid DNA obtained using these procedures may then be manipulated using standard cloning techniques familiar to those skilled in the art. Alternatively, a PCR can be done with primers designed at both ends of the cDNA insertion. The PCR product which corresponds to the cDNA can then be manipulated using standard cloning techniques familiar to those skilled in the art.

The polypeptides encoded by the extended cDNAs may be screened for the presence of known structural or functional motifs or for the presence of signatures, small amino acid sequences which are well conserved amongst the members of a protein family. The conserved regions have been used to derive consensus patterns or matrices included in the PROSITE data bank, in particular in the file prosite.dat (Release 13.0 of November 1995, located at http://expasy.hcuge.ch/sprot/prosite.html. Prosite_convert and prosite_scan programs (http://ulrec3.unil.ch/ftpserveur/prosite_scan) may be used to find signatures on the extended cDNAs.

For each pattern obtained with the prosite convert program from the prosite dat file, the accuracy of the detection on a new protein sequence may be assessed by evaluating the frequency of irrelevant hits on the population of human secreted proteins included in the data bank SWISSPROT. The ratio between the number of hits on shuffled proteins (with a window size of 20 amino acids) and the number of hits on native (unshuffled) proteins may be used as an index. Every pattern for which the ratio is greater than 20% (one hit on shuffled proteins for 5 hits on native proteins) may be skipped during the search with prosite_scan. The program used to shuffle protein sequences (db_shuffled) and the program used to determine the statistics for each pattern in the protein data banks (prosite_statistics) are available on the ftp site http://ulrec3.unil.ch/ftpserveur/prosite_scan.

In addition to PCR based methods for obtaining extended cDNAs, traditional hybridization based methods may also be employed. These methods may also be used to obtain the genomic DNAs which encode the mRNAs from which the 5' ESTs were derived, mRNAs corresponding to the extended cDNAs, or nucleic acids which are homologous to extended cDNAs or 5' ESTs. Example 29 below provides examples of such methods.

### EXAMPLE 29

### Methods for Obtaining cDNAs which include the Entire Coding Region and the Authentic 5'End of the Corresponding mRNA

A full length cDNA library can be made using the strategies described in Examples 13, 14, 15, and 16 above by replacing the random nonamer used in Example 14 with an oligo-dT primer. For instance, the oligonucleotide of SEQ ID NO:14 may be used.

Alternatively, a cDNA library or genomic DNA library may be obtained from a commercial source or made using techniques familiar to those skilled in the art. Such cDNA or genomic DNA librairies may be used to isolate extended cDNAs obtained from 5' EST or nucleic acids homologous to extended cDNAs or 5' EST as follows. The cDNA library or genomic DNA library is hybridized to a detectable probe comprising at least 10 consecutive nucleotides from the 5' EST or extended cDNA using conventional techniques. Preferably, the probe comprises at least 12, 15, or 17 consecutive nucleotides from the 5' EST or extended cDNA. More preferably, the probe comprises at least 20 to 30 consecutive nucleotides from the 5' EST or extended cDNA. In some embodiments, the probe comprises more than 30 nucleotides from the 5' EST or extended cDNA.

Techniques for identifying cDNA clones in a cDNA library which hybridize to a given probe sequence are disclosed in Sambrook *et al.*, *Molecular Cloning: A Laboratory Manual 2d Ed.,* Cold Spring Harbor Laboratory Press, 1989. The same techniques may be used to isolate genomic DNAs.

Briefly, cDNA or genomic DNA clones which hybridize to the detectable probe are identified and isolated for further manipulation as follows. A probe comprising at least 10 consecutive nucleotides from the 5' EST or extended cDNA is labeled with a detectable label such as a radioisotope or a fluorescent molecule. Preferably, the probe comprises at least 12, 15, or 17 consecutive nucleotides from the 5' EST or extended cDNA More preferably, the probe comprises 20 to 30 consecutive nucleotides from the 5' EST or extended cDNA. In some embodiments, the probe comprises more than 30 nucleotides from the 5' EST or extended cDNA.

Techniques for labeling the probe are well known and include phosphorylation with polynucleotide kinase, nick translation, *in vitro* transcription, and non radioactive techniques. The cDNAs or genomic DNAs in the library are transferred to a nitrocellulose or nylon filter and denatured. After blocking of non specific sites, the filter is incubated with the labeled probe for an amount of time sufficient to allow binding of the probe to cDNAs or genomic DNAs containing a sequence capable of hybridizing thereto.

By varying the stringency of the hybridization conditions used to identify extended cDNAs or genomic DNAs which hybridize to the detectable probe, extended cDNAS having different levels of homology to the probe can be identified and isolated as described below.

### 1. Identification of Extended cDNA or Genomic cDNA Sequences Having a High Degree of Homology to the Labeled Probe .

To identify extended cDNAs or genomic DNAs having a high degree of homology to the probe sequence, the melting temperature of the probe may be calculated using the following formulas:

For probes between 14 and 70 nucleotides in length the melting temperature (Tm) is calculated using the formula: Tm=81.5+16.6(log [Na+])+0.41(fraction G+C)(600/N) where N is the length of the probe.

If the hybridization is carried out in a solution containing formamide, the melting temperature may be calculated using the equation Tm=81.5+16.6(log [Na+])+0.41(fraction G+C)-(0.63% formamide)-(600/N) where N is the length of the probe.

Prehybridization may be carried out in 6X SSC, 5X Denhardt's reagent, 0.5% SDS, 100 µg denatured fragmented salmon sperm DNA or 6X SSC, 5X Denhardt's reagent, 0.5% SDS, 100 µg denatured fragmented salmon sperm DNA, 50% formamide. The formulas for SSC and Denhardt's solutions are listed in Sambrook *et al., supra.*

Hybridization is conducted by adding the detectable probe to the prehybridization solutions listed above. Where the probe comprises double stranded DNA, it is denatured before addition to the hybridization solution. The filter is contacted with the hybridization solution for a sufficient period of time to allow the probe to hybridize to extended cDNAs or genomic DNAs containing sequences complementary thereto or homologous thereto. For probes over 200 nucleotides in length, the hybridization may be carried out at 15-25°C below the Tm. For shorter probes, such as oligonucleotide probes, the hybridization may be conducted at 15-25°C below the Tm. Preferably, for hybridizations in 6X SSC, the hybridization is conducted at approximately 68°C. Preferably, for hybridizations in 50% formamide containing solutions, the hybridization is conducted at approximately 42°C.

All of the foregoing hybridizations would be considered to be under "stringent" conditions.

Following hybridization, the filter is washed in 2X SSC, 0.1% SDS at room temperature for 15 minutes. The filter is then washed with 0.1X SSC, 0.5% SDS at room temperature for 30 minutes to 1 hour. Thereafter, the solution is washed at the hybridization temperature in 0.1X SSC, 0.5% SDS. A final wash is conducted in 0.1X SSC at room temperature.

Extended cDNAs, nucleic acids homologous to extended cDNAs or 5' ESTs, or genomic DNAs which have hybridized to the probe are identified by autoradiography or other conventional techniques.

### 2. Obtention of Extended cDNA or Genomic cDNA Sequences Having Lower Degrees of Homology to the Labeled Probe

The above procedure may be modified to identify extended cDNAs, nucleic acids homologous to extended cDNAs, or genomic DNAs having decreasing levels of homology to the probe sequence. For example, to obtain extended cDNAs, nucleic acids homologous to extended cDNAs, or genomic DNAs of decreasing homology to the detectable probe, less stringent conditions may be used. For example, the hybridization temperature may be decreased in increments of 5°C from 68°C to 42°C in a hybridization buffer having a sodium concentration of approximately 1M. Following hybridization, the filter may be washed with 2X SSC, 0.5% SDS at the temperature of hybridization. These conditions are considered to be "moderate" conditions above 50°C and "low" conditions below 50°C.

Alternatively, the hybridization may be carried out in buffers, such as 6X SSC, containing formamide at a temperature of 42°C. In this case, the concentration of formamide in the hybridization buffer may be reduced in 5% increments from 50% to 0% to identify clones having decreasing levels of homology to the probe. Following hybridization, the filter may be washed with 6X SSC, 0.5% SDS at 50°C. These conditions are considered to be "moderate" conditions above 25% formamide and "low" conditions below 25% formamide.

Extended cDNAs, nucleic acids homologous to extended cDNAs, or genomic DNAs which have hybridized to the probe are identified by autoradiography.

### 3. Determination of the Degree of Homology Between the Obtained Extended cDNAs and the Labeled Probe

If it is desired to obtain nucleic acids homologous to extended cDNAs, such as allelic variants thereof or nucleic acids encoding proteins related to the proteins encoded by the extended cDNAs, the level of homology between the hybridized nucleic acid and the extended cDNA or 5' EST used as the probe may be further determined using BLAST2N; parameters may be adapted depending on the sequence length and degree of homology studied. To determine the level of homology between the hybridized nucleic acid and the extended cDNA or 5'EST from which the probe was derived, the nucleotide sequences of the hybridized nucleic acid and the extended cDNA or 5'EST from which the probe was derived are compared. For example, using the above methods, nucleic acids having at least 95% nucleic acid homology to the extended cDNA or 5'EST from which the probe was derived may be obtained and identified. Similarly, by using progressively less stringent hybridization conditions one can obtain and identify nucleic acids having at least 90%, at least 85%, at least 80% or at least 75% homology to the extended cDNA or 5'EST from which the probe was derived.

To determine whether a clone encodes a protein having a given amount of homology to the protein encoded by the extended cDNA or 5' EST, the amino acid sequence encoded by the extended cDNA or 5' EST is compared to the amino acid sequence encoded by the hybridizing nucleic acid. Homology is determined to exist when an amino acid sequence in the extended cDNA or 5' EST is closely related to an amino acid sequence in the hybridizing nucleic acid. A sequence is closely related when it is identical to that of the extended cDNA or 5' EST or when it contains one or more amino acid substitutions therein in which amino acids having similar characteristics have been substituted for one another. Using the above methods and algorithms such as FASTA with parameters depending on the sequence length and degree of homology studied, one can obtain nucleic acids encoding proteins having at least 95%, at least 90%, at least 85%, at least 80% or at least 75% homology to the proteins encoded by the extended cDNA or 5'EST from which the probe was derived.

In addition to the above described methods, other protocols are available to obtain extended cDNAs using 5' ESTs as outlined in the following paragraphs.

Extended cDNAs may be prepared by obtaining mRNA from the tissue, cell, or organism of interest using mRNA preparation procedures utilizing polyA selection procedures or other techniques known to those skilled in the art. A first primer capable of hybridizing to the polyA tail of the mRNA is hybridized to the mRNA and a reverse transcription reaction is performed to generate a first cDNA strand.

The first cDNA strand is hybridized to a second primer containing at least 10 consecutive nucleotides of the sequences of SEQ ID NOs 38-291. Preferably, the primer comprises at least 12, 15, or 17 consecutive nucleotides from the sequences of SEQ ID NOs 38-291. More preferably, the primer comprises 20 to 30 consecutive nucleotides from the sequences of SEQ ID NOs 38-291. In some embodiments, the primer comprises more than 30 nucleotides from the sequences of SEQ ID NOs 38-291. If it is desired to obtain extended cDNAs containing the full protein coding sequence, including the authentic translation initiation site, the second primer used contains sequences located upstream of the translation initiation site. The second primer is extended to generate a second cDNA strand complementary to the first cDNA strand Alternatively, RT-PCR may be performed as described above using primers from both ends of the cDNA to be obtained.

Extended cDNAs containing 5' fragments of the mRNA may be prepared by hybridizing an mRNA comprising the sequence of the 5' EST for which an extended cDNA is desired with a primer comprising at least 10 consecutive nucleotides of the sequences complementary to the 5'EST and reverse transcribing the hybridized primer to make a first cDNA strand from the mRNAs. Preferably, the primer comprises at least 12, 15, or 17 consecutive nucleotides from the 5'EST. More preferably, the primer comprises 20 to 30 consecutive nucleotides from the 5'EST.

Thereafter, a second cDNA strand complementary to the first cDNA strand is synthesized. The second cDNA strand may be made by hybridizing a primer complementary to sequences in the first cDNA strand to the first cDNA strand and extending the primer to generate the second cDNA strand.

The double stranded extended cDNAs made using the methods described above are isolated and cloned. The extended cDNAs may be cloned into vectors such as plasmids or viral vectors capable of replicating in an appropriate host cell. For example, the host cell may be a bacterial, mammalian, avian, or insect cell.

Techniques for isolating mRNA, reverse transcribing a primer hybridized to mRNA to generate a first cDNA strand, extending a primer to make a second cDNA strand complementary to the first cDNA strand, isolating the double stranded cDNA and cloning the double stranded cDNA are well known to those skilled in the art and are described *in Current Protocols in Molecular Biology,* John Wiley and Sons, Inc. 1997 and Sambrook *et al., Molecular Cloning: A Laboratory Manual,* Second Edition, Cold Spring Harbor Laboratory Press, 1989.

Alternatively, procedures such as the one described in Example 29 may be used for obtaining full length cDNAs or extended cDNAs. In this approach, full length or extended cDNAs are prepared from mRNA and cloned into double stranded phagemids as follows. The cDNA library in the double stranded phagemids is then rendered single stranded by treatment with an endonuclease, such as the Gene II product of the phage F1, and an exonuclease (Chang *et al., Gene* **127**:95-8, 1993). A biotinylated oligonucleotide comprising the sequence of a 5' EST, or a fragment containing at least 10 nucleotides thereof, is hybridized to the single stranded phagemids. Preferably, the fragment comprises at least 12, 15, or 17 consecutive nucleotides from the 5' EST. More preferably, the fragment comprises 20-30 consecutive nucleotides from the 5' EST. In some procedures, the fragment may comprise more than 30 consecutive nucleotides from the 5' EST.

Hybrids between the biotinylated oligonucleotide and phagemids having inserts containing the 5' EST sequence are isolated by incubating the hybrids with streptavidin coated paramagnetic beads and retrieving the beads with a magnet (Fry *et al., Biotechniques,* **13**: 124-131, 1992). Therafter, the resulting phagemids containing the 5' EST sequence are released from the beads and converted into double stranded DNA using a primer specific for the 5' EST sequence. Alternatively, protocoles such as the Gene Trapper kit (Gibco BRL) may be used. The resulting double stranded DNA is transformed into bacteria. Extended cDNAs containing the 5' EST sequence are identified by colony PCR or colony hybridization.

Using any of the above described methods in section III, a plurality of extended cDNAs containing full length protein coding sequences or sequences encoding only the mature protein remaining after the signal peptide is cleaved off may be provided as cDNA libraries for subsequent evaluation of the encoded proteins or use in diagnostic assays as described below.

### IV. Expression of Proteins Encoded by Extended cDNAs Isolated Using 5' ESTs

Extended cDNAs containing the full protein coding sequences of their corresponding mRNAs or portions thereof, such as cDNAs encoding the mature protein, may be used to express the encoded secreted proteins or portions thereof as described in Example 30 below. If desired, the extended cDNAs may contain the sequences encoding the signal peptide to facilitate secretion of the expressed protein. It will be appreciated that a plurality of extended cDNAs containing the full protein coding sequences or portions thereof may be simultaneously cloned into expression vectors to create an expression library for analysis of the encoded proteins as described below.

### EXAMPLE 30

### Expression of the Proteins Encoded by the Genes Corresponding to 5'ESTS or Portions Thereof

To express the proteins encoded by the genes corresponding to 5' ESTs (or portions thereof), full length cDNAs containing the entire protein coding region or extended cDNAs containing sequences adjacent to the 5' ESTs (or portions thereof) are obtained as described in Examples 27-29 and cloned into a suitable expression vector. If desired, the nucleic acids may contain the sequences encoding the signal peptide to facilitate secretion of the expressed protein. The nucleic acids inserted into the expression vectors may also contain sequences upstream of the sequences encoding the signal peptide, such as sequences which regulate expression levels or sequences which confer tissue specific expression.

The nucleic acid encoding the protein or polypeptide to be expressed is operably linked to a promoter in an expression vector using conventional cloning technology. The expression vector may be any of the mammalian, yeast, insect or bacterial expression systems known in the art. Commercially available vectors and expression systems are available from a variety of suppliers including Genetics Institute (Cambridge, MA), Stratagene (La Jolla, California), Promega (Madison, Wisconsin), and Invitrogen (San Diego, California). If desired, to enhance expression and facilitate proper protein folding, the codon context and codon pairing of the sequence may be optimized for the particular expression organism in which the expression vector is introduced, as explained by Hatfield, *et al.,* U.S. Patent No. 5,082,767.

The cDNA cloned into the expression vector may encode the entire protein *(i.e.* the signal peptide and the mature protein), the mature protein *(i.e.* the protein created by cleaving the signal peptide off), only the signal peptide or any other portion thereof.

The following is provided as one exemplary method to express the proteins encoded by the extended cDNAs corresponding to the 5' ESTs or the nucleic acids described above. First, the methionine initiation codon for the gene and the polyA signal of the gene are identified. If the nucleic acid encoding the polypeptide to be expressed lacks a methionine to serve as the initiation site, an initiating methionine can be introduced next to the first codon of the nucleic acid using conventional techniques. Similarly, if the extended cDNA lacks a polyA signal, this sequence can be added to the construct by, for example, splicing out the polyA signal from pSG5 (Stratagene) using BglII and SalI restriction endonuclease enzymes and incorporating it into the mammalian expression vector pXT1 (Stratagene). PXT1 contains the LTRs and a portion of the *gag* gene from Moloney Murine Leukemia Virus. The position of the LTRs in the construct allow efficient stable transfection. The vector includes the Herpes Simplex thymidine kinase promoter and the selectable neomycin gene. The extended cDNA or portion thereof encoding the polypeptide to be expressed is obtained by PCR from the bacterial vector using oligonucleotide primers complementary to the extended cDNA or portion thereof and containing restriction endonuclease sequences for Pst I incorporated into the 5'primer and BgIII at the 5' end of the corresponding cDNA 3' primer, taking care to ensure that the extended cDNA is positioned with the poly A signal. The purified fragment obtained from the resulting PCR reaction is digested with PstI, blunt ended with an exonuclease, digested with Bgl II, purified and ligated to pXT1 containing a poly A signal and prepared for this ligation (blunt/BgIII).

The ligated product is transfected into mouse NIH 3T3 cells using Lipofectin (Life Technologies, Inc., Grand Island, New York) under conditions outlined in the product specification. Positive transfectants are selected after growing the transfected cells in 600 µg/ml G418 (Sigma, St. Louis, Missouri). Preferably the expressed protein is released into the culture medium, thereby facilitating purification.

Alternatively, the extended cDNAs may be cloned into pED6dpc2 as described above. The resulting pED6dpc2 constructs may be transfected into a suitable host cell, such as COS 1 cells. Methotrexate resistant cells are selected and expanded. Preferably, the protein expressed from the extended cDNA is released into the culture medium thereby facilitating purification.

Proteins in the culture medium are separated by gel electrophoresis. If desired, the proteins may be ammonium sulfate precipitated or separated based on size or charge prior to electrophoresis.

As a control, the expression vector lacking a cDNA insert is introduced into host cells or organisms and the proteins in the medium are harvested. The secreted proteins present in the medium are detected using techniques familiar to those skilled in the art such as Coomassie blue or silver staining or using antibodies against the protein encoded by the extended cDNA

Antibodies capable of specifically recognizing the protein of interest may be generated using synthetic 15-mer peptides having a sequence encoded by the appropriate 5' EST, extended cDNA, or portion thereof The synthetic peptides are injected into mice to generate antibody to the polypeptide encoded by the 5' EST, extended cDNA, or portion thereof.

Secreted proteins from the host cells or organisms containing an expression vector which contains the extended cDNA derived from a 5' EST or a portion thereof are compared to those from the control cells or organism. The presence of a band in the medium from the cells containing the expression vector which is absent in the medium from the control cells indicates that the extended cDNA encodes a secreted protein. Generally, the band corresponding to the protein encoded by the extended cDNA will have a mobility near that expected based on the number of amino acids in the open reading frame of the extended cDNA. However, the band may have a mobility different than that expected as a result of modifications such as glycosylation, ubiquitination, or enzymatic cleavage.

Alternatively, if the protein expressed from the above expression vectors does not contain sequences directing its secretion, the proteins expressed from host cells containing an expression vector with an insert encoding a secreted protein or portion thereof can be compared to the proteins expressed in control host cells containing the expression vector without an insert. The presence of a band in samples from cells containing the expression vector with an insert which is absent in samples from cells containing the expression vector without an insert indicates that the desired protein or portion thereof is being expressed. Generally, the band will have the mobility expected for the secreted protein or portion thereof However, the band may have a mobility different than that expected as a result of modifications such as glycosylation, ubiquitination, or enzymatic cleavage.

The protein encoded by the extended cDNA may be purified using standard immunochromatography techniques. In such procedures, a solution containing the secreted protein, such as the culture medium or a cell extract, is applied to a column having antibodies against the secreted protein attached to the chromatography matrix. The secreted protein is allowed to bind the immunochromatography column. Thereafter, the column is washed to remove non-specifically bound proteins. The specifically bound secreted protein is then released from the column and recovered using standard techniques.

If antibody production is not possible, the extended cDNA sequence or portion thereof may be incorporated into expression vectors designed for use in purification schemes employing chimeric polypeptides. In such strategies, the coding sequence of the extended cDNA or portion thereof is inserted in frame with the gene encoding the other half of the chimera. The other half of the chimera may be β-globin or a nickel binding polypeptide. A chromatography matrix having antibody to β-globin or nickel attached thereto is then used to purify the chimeric protein. Protease cleavage sites may be engineered between the β-globin gene or the nickel binding polypeptide and the extended cDNA or portion thereof Thus, the two polypeptides of the chimera may be separated from one another by protease digestion.

One useful expression vector for generating β-globin chimerics is pSG5 (Stratagene), which encodes rabbit β-clobin. Intron II of the rabbit β-globin gene facilitates splicing of the expressed transcript, and the polyadenylation signal incorporated into the construct increases the level of expression. These techniques as described are well known to those skilled in the art of molecular biology. Standard methods are published in methods texts such as Davis *et al.., (Basic Methods in Molecular Biology,* Davis, Dibner, and Battey, ed., Elsevier Press, NY, 1986) and many of the methods are available from Stratagene, Life Technologies, Inc., or Promega. Polypeptide may additionally be produced from the construct using *in vitro* translation systems such as the *In vitro* Express^{™} Translation Kit (Stratagene).

Following expression and purification of the secreted proteins encoded by the 5' ESTs, extended cDNAs, or fragments thereof, the purified proteins may be tested for the ability to bind to the surface of various cell types as described in Example 31 below. It will be appreciated that a plurality of proteins expressed from these cDNAs may be included in a panel of proteins to be simultaneously evaluated for the activities specifically described below, as well as other biological roles for which assays for determining activity are available.

### EXAMPLE 31

### Analysis of Secreted Proteins to Determine Whether they Bind to the Cell Surface

The proteins encoded by the 5' ESTs, extended cDNAs, or fragments thereof are cloned into expression vectors such as those described in Example 30. The proteins are purified by size, charge, immunochromatography or other techniques familiar to those skilled in the art. Following purification, the proteins are labeled using techniques known to those skilled in the art. The labeled proteins are incubated with cells or cell lines derived from a variety of organs or tissues to allow the proteins to bind to any receptor present on the cell surface. Folloyving the incubation, the cells are washed to remove non-specifically bound protein. The labeled proteins are detected by autoradiography. Alternatively, unlabeled proteins may be incubated with the cells and detected with antibodies having a detectable label, such as a fluorescent molecule, attached thereto.

Specificity of cell surface binding may be analyzed by conducting a competition analysis in which various amounts of unlabeled protein are incubated along with the labeled protein. The amount of labeled protein bound to the cell surface decreases as the amount of competitive unlabeled protein increases. As a control, various amounts of an unlabeled protein unrelated to the labeled protein is included in some binding reactions. The amount of labeled protein bound to the cell surface does not decrease in binding reactions containing increasing amounts of unrelated unlabeled protein, indicating that the protein encoded by the cDNA binds specifically to the cell surface.

As discussed above, secreted proteins have been shown to have a number of important physiological effects and, consequently, represent a valuable therapeutic resource. The secreted proteins encoded by the extended cDNAs or portions thereof made according to Examples 27-29 may be evaluated to determine their physiological activities as described below.

### EXAMPLE 32

### Assaying the Proteins Expressed from Extended cDNAs or Portions Thereof for Cutokine, Cell Proliferation or Cell Differentiation Activity

As discussed above, secreted proteins may act as cytokines or may affect cellular proliferation or differentiation. Many protein factors discovered to date, including all known cytokines, have exhibited activity in one or more factor dependent cell proliferation assays, and hence the assays serve as a convenient confirmation of cytokine activity. The activity of a protein encoded by the extended cDNAs is evidenced by any one of a number of routine factor dependent cell proliferation assays for cell lines including, without limitation, 32D, DA2, DA1G, T10, B9, B9/11, BaF3, MC9/G, M⁺ (preB M⁺), 2E8, RB5, DA1, 123, T1165, HT2, CTLL2, TF-1, Mo7c and CMK The proteins encoded by the above extended cDNAs or portions thereof may be evaluated for their ability to regulate T cell or thymocyte proliferation in assays such as those described above or in the following references: *Current Protocols in Immunology,* Ed. by Coligan *et al..,* Greene Publishing Associates and Wiley-Interscience; Takai *et al. J. Immunol* **137**:3494-3500, 1986., Bertagnolli *et al., J. Immunol.* **145**:1706-1712, 1990., Bertagnolli *et al.,* Cell. *Immunol.* **133**:327-341, 1991; Bertagnolli, *et al., J. Immunol.* **149**:3778-3783, 1992; Bowman *et al., J. Immunol.* **152**: 1756-1761, 1994.

In addition, numerous assays for cytokine production and/or the proliferation of spleen cells, lymph node cells and thymocytes are known. These include the techniques disclosed in *Current Protocols in Immunology, supra* **1**:3.12.1-3.12.14; and Schreiber In *Current Protocols in Immunology, supra 1 :* 6.8.1-6.8.8.

The proteins encoded by the cDNAs may also be assayed for the ability to regulate the proliferation and differentiation of hematopoietic or lymphopoietic cells. Many assays for such activity are familiar to those skilled in the art, including the assays in the following references: Bottomly *et al.,* In *Current Protocols in Immunology., supra.* **1**: 6.3.1-6.3.12,; deVries *et al., J. Exp. Med.* **173**:1205-1211, 1991; Moreau *et al., Nature* **36**:690-692, 1988; Greenberger *et al., Proc. Natl. Acad. Sci. U.S.A.* **80**:2931-2938, 1983; Nordan, R, In *Current Protocols in Immunology., supra.* **1** : 6.6.1-6.6.5; Smith et al., *Proc. Natl. Acad Sci. U.S.A.* **83**:1857-1861, 1986; Bennett *et al.,* in *Current Protocols in Immunology supra* **1** : 6.15.1; Ciarletta *et al.,* In *Current Protocols in Immunology. supra* **1** : 6.13.1.

The proteins encoded by the cDNAs may also be assayed for their ability to regulate T-cell responses to antigens. Many assays for such activity are familiar to those skilled in the art, including the assays described in the following references: Chapter 3 (*In Vitro* Assays for Mouse Lymphocyte Function), Chapter 6 (Cytokines and Their Cellular Receptors) and Chapter 7, (Immunologic Studies in Humans) *in Current Protocols in Immunology supra,* Weinberger *et al., Proc. Natl. Acad Sci. USA* **77**:6091-6095, 1980; Weinberger *et al., Eur. J. Immun.* **11**:405-411, 1981; Takai *et al., J. Immunol.* **137**:3494-3500, 1986; Takai *et al., J. Immunol.* **140**:508-512, 1988.

Those proteins which exhibit cytokine, cell proliferation, or cell differentiation activity may then be formulated as pharmaceuticals and used to treat clinical conditions in which induction of cell proliferation or differentiation is beneficial. Alternatively, as described in more detail below, genes encoding these proteins or nucleic acids regulating the expression of these proteins may be introduced into appropriate host cells to increase or decrease the expression of the proteins as desired.

### EXAMPLE 33

### Assaying the Proteins Expressed from Extended cDNAs or Portions Thereof for Activity as Immune System Regulators

The proteins encoded by the cDNAs may also be evaluated for their effects as immune regulators. For example, the proteins may be evaluated for their activity to influence thymocyte or splenocyte cytotoxicity. Numerous assays for such activity are familiar to those skilled in the art including the assays described in the following references: Chapter 3 *(In vitro* Assays for Mouse Lymphocyte Function 3.1-3.19) and Chapter 7 (Immunologic studies in Humans) in *Current Protocols in Immunology,* Coligan *et al.,* Eds, Greene Publishing Associates and Wiley-Interscience; Herrmann el *al., Proc. Nail. Acad Sci. USA* **78**:2488-2492, 1981; Herrmann *et al., J*. *Immunol.* **128**:1968-1974, 1982; Handa *et al., J. Immunol.* **135**:1564-1572, 1985; Takai *et al., J. Immunol.* **137**:3494-3500, 1986; Takai *et al., J. Immunol.* **140**:508-512, 1988; Bowman *et al., J. Virology* **61**:1992-1998; Bertagnolli *et al., Cell Immunol.* **133**:327-341, 1991; Brown *et al., J. Immunol.* **153**:3079-3092, 1994.

The proteins encoded by the cDNAs may also be evaluated for their effects on T-cell dependent immunoglobulin responses and isotype switching. Numerous assays for such activity are familiar to those skilled in the art, including the assays disclosed in the following references: Maliszewski, *J. Immunol* **144**:3028-3033, 1990; Mond *et al.* in *Current Protocols in Immunology,* **1** : 3.8.1-3.8.16, *supra.*

The proteins encoded by the cDNAs may also be evaluated for their effect on immune effector cells, including their effect on Thl cells and cytotoxic lymphocytes. Numerous assays for such activity are familiar to those skilled in the art, including the assays disclosed in the following references: Chapter 3 *(In Vitro* Assays for Mouse Lymphocyte Function 3.1-3.19) and Chapter 7 (Immunologic Studies in Humans) in *Current Protocols in Immunology, supra;* Takai *et al., J. Immunol.* **137**:3494-3500, 1986; Takai *et al., J. Imminol.* **140**:508-512, 1988; Bertagnolli *el al., J. Immunol* **149**:3778-3783, 1992.

The proteins encoded by the cDNAs may also be evaluated for their effect on dendritic cell mediated activation of naive T-cells. Numerous assays for such activity are familiar to those skilled in the art, including the assays disclosed in the following references Guery *et al., J Imminol.* **134**:536-544, 1995; Inaba *et al., J. Exp. Med.* **173**:549-559, 1991; Macatonia *et al., J. Immunol.* **154**:5071-5079, 1995; Porgador *et al.J. Exp. Med* **182**:255-260, 1995; Nair *et al., J. Virol.* **67**:4062-4069, 1993; Huang *et al., Science* **264**:961-965, 1994; Macatonia *et al.J. Exp. Med.* **169**:1255-1264, 1989; Bhardwaj *et al., Journal of Clinical Investigation* **94**:797-807, 1994; and Inaba *et al., J. Exp. Med* **172**:631-640, 1990.

The proteins encoded by the cDNAs may also be evaluated for their influence on the lifetime of lymphocytes. Numerous assays for such activity are familiar to those skilled in the art, including the assays disclosed in the following references Darzynidewiez *et al., Cytometry* **13**:795-808, 1992; Gorczyca *et al., Leukemia* 7:659-670, 1993; Gorczyca *et al., Cancer Res.* **53**:1945-1951, 1993; Itoh *et al., Cell* **66**:233-243, 1991; Zacharchuk, *J. Immunol.* **145**:4037-4045, 1990; Zamai *et al., Cytometry* **14**:891-897, 1993; Gorczyca *et al., Int. J. Oncol.* **1**:639-648, 1992.

The proteins encoded by the cDNAs may also be evaluated for their influence on early steps of T-cell commitment and development. Numerous assays for such activity are familiar to those skilled in the art, including without limitation the assays disclosed in the following references: Antica *et al., Blood* **84**:111-117, 1994; Fine *et al., Cell. Immunol.* **155**:111-122, 1994; Galy *et al., Blood* **85**:2770-2778, 1995; Toki *et al., Proc. Nat. Acad Sci. USA* **88**:7548-7551, 1991.

Those proteins which exhibit activity as immune system regulators activity may then be formulated as pharmaceuticals and used to treat clinical conditions in which regulation of immune activity is beneficial. For example, the protein may be useful in the treatment of various immune deficiencies and disorders (including severe combined immunodeficiency), e.g., in regulating (up or down) growth and proliferation ofT and/or B lymphocytes, as well as effecting the cytolytic activity of NK cells and other cell populations. These immune deficiencies may be genetic or be caused by viral (e.g., HIV) as well as bacterial or fungal infections, or may result from autoimmune disorders. More specifically, infectious diseases caused by viral, bacterial, fungal or other infection may be treatable using a protein encoded by extended cDNAs derived from the 5' ESTs of the present invention, including infections by HIV, hepatitis viruses, herpesvimses, mycobacteria, Leishmania spp., plamodium and various fungal infections such as candidiasis. Of course, in this regard, a protein encoded by extended cDNAs derived from the 5' ESTs of the present invention may also be useful where a boost to the immune system generally may be desirable, *i.e.,* in the treatment of cancer.

Alternatively, proteins encoded by extended cDNAs derived from the 5' ESTs of the present invention may be used in treatment of autoimmune disorders including, for example, connective tissue disease, multiple sclerosis, systemic lupus erythematosus, rheumatoid arthritis, autoimmune pulmonary inflammation, Guillain-Barre syndrome, autoimmune thyroiditis, insulin dependent diabetes mellitis, myasthenia gravis, graft-versus-host disease and autoimmune inflammatory eye disease. Such a protein encoded by extended cDNAs derived from the 5' ESTs of the present invention may also to be useful in the treatment of allergic reactions and conditions, such as asthma (particularly allergic asthma) or other respiratory problems. Other conditions, in which immune suppression is desired (including, for example, organ transplantation), may also be treatable using a protein encoded by extended cDNAs derived from the 5' ESTs of the present invention.

Using the proteins of the invention it may also be possible to regulate immune responses either up or down.

Down regulation may involve inhibiting or blocking an immune response already in progress or may involve preventing the induction of an immune response. The functions of activated T-cells may be inhibited by suppressing T cell responses or by inducing specific tolerance in T cells, or both. Immunosuppression of T cell responses is generally an active non-antigen-specific process which requires continuous exposure of the T cells to the suppressive agent. Tolerance, which involves inducing non-responsiveness or anergy in T cells, is distinguishable from immunosuppression in that it is generally antigen-specific and persists after the end of exposure to the tolerizing agent. Operationally, tolerance can be demonstrated by the lack of a T cell response upon reexposure to specific antigen in the absence of the tolerizing agent.

Down regulating or preventing one or more antigen functions (including without limitation B lymphocyte antigen functions, such as, for example, B7 costimulation), e.g., preventing high level lymphokine synthesis by activated T cells, will be useful in situations of tissue, skin and organ transplantation and in graft-versus-host disease (GVHD). For example, blockage of T cell function should result in reduced tissue destruction in tissue transplantation. Typically, in tissue transplants, rejection of the transplant is initiated through its recognition as foreign by T cells, followed by an immune reaction that destroys the transplant. The administration of a molecule which inhibits or blocks interaction of a B7 lymphocyte antigen with its natural ligand(s) on immune cells (such as a soluble, monomeric form of a peptide having B7-2 activity alone or in conjunction with a monomeric form of a peptide having an activity of another B lymphocyte antigen (e.g., B7-1, B7-3) or blocking antibody), prior to transplantation, can lead to the binding of the molecule to the natural ligand(s) on the immune cells without transmitting the corresponding costimulatory signal. Blocking B lymphocyte antigen function in this matter prevents cytokine synthesis by immune cells, such as T cells, and thus acts as an immunosuppressant. Moreover, the lack of costimulation may also be sufficient to anergize the T cells, thereby inducing tolerance in a subject. Induction of long-term tolerance by B lymphocyte antigen-blocking reagents may avoid the necessity of repeated administration of these blocking reagents. To achieve sufficient immunosuppression or tolerance in a subject, it may also be necessary to block the function of a combination of B lymphocyte antigens.

The efficacy of particular blocking reagents in preventing organ transplant rejection or GVHD can be assessed using animal models that are predictive of efficacy in humans. Examples of appropriate systems which can be used include allogeneic cardiac grafts in rats and xenogeneic pancreatic islet cell grafts in mice, both of which have been used to examine the immunosuppressive effects of CTLA4Ig fusion proteins *in vivo* as described in Lenschow *et al., Science* **257**:789-792, 1992 and Turka *et al., Proc. Natl. Acad. Sci USA,* **89**:11102-11105, 1992. In addition, murine models of GVHD (see Paul ed., *Fundamental Immunology,* Raven Press, New York, 1989, pp. 846-847) can be used to determine the effect of blocking B lymphocyte antigen function *in vivo* on the development of that disease.

Blocking antigen function may also be therapeutically useful for treating autoimmune diseases. Many autoimmune disorders are the result of inappropriate activation of T cells that are reactive against self tissue and which promote the production of cytokines and autoantibodies involved in the pathology of the diseases. Preventing the activation of autoreactive T cells may reduce or eliminate disease symptoms. Administration of reagents which block costimulation of T cells by disrupting receptor/ligand interactions of B lymphocyte antigens can be used to inhibit T cell activation and prevent production of autoantibodies or T cell-derived cytokines which potentially involved in the disease process. Additionally, blocking reagents may induce antigen-specific tolerance of autoreactive T cells which could lead to long-term relief from the disease. The efficacy of blocking reagents in preventing or alleviating autoimmune disorders can be determined using a number of well-characterized animal models of human autoimmune diseases. Examples include murine experimental autoimmune encephalitis, systemic lupus erythmatosis in MRL/pr/pr mice or NZB hybrid mice, murine autoimmuno collagen arthritis, diabetes mellitus in OD mice and BB rats, and murine experimental myasthenia gravis (see Paul ed., *supra,* pp. 840-856).

Upregulation of an antigen function (preferably a B lymphocyte antigen function), as a means of up regulating immune responses, may also be useful in therapy. Upregulation of immune responses may involve either enhancing an existing immune response or eliciting an initial immune response as shown by the following examples. For instance, enhancing an immune response through stimulating B lymphocyte antigen function may be useful in cases of viral infection. In addition, systemic viral diseases such as influenza, the common cold, and encephalitis might be alleviated by the administration of stimulatory form of B lymphocyte antigens systemically.

Alternatively, antiviral immune responses may be enhanced in an infected patient by removing T cells from the patient, costimulating the T cells *in vitro* with viral antigen-pulsed APCs either expressing a peptide encoded by extended cDNAs derived from the 5' ESTs of the present invention or together with a stimulatory form of a soluble peptide encoded by extended cDNAs derived from the 5' ESTs of the present invention and reintroducing the *in vitro* primed T cells into the patient. The infected cells would now be capable of delivering a costimulatory signal to T cells *in vivo,* thereby activating the T cells.

In another application, upregulation or enhancement of antigen function (preferably B lymphocyte antigen function) may be useful in the induction of tumor immunity. Tumor cells (e.g., sarcoma, melanoma, lymphoma, leukemia, neuroblastoma, carcinoma) transfected with a nucleic acid encoding at least one peptide encoded by extended cDNAs derived from the 5' ESTs of the present invention can be administered to a subject to overcome tumor-specific tolerance in the subject. If desired, the tumor cell can be transfected to express a combination of peptides. For example, tumor cells obtained from a patient can be transfected *ex vivo* with an expression vector directing the expression of a peptide having B7-2-like activity alone, or in conjunction with a peptide having B7-1-like activity and/or B7-3-like activity. The transfected tumor cells are returned to the patient to result in expression of the peptides on the surface of the transfected cell. Alternatively, gene therapy techniques can be used to target a tumor cell for transfection *in vivo.*

The presence of the peptide encoded by extended cDNAs derived from the 5' ESTs of the present invention having the activity of a B lymphocyte antigen(s) on the surface of the tumor cell provides the necessary costimulation signal to T cells to induce a T cell mediated immune response against the transfected tumor cells. In addition, tumor cells which lack or which fail to reexpress sufficient amounts of MHC class I or MHC class II molecules can be transfected with nucleic acids encoding all or a portion of (e.g., a cytoplasmic-domain truncated portion) of an MHC class I α chain and β₂ microglobulin or an MHC class II α chain and an MHC class II β chain to thereby express MHC class I or MHC class II proteins on the cell surface, respectively. Expression of the appropriate MHC class I or class II molecules in conjunction with a peptide having the activity of a B lymphocyte antigen (e.g., B7-1, B7-2, B7-3) induces a T cell mediated immune response against the transfected tumor cell. Optionally, a gene encoding an antisense construct which blocks expression of an MHC class II associated protein, such as the invariant chain, can also be cotransfected with a DNA encoding a peptide having the activity of a B lymphocyte antigen to promote presentation of tumor associated antigens and induce tumor specific immunity. Thus, the induction of a T cell mediated immune response in a human subject may be sufficient to overcome tumor-specific tolerance in the subject. Alternatively, as described in more detail below, genes encoding these immune system regulator proteins or nucleic acids regulating the expression of such proteins may be introduced into appropriate host cells to increase or decrease the expression of the proteins as desired.

### EXAMPLE 34

### Assaying the Proteins Expressed from Extended cDNAs or Portions Thereof for Hematopoiesis Regulating Activity

The proteins encoded by the extended cDNAs or portions thereof may also be evaluated for their hematopoiesis regulating activity. For example, the effect of the proteins on embryonic stem cell differentiation may be evaluated. Numerous assays for such activity are familiar to those skilled in the art, including the assays disclosed in the following references: Johansson *et al. Cell. Biol.* **15**: 141-151, 1995; Keller *et al., Mol. Cell. Biol.* **13**:473-486, 1993; McClanahan *et al., Blood* **81**:2903-2915, 1993.

The proteins encoded by the extended cDNAs or portions thereof may also be evaluated for their influence on the lifetime of stem cells and stem cell differentiation. Numerous assays for such activity are familiar to those skilled in the art, including the assays disclosed in the following references: Freshney, Methylcellulose Colony Forming Assays, in *Culture of Hematopoietic Cells.,* Freshney, *et al.,* Eds. pp. 265-268, Wiley-Liss, Inc., New York, NY. 1994; Hirayama *et al.,* Proc. *Natl. Acad. Sci. USA* **89**:5907-5911, 1992; McNiece and Briddell, in *Culture of Hematopoietic Cells, supra;* Neben et *al., Exp. Hematol.* **22:**353-359, 1994; Ploemacher and Cobblestone In *Culture of Hematopoietic Cells, supra*1*-*21*,* Spooncer *et al, in Culture of Hematopoietic Cells, supra*163-179 and Sutherland in Culture *of Hematopoietic* Cells, *supra.* 139-162.

Those proteins which exhibit hematopoiesis regulatory activity may then be formulated as pharmaceuticals and used to treat clinical conditions in which regulation of hematopoeisis is beneficial, such as in the treatment of myeloid or lymphoid cell deficiencies. Involvement in regulating hematopoiesis is indicated even by marginal biological activity in support of colony forming cells or of factor-dependent cell lines. For example, proteins supporting the growth and proliferation of erythroid progenitor cells alone or in combination with other cytokines, indicates utility, for example, in treating various anemias or for use in conjunction with irradiation/chemotherapy to stimulate the production of erythroid precursors and/or erythroid cells. Proteins supporting the growth and proliferation of myeloid cells such as granulocytes and monocytes/macrophages (*i.e.,* traditional CSF activity) may be useful, for example, in conjunction with chemotherapy to prevent or treat consequent myelo-suppression. Proteins supporting the growth and proliferation of megakaryocytes and consequently of platelets allows prevention or treatment of various platelet disorders such as thrombocytopenia, and generally may be used in place of or complementary to platelet transfusions. Proteins supporting the growth and proliferation of hematopoietic stem cells which are capable of maturing to any and all of the above-mentioned hematopoietic cells may therefore find therapeutic utility in various stem cell disorders (such as those usually treated with transplantion, including, without limitation, aplastic anemia and paroxysmal nocturnal hemoglobinuria), as well as in repopulating the stem cell compartment post irradiation/chemotherapy, either *in vivo* or *ex vivo* (*i.e*., in conjunction with bone marrow transplantation or with peripheral progenitor cell transplantation (homologous or heterologous)) as normal cells or genetically manipulated for gene therapy. Alternatively, as described in more detail below, genes encoding hematopoiesis regulating activity proteins or nucleic acids regulating the expression of such proteins may be introduced into appropriate host cells to increase or decrease the expression of the proteins as desired.

### EXAMPLE 35

### Assaying the Proteins Expressed from Extended cDNAs or Portions Thereof for Regulation of Tissue Growth

The proteins encoded by the extended cDNAs or portions thereof may also be evaluated for their effect on tissue growth. Numerous assays for such activity are familiar to those skilled in the art, including the assays disclosed in International Patent Publication No. WO95/16035, International Patent Publication No. WO95/05846 and International Patent Publication No. WO91/07491.

Assays for wound healing activity include, without limitation, those described in: Winter, *Epidermal Wound Healing,* pps. 71-112, Maibach and Rovee, eds., Year Book Medical Publishers, Inc., Chicago, as modified by Eaglstein and Mertz, *J*. *Invest.* Dermatol. 71:382-84, 1978.

Those proteins which are involved in the regulation of tissue growth may then be formulated as pharmaceuticals and used to treat clinical conditions in which regulation of tissue growth is beneficial. For example, a protein encoded by extended cDNAs derived from the 5' ESTs of the present invention also may have utility in compositions used for bone, cartilage, tendon, ligament and/or nerve tissue growth or regeneration, as well as for wound healing and tissue repair and replacement, and in the treatment of burns, incisions and ulcers.

A protein encoded by extended cDNAs derived from the 5' ESTs of the present invention, which induces cartilage and/or bone growth in circumstances where bone is not normally formed, has application in the healing of bone fractures and cartilage damage or defects in humans and other animals. Such a preparation employing a protein of the invention may have prophylactic use in closed as well as open fracture reduction and also in the improved fixation of artificial joints. *De novo* bone synthesis induced by an osteogenic agent contributes to the repair of congenital, trauma induced, or oncologic resection induced craniofacial defects, and also is useful in cosmetic plastic surgery.

A protein of this invention may also be used in the treatment of periodontal disease, and in other tooth repair processes. Such agents may provide an environment to attract bone-forming cells, stimulate growth of bone-forming cells or induce differentiation of bone-forming cell progenitors. A protein of the invention may also be useful in the treatment of osteoporosis or osteoarthritis, such as through stimulation of bone and/or cartilage repair or by blocking inflammation or processes of tissue destruction (collagenase activity, osteoclast activity, etc.) mediated by inflammatory processes.

Another category of tissue regeneration activity that may be attributable to the protein encoded by extended cDNAs derived from the 5' ESTs of the present invention is tendon/ligament formation. A protein encoded by extended cDNAs derived from the 5' ESTs of the present invention, which induces tendon/ligament-like tissue or other tissue formation in circumstances where such tissue is not normally formed, has application in the healing of tendon or ligament tears, deformities and other tendon or ligament defects in humans and other animals. Such a preparation employing a tendon/ligament-like tissue inducing protein may have prophylactic use in preventing damage to tendon or ligament tissue, as well as use in the improved fixation of tendon or ligament to bone or other tissues, and in repairing defects to tendon or ligament tissue. *De novo* tendon/ligament-like tissue formation induced by a composition encoded by extended cDNAs derived from the 5' ESTs of the present invention contributes to the repair of tendon or ligaments defects of congenital, traumatic or other origin and is also useful in cosmetic plastic surgery for attachment or repair of tendons or ligaments. The compositions encoded by extended cDNAs derived from the 5' ESTs of the present invention may provide an environment to attract tendon- or ligament-forming cells, stimulate growth of tendon- or ligament-forming cells, induce differentiation of progenitors of tendon- or ligament-forming cells, or induce growth of tendon/ligament cells or progenitors *ex vivo* for return *in vivo* to effect tissue repair. The compositions of the invention may also be useful in the treatment of tendinitis, carpal tunnel syndrome and other tendon or ligament defects. The compositions may also include an appropriate matrix and/or sequestering agent as a carrier as is well known in the art.

The protein encoded by extended cDNAs derived from the 5' ESTs of the present invention may also be useful for proliferation of neural cells and for regeneration of nerve and brain tissue, *i.e.,* for the treatment of central and peripheral nervous system diseases and neuropathies, as well as mechanical and traumatic disorders, which involve degeneration, death or trauma to neural cells or nerve tissue. More specifically, a protein may be used in the treatment of diseases of the peripheral nervous system, such as peripheral nerve injuries, peripheral neuropathy and localized neuropathies, and central nervous system diseases, such as Alzheimer's, Parkinson's disease, Huntington's disease, amyotrophic lateral sclerosis, and Shy-Drager syndrome. Further conditions which may be treated in accordance with the present invention include mechanical and traumatic disorders, such as spinal cord disorders, head trauma and cerebrovascular diseases such as stroke. Peripheral neuropathies resulting from chemotherapy or other medical therapies may also be treatable using a protein of the invention.

Proteins of the invention may also be useful to promote better or faster closure of non-healing wounds, including without limitation pressure ulcers, ulcers associated with vascular insufficiency, surgical and traumatic wounds, and the like.

It is expected that a protein encoded by extended cDNAs derived from the 5' ESTs of the present invention may also exhibit activity for generation or regeneration of other tissues, such as organs (including, for example, pancreas, liver, intestine, kidney, skin, endothelium) muscle (smooth, skeletal or cardiac) and vascular (including vascular endothelium) tissue, or for promoting the growth of cells comprising such tissues. Part of the desired effects may be by inhibition or modulation of fibrotic scarring to allow normal tissue to generate. A protein of the invention may also exhibit angiogenic activity.

A protein encoded by extended cDNAs derived from the 5' ESTs of the present invention may also be useful for gut protection or regeneration and treatment of lung or liver fibrosis, reperfusion injury in various tissues, and conditions resulting from systemic cytokinc damage.

A protein encoded by extended cDNAs derived from the 5' ESTs of the present invention may also be useful for promoting or inhibiting differentiation of tissues described above from precursor tissues or cells; or for inhibiting the growth of tissues described above.

Alternatively, as described in more detail below, genes encoding tissue growth regulating activity proteins or nucleic acids regulating the expression of such proteins may be introduced into appropriate host cells to increase or decrease the expression of the proteins as desired.

### EXAMPLE 36

### Assaying the Proteins Expressed from Extended cDNAs or Portions Thereof for Regulation of Reproductive Hormones

The proteins encoded by the extended cDNAs or portions thereof may also be evaluated for their ability to regulate reproductive hormones, such as follicle stimulating hormone. Numerous assays for such activity are familiar to those skilled in the art, including the assays disclosed in the following references: Vale *et al., Endocrinol.* **91**:562-572, 1972; Ling *et al., Nature* **321**:779-782, 1986; Vale *et al., Nature* **321**:776-779, 1986; Mason *et al., Nature* **318**:659-663, 1985; Forage *et al., Proc. Natl. Acad Sci. USA* **83**:3091-3095, 1986, Chapter 6.12 in *Current Protocols in Immunology,* Coligan *et al.* Eds. Greene Publishing Associates and Wiley-Intersciece ; Taub *et al., J. Clin. Invest.* **95**:1370-1376, 1995; Lind *et al., APMIS* **103**:140-146, 1995; Muller *et al., Eur. J. Immunol.* **25**:1744-1748; Gruber *et al., J. Immunol.* **152**:5860-5867, 1994; Johnston *et al., J Immunol.* **153**:1762-1768, 1994.

Those proteins which exhibit activity as reproductive hormones or regulators of cell movement may then be formulated as pharmaceuticals and used to treat clinical conditions in which regulation of reproductive hormones are beneficial. For example, a protein encoded by extended cDNAs derived from the 5' ESTs of the present invention may also exhibit activin- or inhibin-related activities. Inhibins are characterized by their ability to inhibit the release of follicle stimulating hormone (FSH), while activins are characterized by their ability to stimulate the release of FSH. Thus, a protein encoded by extended cDNAs derived from the 5' ESTs of the present invention, alone or in heterodimers with a member of the inhibin α family, may be useful as a contraceptive based on the ability of inhibins to decrease fertility in female mammals and decrease spermatogenesis in male mammals. Administration of sufficient amounts of other inhibins can induce infertility in these mammals. Alternatively, the protein of the invention, as a homodimer or as a heterodimer with other protein subunits of the inhibin-B group, may be useful as a fertility inducing therapeutic, based upon the ability of activin molecules in stimulating FSH release from cells of the anterior pituitary. See, for example, United States Patent 4,798,885. A protein of the invention may also be useful for advancement of the onset of fertility in sexually immature mammals, so as to increase the lifetime reproductive performance of domestic animals such as cows, sheep and pigs.

Alternatively, as described in more detail below, genes encoding reproductive hormone regulating activity proteins or nucleic acids regulating the expression of such proteins may be introduced into appropriate host cells to increase or decrease the expression of the proteins as desired.

### EXAMPLE 37

### Assaying the Proteins Expressed from Extended cDNAs or Portions Thereof for Chemotactic/Chemokinetic Activity

The proteins encoded by the extended cDNAs or portions thereof may also be evaluated for chemotactic/chemokinetic activity. For example, a protein encoded by extended cDNAs derived from the 5' ESTs of the present invention may have chemotactic or chemokinetic activity (e.g., act as a chemokine) for mammalian cells, including, for example, monocytes, fibroblasts, neutrophils, T-cells, mast cells, eosinophils, epithelial and/or endothelial cells. Chemotactic and chemokinetic proteins can be used to mobilize or attract a desired cell population to a desired site of action. Chemotactic or chemokinetic proteins provide particular advantages in treatment of wounds and other trauma to tissues, as well as in treatment of localized infections. For example, attraction of lymphocytes, monocytes or neutrophils to tumors or sites of infection may result in improved immune responses against the tumor or infecting agent.

A protein or peptide has chemotactic activity for a particular cell population if it can stimulate, directly or indirectly, the directed orientation or movement of such cell population. Preferably, the protein or peptide has the ability to directly stimulate directed movement of cells. Whether a particular protein has chemotactic activity for a population of cells can be readily determined by employing such protein or peptide in any known assay for cell chemotaxis.

The activity of a protein of the invention may, among other means, be measured by the following methods:

Assays for chemotactic activity (which will identify proteins that induce or prevent chemotaxis) consist of assays that measure the ability of a protein to induce the migration of cells across a membrane as well as the ability of a protein to induce the adhesion of one cell population to another cell population. Suitable assays for movement and adhesion include, without limitation, those described in: *Current Protocols in Immunology,* Ed by Coligan, Kruisbeek, Margulies, Shevach and Strober, Pub. Greene Publishing Associates and Wiley-Interscience, Chapter **6.12:** 6.12.1-6.12.28; Taub *et al, J. Clin. Invest.* **95**:1370-1376, 1995; Lind *et al., APMIS* **103**:140-146, 1995; Mueller *et al., Eur. J. Immunol.* **25**:1744-1748; Gruber *et al, J. Immunol.* **152**:5860-5867, 1994; Johnston *et al. J. Immunol.,* **153**:1762-1768, 1994.

### EXAMPLE 38

### Assaying the Proteins Expressed from Extended cDNAs or Portions Thereof for Regulation of Blood Clotting

The proteins encoded by the extended cDNAs or portions thereof may also be evaluated for their effects on blood clotting. Numerous assays for such activity are familiar to those skilled in the art, including the assays disclosed in the following references: Linet *et al., J. Clin. Pharmacol.* **26**:131-140, 1986; Burdick *et al., Thrombosis Res.* **45**:413-419, 1987; Humphrey *et al., Fibrinolysis* **5**:71-79, 1991; Schaub, *Prostaglandins* **35**:467-474, 1988.

Those proteins which are involved in the regulation of blood clotting may then be formulated as pharmaceuticals and used to treat clinical conditions in which regulation of blood clotting is beneficial. For example, a protein of the invention may also exhibit hemostatic or thrombolytic activity. As a result, such a protein is expected to be useful in treatment of various coagulations disorders (including hereditary disorders, such as hemophilias) or to enhance coagulation and other hemostatic events in treating wounds resulting from trauma, surgery or other causes. A protein of the invention may also be useful for dissolving or inhibiting formation of thromboses and for treatment and prevention of conditions resulting therefrom (such as infarction of cardiac and central nervous system vessels (e.g., stroke)). Alternatively, as described in more detail below, genes encoding blood clotting activity proteins or nucleic acids regulating the expression of such proteins may be introduced into appropriate host cells to increase or decrease the expression of the proteins as desired.

### EXAMPLE 39

### Assaying the Proteins Expressed from Extended cDNAs or Portions Thereof for Involvement in Receptor/Ligand Interactions

The proteins encoded by the extended cDNAs or a portion thereof may also be evaluated for their involvement in receptor/ligand interactions. Numerous assays for such involvement are familiar to those skilled in the art, including the assays disclosed in the following references Chapter **7**. 7.28.1-7.28.22 in *Current Protocols in Immunology,* Coligan *et al.* Eds. Greene Publishing Associates and Wiley-Intersdence; Takai *et al., Proc. Natl. Acad Sci. USA* **84**:6864-6868, 1987; Bierer *et al., J. Exp. Med* **168**:1145-1156, 1988; Rosenstein *et al., J.* Exp. *Med.* **169**:149-160, 1989; Stoltenborg *et al., J. Immunol. Methods* **175**:59-68, 1994; Stitt *et al., Cell* **80**:661-670, 1995; Gyuris *et al., Cell* **75**:791-803, 1993.

For example, the proteins encoded by extended cDNAs derived from the 5' ESTs of the present invention may also demonstrate activity as receptors, receptor ligands or inhibitors or agonists of receptor/ligand interactions. Examples of such receptors and ligands include, without limitation, cytokine receptors and their ligands, receptor kinases and their ligands, receptor phosphatases and their ligands, receptors involved in cell-cell interactions and their ligands (including without limitation, cellular adhesion molecules (such as selectins, integrins and their ligands) and receptor/ligand pairs involved in antigen presentation, antigen recognition and development of cellular and humoral immune responses). Receptors and ligands are also useful for screening of potential peptide or small molecule inhibitors of the relevant receptor/ligand interaction. A protein encoded by extended cDNAs derived from the 5' ESTs of the present invention (including, without limitation, fragments of receptors and ligands) may themselves be useful as inhibitors of receptor/ligand interactions. Alternatively, as described in more detail below, genes encoding proteins involved in receptor/ligand interactions or nucleic acids regulating the expression of such proteins may be introduced into appropriate host cells to increase or decrease the expression of the proteins as desired.

### EXAMPLE 40

### Assaying the Proteins Expressed from Extended cDNAs or Portions Thereof for Anti-Inflammatory Activity

The proteins encoded by the extended cDNAs or a portion thereof may also be evaluated for anti-inflammatory activity. The anti-inflammatory activity may be achieved by providing a stimulus to cells involved in the inflammatory response, by inhibiting or promoting cell-cell interactions (such as, for example, cell adhesion), by inhibiting or promoting chemotaxis of cells involved in the inflammatory process, inhibiting or promoting cell extravasation, or by stimulating or suppressing production of other factors which more directly inhibit or promote an inflammatory response. Proteins exhibiting such activities can be used to treat inflammatory conditions including chronic or acute conditions, including without limitation inflammation associated with infection (such as septic shock, sepsis or systemic inflammatory response syndrome), ischemia-reperfusioninury, endotoxin lethality, arthritis, complement-mediated hyperacute rejection, nephritis, cytokine- or chemokine-induced lung injury, inflammatory bowel disease, Crohn's disease or resulting from over production of cytokines such as TNF or IL-1. Proteins of the invention may also be useful to treat anaphylaxis and hypersensitivity to an antigenic substance or material. Alternatively, as described in more detail below, genes encoding anti-inflammatory activity proteins or nucleic acids regulating the expression of such proteins may be introduced into appropriate host cells to increase or decrease the expression of the proteins as desired.

### EXAMPLE 41

### Assaying the Proteins Expressed from Extended cDNAs or Portions Thereof for Tumor Inhibition Activity

The proteins encoded by the extended cDNAs or a portion thereof may also be evaluated for tumor inhibition activity. In addition to the activities described above for immunological treatment or prevention of tumors, a protein of the invention may exhibit other anti-tumor activities. A protein may inhibit tumor growth directly or indirectly (such as, for example, via ADCC). A protein may exhibit its tumor inhibitory activity by acting on tumor tissue or tumor precursor tissue, by inhibiting formation of tissues necessary to support tumor growth (such as, for example, by inhibiting angiogenesis), by causing production of other factors, agents or cell types which inhibit tumor growth, or by suppressing, eliminating or inhibiting factors, agents or cell types which promote tumor growth. Alternatively, as described in more detail below, genes tumor inhibition activity proteins or nucleic acids regulating the expression of such proteins may be introduced into appropriate host cells to increase or decrease the expression of the proteins as desired.

A protein of the invention may also exhibit one or more of the following additional activities or effects: inhibiting the growth, infection or function of, or killing, infectious agents, including, without limitation, bacteria, viruses, fungi and other parasites; effecting (suppressing or enhancing) bodily characteristics, including, without limitation, height, weight, hair color, eye color, skin, fat to lean ratio or other tissue pigmentation, or organ or body part size or shape (such as, for example, breast augmentation or diminution, change in bone form or shape); effecting biorhythms or circadian cycles or rhythms; effecting the fertility of male or female subjects; effecting the metabolism, catabolism, anabolism, processing, utilization, storage or elimination of dietary fat, lipid, protein, carbohydrate, vitamins, minerals, cofactors or other nutritional factors or component(s); effecting behavioral characteristics, including, without limitation, appetite, libido, stress, cognition (including cognitive disorders), depression (including depressive disorders) and violent behaviors; providing analgesic effects or other pain reducing effects; promoting differentiation and growth of embryonic stem cells in lineages other than hematopoietic lineages; hormonal or endocrine activity; in the case of enzymes, correcting deficiencies of the enzyme and treating deficiency-related diseases; treatment of hyperproliferative disorders (such as, for example, psoriasis); immunoglobulin-like activity (such as, for example, the ability to bind antigens or complement); and the ability to act as an antigen in a vaccine composition to raise an immune response against such protein or another material or entity which is cross-reactive with such protein. Alternatively, as described in more detail below, genes encoding proteins involved in any of the above mentioned activities or nucleic acids regulating the expression of such proteins may be introduced into appropriate host cells to increase or decrease the expression of the proteins as desired.

### EXAMPLE 42

### Identification of Proteins which Interact with Polypeptides Encoded by Extended cDNAs

Proteins which interact with the polypeptides encoded by cDNAs derived from the 5' ESTs or fragments thereof, such as receptor proteins, may be identified using two hybrid systems such as the Matchmaker Two Hybrid System 2 (Catalog No. K1604-1, Clontech). As described in the manual accompanying the kit which is incorporated herein by reference, the the cDNAs derived from 5' ESTs, or fragments thereof, are inserted into an expression vector such that they are in frame with DNA encoding the DNA binding domain of the yeast transcriptional activator GAL4. cDNAs in a cDNA library which encode proteins which might interact with the polypeptides encoded by the extended cDNAs or portions thereof are inserted into a second expression vector such that they are in frame with DNA encoding the activation domain of GAL4. The two expression plasmids are transfonned into yeast and the yeast are plated on selection medium which selects for expression of selectable markers on each of the expression vectors as well as GAL4 dependent expression of the HIS3 gene. Transformants capable of growing on medium lacking histidine are screened for GAL4 dependent lacZ expression. Those cells which are positive in both the histidine selection and the lacZ assay contain plasmids encoding proteins which interact with the polypeptide encoded by the extended cDNAs or portions thereof.

Alternatively, the system described in Lustig *et al., Methods in Enzymology* **283**: 83-99, 1997, and in U.S. Patent No. 5,654,150, may be used for identifying molecules which interact with the polypeptides encoded by extended cDNAs. In such systems, *in vitro* transcription reactions are performed on a pool of vectors containing extended cDNA inserts cloned downstream of a promoter which drives *in vitro* transcription. The resulting pools of mRNAs are introduced into *Xenopus laevis* oocytes. The oocytes are then assayed for a desired activity.

Alternatively, the pooled *in vitro* transcription products produced as described above may be translated *in vitro.* The pooled *in vitro* translation products can be assayed for a desired activity or for interaction with a known polypeptide.

Proteins or other molecules interacting with polypeptides encoded by extended cDNAs can be found by a variety of additional techniques. In one method, affinity columns containing the polypeptide encoded by the extended cDNA or a portion thereof can be constructed. In some versions, of this method the affinity column contains chimeric proteins in which the protein encoded by the extended cDNA or a portion thereof is fused to glutathione S-transferase. A mixture of cellular proteins or pool of expressed proteins as described above and is applied to the affinity column. Proteins interacting with the polypeptide attached to the column can then be isolated and analyzed on 2-D electrophoresis gel as described in Ramunsen *et al., Electrophoresis* **18**:588-598, 1997. Alternatively, the proteins retained on the affinity column can be purified by electrophoresis based methods and sequenced. The same method can be used to isolate antibodies, to screen phage display products, or to screen phage display human antibodies.

Proteins interacting with polypeptides encoded by extended cDNAs or portions thereof can also be screened by using an Optical Biosensor as described in Edwards and Leatherbarrow, *Analytical Biochemistry* **246**:1-6, 1997, The main advantage of the method is that it allows the determination of the association rate between the protein and other interacting molecules. Thus, it is possible to specifically select interacting molecules with a high or low association rate. Typically a target molecule is linked to the sensor surface (through a carboxymethl dextran matrix) and a sample of test molecules is placed in contact with the target molecules. The binding of a test molecule to the target molecule causes a change in the refractive index and/ or thickness. This change is detected by the Biosensor provided it occurs in the evanescent field (which extend a few hundred nanometers from the sensor surface). In these screening assays, the target molecule can be one of the polypeptides encoded by extended cDNAs or a portion thereof and the test sample can be a collection of proteins extracted from tissues or cells, a pool of expressed proteins, combinatorial peptide and/ or chemical libraries, or phage displayed peptides. The tissues or cells from which the test proteins are extracted can originate from any species.

In other methods, a target protein is immobilized and the test population is a collection of unique polypeptides encoded by the extended cDNAs or portions thereof.

To study the interaction of the proteins encoded by the extended cDNAs or portions thereof with drugs, the microdialysis coupled to HPLC method described by Wang *et al., Chromatographia* **44**:205-208, 1997 or the affinity capillary electrophoresis method described by Busch *et al., J. Chromatogr.* **777**:311-328, 1997, can be used.

It will be appreciated by those skilled in the art that the proteins expressed from the extended cDNAs or portions may be assayed for numerous activities in addition to those specifically enumerated above. For example, the expressed proteins may be evaluated for applications involving control and regulation of inflammation, tumor proliferation or metastasis, infection, or other clinical conditions. In addition, the proteins expressed from the extended cDNAs or portions thereof may be useful as nutritional agents or cosmetic agents.

The proteins expressed from the cDNAs or portions thereof may be used to generate antibodies capable of specifically binding to the expressed protein or fragments thereof as described in Example 40 below. The antibodies may capable of binding a full length protein encoded by a cDNA derived from a 5' EST, a mature protein (*i.e.* the protein generated by cleavage of the signal peptide) encoded by a cDNA derived from a 5' EST, or a signal peptide encoded by a cDNA derived from a 5' EST. Alternatively, the antibodies may be capable of binding fragments of at least 10 amino acids of the proteins encoded by the above cDNAs. In some embodiments, the antibodies may be capable of binding fragments of at least 15 amino acids of the proteins encoded by the above cDNAs. In other embodiments, the antibodies may be capable of binding fragments of at least 25 amino acids of the proteins expressed from the extended cDNAs which comprise at least 25 amino acids of the proteins encoded by the above cDNAs. In further embodiments, the antibodies may be capable of binding fragments of at least 40 amino acids of the proteins encoded by the above cDNAs.

### EXAMPLE 43

### Production of an Antibody to a Human Protein

Substantially pure protein or polypeptide is isolated from the transfected or transformed cells as described in Example 30. The concentration of protein in the final preparation is adjusted, for example, by concentration on an Amicon filter device, to the level of a few µg/ml. Monoclonal or polyclonal antibody to the protein can then be prepared as follows:

### 1. Monoclonal Antibody Production by Hybridoma Fusion

Monoclonal antibody to epitopes of any of the peptides identified and isolated as described can be prepared from murine hybridomas according to the classical method of Kohler, and Milstein, *Nature* 256:495, 1975 or derivative methods thereof. Briefly, a mouse is repetitively inoculated with a few micrograms of the selected protein or peptides derived therefrom over a period of a few weeks. The mouse is then sacrificed, and the antibody producing cells of the spleen isolated. The spleen cells are fused by means of polyethylene glycol with mouse myeloma cells, and the excess unfused cells destroyed by growth of the system on selective media comprising aminopterin (HAT media). The successfully fused cells are diluted and aliquots of the dilution placed in wells of a microtiter plate where growth of the culture is continued. Antibody-producing clones are identified by detection of antibody in the supernatant fluid of the wells by immunoassay procedures, such as ELISA, as originally described by Engvall, *Meth. Enzymol.* **70**:419, 1980 and derivative methods thereof. Selected positive clones can be expanded and their monoclonal antibody product harvested for use. Detailed procedures for monoclonal antibody production are described in Davis *et al. in Basic Methods in Molecular Biology* Elsevier, New York. Section 21-2.

### 2. Polyclonal Antibody Production by Immunization

Polyclonal antiserum containing antibodies to heterogenous epitopes of a single protein can be prepared by immunizing suitable animals with the expressed protein or peptides derived therefrom, which can be unmodified or modified to enhance immunogenicity. Effecave polyclonal antibody production is affected by many factors related both to the antigen and the host species. For example, small molecules tend to be less immunogenic than others and may require the use of carriers and adjuvant. Also, host animals response vary depending on site of inoculations and doses, with both inadequate or excessive doses of antigen resulting in low titer antisera. Small doses (ng level) of antigen administered at multiple intradermal sites appears to be most reliable. An effective immunization protocol for rabbits can be found in Vaitukaitis. *et al, J. Clin. Endocrinol. Metab.* **33**:988-991 (1971)*.*

Booster injections can be given at regular intervals, and antiserum harvested when antibody titer thereof, as determined semi-quantitatively, for example, by double immunodiffusion in agar against known concentrations of the antigen, begins to fall. See, for example, Ouchterlony, *et al.,* Chap. 19 in: *Handbook of Experimental Immunology* D. Wier (ed) Blackwell (1973). Plateau concentration of antibody is usually in the range of 0.1 to 0.2 mg/ml of serum (about 12 µM). Affinity of the antisera for the antigen is determined by preparing competitive binding curves, as described, for example, by Fisher, D., Chap. 42 in: *Manual of Clinical Immunology,* 2d Ed. (Rose and Friedman, Eds.) Amer. Soc. For Mcrobiol., Washington, D.C. (1980).

Antibody preparations prepared according to either protocol are useful in quantitative immunoassays which determine concentrations of antigen-bearing substances in biological samples; they are also used semi-quantitatively or qualitatively to identify the presence of antigen in a biological sample. The antibodies may also be used in therapeutic compositions for killing cells expressing the protein or reducing the levels of the protein in the body.

### V. Use of 5' ESTs or Sequences Obtainable Therefrom or Portions Thereof as Reagents

The 5' ESTs of the present invention (or cDNAs or genomic DNAs obtainable therefrom) may be used as reagents in isolation procedures, diagnostic assays, and forensic procedures. For example, sequences from the 5' ESTs (or cDNAs or genomic DNAs obtainable therefrom) may be detectably labeled and used as probes to isolate other sequences capable of hybridizing to them. In addition, sequences from 5' ESTs (or cDNAs or genomic DNAs obtainable therefrom) may be used to design PCR primers to be used in isolation, diagnostic, or forensic procedures.

### 1. Use of 5' ESTs or Sequences Obtainable Therefrom or Portions Thereof in Isolation. Diagnostic and Forensic Procedures

### EXAMPLE 44

### Preparation of PCR Primers and Amplification of DNA

The 5' EST sequences (or cDNAs or genomic DNAs obtainable therefrom) may be used to prepare PCR primers for a variety of applications, including isolation procedures for cloning nucleic acids capable of hybridizing to such sequences, diagnostic techniques and forensic techniques. The PCR primers are at least 10 bases, and preferably at least 12, 15, or 17 bases in length. More preferably, the PCR primers are at least 20-30 bases in length. In some embodiments, the PCR primers may be more than 30 bases in length. It is preferred that the primer pairs have approximately the same G/C ratio, so that melting temperatures are approximately the same. A variety of PCR techniques are familiar to those skilled in the art. For a review of PCR technology, see Molecular Cloning to Genetic Engineering, White Ed. *in Methods in Molecular Biology* **67**: Humana Press, Totowa 1997. In each of these PCR procedures, PCR primers on either side of the nucleic acid sequences to be amplified are added to a suitably prepared nucleic acid sample along with dNTPs and a thermostable polymerase such as Taq polymerase, Pfu polymerase, or Vent polymerase. The nucleic acid in the sample is denatured and the PCR primers are specifically hybridized to complementary nucleic acid sequences in the sample. The hybridized primers are extended. Thereafter, another cycle of denaturation, hybridization, and extension is initiated. The cycles are repeated multiple times to produce an amplified fragment containing the nucleic acid sequence between the primer sites.

### EXAMPLE 45

### Use of 5'ESTs as Probes

Probes derived from 5' ESTs (or cDNAs or genomic DNAs obtainable therefrom), including full length cDNAs or genomic sequences, may be labeled with detectable labels familiar to those skilled in the art, including radioisotopes and non-radioactive labels, to provide a detectable probe. The detectable probe may be single stranded or double stranded and may be made using techniques known in the art, including *in vitro* transcription, nick translation, or kinase reactions. A nucleic acid sample containing a sequence capable of hybridizing to the labeled probe is contacted with the labeled probe. If the nucleic acid in the sample is double stranded, it may be denatured prior to contacting the probe. In some applications, the nucleic acid sample may be immobilized on a surface such as a nitrocellulose or nylon membrane. The nucleic acid sample may comprise nucleic acids obtained from a variety of sources, including genomic DNA, cDNA libraries, RNA, or tissue samples.

Procedures used to detect the presence of nucleic acids capable of hybridizing to the detectable probe include well known techniques such as Southern blotting, Northern blotting, dot blotting, colony hybridization, and plaque hybridization. In some applications, the nucleic acid capable of hybridizing to the labeled probe may be cloned into vectors such as expression vectors, sequencing vectors, or *in vitro* transcription vectors to facilitate the characterization and expression of the hybridizing nucleic acids in the sample. For example, such techniques may be used to isolate and clone sequences in a genomic library or cDNA library which are capable of hybridizing to the detectable probe as described in Example 30 above.

PCR primers made as described in Example 44 above may be used in forensic analyses, such as the DNA fingerprinting techniques described in Examples 46-50 below. Such analyses may utilize detectable probes or primers based on the sequences of the the 5' ESTs or of cDNAs or genomic DNAs isolated using the 5' ESTs.

### EXAMPLE 46

### Forensic Matching by DNA Sequencing

In one exemplary method, DNA samples are isolated from forensic specimens of, for example, hair, semen, blood or skin cells by conventional methods. A panel of PCR primers based on a number of the 5' ESTs of Example 25, or cDNAs or genomic DNAs isolated therefrom as described above, is then utilized in accordance with Example 44 to amplify DNA of approximately 100-200 bases in length from the forensic specimen. Corresponding sequences are obtained from a test subject. Each of these identification DNAs is then sequenced using standard techniques, and a simple database comparison determines the differences, if any, between the sequences from the subject and those from the sample. Statistically significant differences between the suspect's DNA sequences and those from the sample conclusively prove a lack of identity. This lack of identity can be proven, for example, with only one sequence. Identity, on the other hand, should be demonstrated with a large number of sequences, all matching. Preferably, a minimum of 50 statistically identical sequences of 100 bases in length are used to prove identity between the suspect and the sample.

### EXAMPLE 47

### Positive Identification by DNA Sequencing

The technique outlined in the previous example may also be used on a larger scale to provide a unique fingerprint-type identification of any individual. In this technique, primers are prepared from a large number of 5'EST sequences from Example 25, or cDNA or genomic DNA sequences obtainable therefrom. Preferably, 20 to 50 different primers are used. These primers are used to obtain a corresponding number of PCR-generated DNA segments from the individual in question in accordance with Example 44. Each of these DNA segments is sequenced, using the methods set forth in Example 46. The database of sequences generated through this procedure uniquely identifies the individual from whom the sequences were obtained. The same panel of primers may then be used at any later time to absolutely correlate tissue or other biological specimen with that individual.

### EXAMPLE 48

### Southern Blot Forensic Identification

The procedure of Example 47 is repeated to obtain a panel of at least 10 amplified sequences from an individual and a specimen. Preferably, the panel contains at least 50 amplified sequences. More preferably, the panel contains 100 amplified sequences. In some embodiments, the panel contains 200 amplified sequences. This PCR-generated DNA is then digested with one or a combination of; preferably, four base specific restriction enzymes. Such enzymes are commercially available and known to those of skill in the art. After digestion, the resultant gene fragments are size separated in multiple duplicate wells on an agarose gel and transferred to nitrocellulose using Southern blotting techniques well known to those with skill in the art. For a review of Southern blotting see Davis *et al.* (Basic Methods in Molecular Biology, 1986, Elsevier Press. pp 62-65).

A panel of probes based on the sequences of 5' ESTs (or cDNAs or genomic DNAs obtainable therefrom), or fragments thereof of at least 10 bases, are radioactively or colorimetrically labeled using methods known in the art, such as nick translation or end labeling, and hybridized to the Southern blot using techniques known in the art (Davis *et al.,* supra). Preferably, the probe comprises at least 12, 15, or 17 consecutive nucleotides from the 5' EST (or cDNAs or genomic DNAs obtainable therefrom). More preferably, the probe comprises at least 20-30 consecutive nucleotides from the 5' EST (or cDNAs or genomic DNAs obtainable therefrom). In some embodiments, the probe comprises more than 30 nucleotides from the 5' EST (or cDNAs or genomic DNAs obtainable therefrom).

Preferably, at least 5 to 10 of these labeled probes are used, and more preferably at least about 20 or 30 are used to provide a unique pattern. The resultant bands appearing from the hybridization of a large sample of 5' EST (or cDNAs or genomic DNAs obtainable therefrom) will be a unique identifier. Since the restriction enzyme cleavage will be different for every individual, the band pattern on the Southern blot will also be unique. Increasing the number of 5' EST (or cDNAs or genomic DNAs obtainable therefrom) probes will provide a statistically higher level of confidence in the identification since there will be an increased number of sets of bands used for identification.

### EXAMPLE 49

### Dot Blot Identification Procedure

Another technique for identifying individuals using the 5' EST sequences disclosed herein utilizes a dot blot hybridization technique.

Genomic DNA is isolated from nuclei of subject to be identified. Oligonucleotide probes of approximately 30 bp in length are synthesized that correspond to at least 10, preferably 50 sequences from the 5' ESTs or cDNAs or genomic DNAs obtainable therefrom. The probes are used to hybridize to the genomic DNA through conditions known to those in the art. The oligonucleotides are end labeled with P³² using polynucleotide kinase (pharmacia). Dot Blots are created by spotting the genomic DNA onto nitrocellulose or the like using a vacuum dot blot manifold (BioRad, Richmond California). The nitrocellulose filter containing the genomic sequences is baked or UV linked to the filter, prehybridized and hybridized with labeled probe using techniques known in the art (Davis et *al., supra).* The ³²P labeled DNA fragments are sequentially hybridized with successively stringent conditions to detect minimal differences between the 30 bp sequence and the DNA Tetramethylammonium chloride is useful for identifying clones containing small numbers of nucleotide mismatches (Wood *et al., Proc. Natl. Acad Sci. USA* **82**(6):1585-1588, 1985). A unique pattern of dots distinguishes one individual from another individual.

5' EST sequences (or cDNAs or genomic DNAs obtainable therefrom) or oligonucleotides containing at least 10 consecutive bases from these sequences can be used as probes in the following alternative fingerprinting technique. Preferably, the probe comprises at least 12, 15, or 17 consecutive nucleotides from the 5' EST sequences (or cDNAs or genomic DNAs obtainable therefrom). More preferably, the probe comprises at least 20-30 consecutive nucleotides from the 5' EST sequences (or cDNAs or genomic DNAs obtainable therefrom). In some embodiments, the probe comprises more than 30 nucleotides from the 5' EST sequences (or cDNAs or genomic DNAs obtainable therefrom).

Preferably, a plurality of probes having sequences from different genes are used in the alternative fingerprinting technique. Example 50 below provides a representative alternative fingerprinting procedure in which the probes are derived from 5'EST.

### EXAMPLE 50

### Alternative "Fingerprint" Identification Technique

20-mer oligonucleotides are prepared from a large number, e.g. 50, 100, or 200, of 5'EST using commercially available oligonucleotide services such as Genset, Paris, France. Cell samples from the test subject are processed for DNA using techniques well known to those with skill in the art. The nucleic acid is digested with restriction enzymes such as EcoRI and Xbals. Following digestion, samples are applied to wells for electrophoresis. The procedure, as known in the art, may be modified to accommodate polyacrylamide electrophoresis, however in this example, samples containing 5 ug of DNA are loaded into wells and separated on 0.8% agarose gels. The gels are transferred onto nitrocellulose using standard Southern blotting techniques.

10 ng of each of the oligonucleotides are pooled and end-labeled with ³²P. The nitrocellulose is prehybridized with blocking solution and hybridized with the labeled probes. Following hybridization and washing, the nitrocellulose filter is exposed to X-Omat AR X-ray film. The resulting hybridization pattern will be unique for each individual.

It is additionally contemplated within this example that the number of probe sequences used can be varied for additional accuracy or clarity.

The proteins encoded by the extended cDNAs may also be used to generate antibodies as explained in Examples 30 and 43 in order to identify the tissue type or cell species from which a sample is derived as described in example 51.

### EXAMPLE 51

### Identification of Tissue Types or Cell Species by Means of Labeled Tissue Specific Antibodies

Identification of specific tissues is accomplished by the visualization of tissue specific antigens by means of antibody preparations according to Examples 30 and 43 which are conjugated, directly or indirectly to a detectable marker. Selected labeled antibody species bind to their specific antigen binding partner in tissue sections, cell suspensions, or in extracts of soluble proteins from a tissue sample to provide a pattern for qualitative or semi-qualitative interpretation.

Antisera for these procedures must have a potency exceeding that of the native preparation, and for that reason, antibodies are concentrated to a mg/ml level by isolation of the gamma globulin fraction, for example, by ion-exchange chromatography or by ammonium sulfate fractionation. Also, to provide the most specific antisera, unwanted antibodies, for example to common proteins must be removed from the gamma globulin fraction, for example by means of insoluble immunoabsorbents, before the antibodies are labeled with the marker. Either monoclonal or heterologous antisera is suitable for either procedure.

### A. Immunohistochemical techniques

Purified, high-titer antibodies, prepared as described above, are conjugated to a detectable marker, as described, for example, by Fudenberg, Chap. 26 in: *Basic and Clinical Immunology,* 3rd Ed. Lange, Los Altos, California, 1980, or Rose,. *et al*., Chap. 12 in: *Methods in Immunodiagnosis,* 2d Ed. John Wiley and Sons, New York (1980).

A fluorescent marker, either fluorescein or rhodamine, is preferred, but antibodies can also be labeled with an enzyme that supports a color producing reaction with a substrate, such as horseradish peroxidase. Markers can be added to tissue-bound antibody in a second step, as described below. Alternatively, the specific antitissue antibodies can be labeled with ferritin or other electron dense particles, and localization of the ferritin coupled antigen-antibody complexes achieved by means of an electron microscope. In yet another approach, the antibodies are radiolabeled, with, for example ¹²⁵I, and detected by overlaying the antibody treated preparation with photographic emulsion.

Preparations to carry out the procedures can comprise monoclonal or polyclonal antibodies to a single protein or peptide identified as specific to a tissue type, for example, brain tissue, or antibody preparations to several antigenically distinct tissue specific antigens can be used in panels, independently or in mixtures, as required.

Tissue sections and cell suspensions are prepared for immunohistochemical examination according to common histological techniques. Multiple cryostat sections (about 4 µm, unfixed) of the unknown tissue and known control, are mounted and each slide covered with different dilutions of the antibody preparation. Sections of known and unknown tissues should also be treated with preparations to provide a positive control, a negative control, for example, pre-immune sera, and a control for non-specific staining, for example, buffer.

Treated sections are incubated in a humid chamber for 30 min at room temperature, rinsed, then washed in buffer for 30-45 min. Excess fluid is blotted away, and the marker developed.

If the tissue specific antibody was not labeled in the first incubation, it can be labeled at this time in a second antibody-antibody reaction, for example, by adding fluorescein- or enzyme-conjugated antibody against the immunoglobulin class of the antiserum-producing species, for example, fluorescein labeled antibody to mouse IgG. Such labeled sera are commercially available.

The antigen found in the tissues by the above procedure can be quantified by measuring the intensity of color or fluorescence on the tissue section, and calibrating that signal using appropriate standards.

### B. Identification of tissue specific soluble proteins

The visualization of tissue specific proteins and identification of unknown tissues from that procedure is carried out using the labeled antibody reagents and detection strategy as described for immunohistochemistry; however the sample is prepared according to an electrophoretic technique to distribute the proteins extracted from the tissue in an orderly array on the basis of molecular weight for detection.

A tissue sample is homogenized using a Virtis apparatus; cell suspensions are disrupted by Dounce homogenization or osmotic lysis, using detergents in either case as required to disrupt cell membranes, as is the practice in the art. Insoluble cell components such as nuclei, microsomes, and membrane fragments are removed by ultracentrifugation, and the soluble protein-containing fraction concentrated if necessary and reserved for analysis.

A sample of the soluble protein solution is resolved into individual protein species by conventional SDS polyacrylamide electrophoresis as described, for example, by *Davis, et al.,* Section **19-2** in: *Basic Methods in Molecular Biology,* Leder ed., Elsevier, New York, 1986 using a range of amounts of polyacrylamide in a set of gels to resolve the entire molecular weight range of proteins to be detected in the sample. A size marker is run in parallel for purposes of estimating molecular weights of the constituent proteins. Sample size for analysis is a convenient volume of from 5 to 55 µl, and containing from about 1 to 100 µg protein. An aliquot of each of the resolved proteins is transferred by blotting to a nitrocellulose filter paper, a process that maintains the pattern of resolution. Multiple copies are prepared. The procedure, known as Western Blot Analysis, is well described in Davis, L. *et al., supra* Section 19-3. One set of nitrocellulose blots is stained with Coomassie blue dye to visualize the entire set of proteins for comparison with the antibody bound proteins. The remaining nitrocellulose filters are then incubated with a solution of one or more specific antisera to tissue specific proteins prepared as described in Examples 30 and 43. In this procedure, as in procedure A above, appropriate positive and negative sample and reagent controls are run.

In either procedure A or B, a detectable label can be attached to the primary tissue antigen-primary antibody complex according to various strategies and permutations thereof In a straightforward approach, the primary specific antibody can be labeled; alternatively, the unlabeled complex can be bound by a labeled secondary anti-IgG antibody. In other approaches, either the primary or secondary antibody is conjugated to a biotin molecule, which can, in a subsequent step, bind an avidin conjugated marker. According to yet another strategy, enzyme labeled or radioactive protein A, which has the property of binding to any IgG, is bound in a final step to either the primary or secondary antibody.

The visualization of tissue specific antigen binding at levels above those seen in control tissues to one or more tissue specific antibodies, prepared from the gene sequences identified from extended cDNA sequences, can identify tissues of unknown origin, for example, forensic samples, or differentiated tumor tissue that has metastasized to foreign bodily sites.

In addition to their applications in forensics and identification, 5' ESTs (or cDNAs or genomic DNAs obtainable therefrom) may be mapped to their chromosomal locations. Example 52 below describes radiation hybrid (RH) mapping of human chromosomal regions using 5'ESTs. Example 53 below describes a representative procedure for mapping an 5' EST to its location on a human chromosome. Example 54 below describes mapping of 5' ESTs on metaphase chromosomes by Fluorescence In Situ Hybridization (FISH). Those skilled in the art will appreciate that the method of Examples 52-54 may also be used to map cDNAs or genomic DNAs obtainable from the 5' ESTs to their chromosomal locations.

### 2. Use of 5' ESTs or Sequences Obtainable Therefrom or Portions Thereof in Chromosome Mapping

### EXAMPLE 52

### Radiation hybrid mapping of 5'ESTs to the human genome

Radiation hybrid (RH) mapping is a somatic cell genetic approach that can be used for high resolution mapping of the human genome. In this approach, cell lines containing one or more human chromosomes are lethally irradiated, breaking each chromosome into fragments whose size depends on the radiation dose. These fragments are rescued by fusion with cultured rodent cells, yielding subclones containing different portions of the human genome. This technique is described by Benham *et al., Genomics* **4:**509-517, 1989; and Cox *et al., Science* **250**:245-250, 1990. The random and independent nature of the subclones permits efficient mapping of any human genome marker. Human DNA isolated from a panel of 80-100 cell lines provides a mapping reagent for ordering 5'EST. In this approach, the frequency of breakage between markers is used to measure distance, allowing construction of fine resolution maps as has been done using conventional ESTs (Schuler *et al., Science* **274**:540-546, 1996).

RH mapping has been used to generate a high-resolution whole genome radiation hybrid map of human chromosome 17q22-q25.3 across the genes for growth hormone (GH) and thymidine kinase (TK) (Foster et *al., Genomics* **33**:185-192, 1996), the region surrounding the Gorlin syndrome gene (Obermayr *et al., Eur. J. Hum. Genet.* **4**:242-245, 1996), 60 loci covering the entire short arm of chromosome 12 (Raeymaekers *et al., Genomics* **29**:170-178, 1995), the region of human chromosome 22 containing the neurofibromatosis type 2 locus (Frazer *et al., Genomics* **14:**574-584, 1992) and 13 loci on the long arm of chromosome 5 (Warrington *et al., Genomics* **11**: 701-708, 1991).

### EXAMPLE 53

### Mapping of 5'ESTs to HumanChromosomes using PCR techniques

5' ESTs (or cDNAs or genomic DNAs obtainable therefrom) may be assigned to human chromosomes using PCR based methodologies. In such approaches, oligonucleotide primer pairs are designed from the 5' ESTs (or cDNAs or genomic DNAs obtainable therefrom) to minimize the chance of amplifying through an intron. Preferably, the oligonucleotide primers are 18-23 bp in length and are designed for PCR amplification. The creation of PCR primers from known sequences is well known to those with skill in the art. For a review of PCR technology see Erlich in *PCR Technology, Principles and Applications for DNA Amplification,* Freeman and Co., New York, 1992.

The primers are used in polymerase chain reactions (PCR) to amplify templates from total human genomic DNA. PCR conditions are as follows: 60 ng of genomic DNA is used as a template for PCR with 80 ng of each oligonucleotide primer, 0.6 unit of Taq polymerase, and 1 µCu of a ³²P-labeled deoxycytidine triphosphate. The PCR is performed in a microptate thermocycler (Techne) under the following conditions: 30 cycles of 94°C, 1.4 min; 55°C, 2 min; and 72°C, 2 min; with a final extension at 72°C for 10 min. The amplified products are analyzed on a 6% polyacrylamide sequencing gel and visualized by autoradiography. If the length of the resulting PCR product is identical to the distance between the ends of the primer sequences in the extended cDNA from which the primers are derived, then the PCR reaction is repeated with DNA templates from two panels of human-rodent somatic cell hybrids, BIOS PCRable DNA (BIOS Corporation) and NIGMS Human-Rodent Somatic Cell Hybrid Mapping Panel Number 1 (NIGMS, Camden, NJ).

PCR is used to screen a series of somatic cell hybrid cell lines containing defined sets of human chromosomes for the presence of a given 5' EST (or cDNA or genomic DNA obtainable therefrom). DNA is isolated from the somatic hybrids and used as starting templates for PCR_reactions using the primer pairs from the 5' EST (or cDNA or genomic DNA obtainable therefrom). Only those somatic cell hybrids with chromosomes containing the human gene corresponding to the 5' EST (or cDNA or genomic DNA obtainable therefrom) will yield an amplified fragment. The 5' EST (or cDNA or genomic DNA obtainable therefrom) are assigned to a chromosome by analysis of the segregation pattern of PCR products from the somatic hybrid DNA templates. The single human chromosome present in all cell hybrids that give rise to an amplified fragment is the chromosome containing that 5'EST (or cDNA or genomic DNA obtainable therefrom). For a review of techniques and analysis of results from somatic cell gene mapping experiments, see *Ledbetter et al., Genomics* **6**:475-481,1990.

### EXAMPLE 54

### Mapping of Extended 5' ESTs to Chromosomes Using Fluorescence In Situ Hybridization

Fluorescence in situ hybridization allows the 5'EST (or cDNA or genomic DNA obtainable therefrom) to be mapped to a particular location on a given chromosome. The chromosomes to be used for fluorescence in situ hybridization techniques may be obtained from a variety of sources including cell cultures, tissues, or whole blood.

In a preferred embodiment, chromosomal localization of an 5'EST (or cDNA or genomic DNA obtainable therefrom) is obtained by FISH as described by Cherif *et al. (Proc. Natl. Acad Sci. U.SA.,* **87**:6639-6643, 1990). Metaphase chromosomes are prepared from phytohemagglutinin (PHA)-stimulated blood cell donors. PHA-stimulated lymphocytes from healthy males are cultured for 72 h in RPMI-1640 medium. For synchronization, methotrexate (10 µM) is added for 17 h, followed by addition of 5-bromodeoxyuridine (5-BrdU, 0.1 mM) for 6 h. Colcemid (1 µg/ml) is added for the last 15 min before harvesting the cells. Cells are collected, washed in RPMI, incubated with a hypotonic solution of KCl (75 mM) at 37°C for 15 min and fixed in three changes of methanol:acetic acid (3:1). The cell suspension is dropped onto a glass slide and air dried. The 5'EST (or cDNA or genomic DNA obtainable therefrom) is labeled with biotin-16 dUTP by nick translation according to the manufacturer's instructions (Bethesda Research Laboratories, Bethesda, MD), purified using a Sephadex G-50 column (Pharmacia, Upsala, Sweden) and precipitated. Just prior to hybridization, the DNA pellet is dissolved in hybridization buffer (50% formamide, 2 X SSC, 10% dextran sulfate, 1 mg/ml sonicated salmon sperm DNA, pH 7) and the probe is denatured at 70°C for 5-10 min.

Slides kept at -20°C are treated for 1 h at 37°C with RNase A (100 µg/ml), rinsed three times in 2 X SSC and dehydrated in an ethanol series. Chromosome preparations are denatured in 70% formamide, 2 X SSC for 2 min at 70°C, then dehydrated at 4°C. The slides are treated with proteinase K (10 µg/100 ml in 20 mM Tris-HCl, 2 mM CaCl₂) at 37°C for 8 min and dehydrated. The hybridization mixture containing the probe is placed on the slide, covered with a coverslip, sealed with rubber cement and incubated overnight in a humid chamber at 37°C. After hybridization and post-hybridization washes, the biotinylated probe is detected by avidin-FITC and amplified with additional layers of biotinylated goat anti-avidin and avidin-FITC. For chromosomal localization, fluorescent R-bands are obtained as previously described (Cherif *et al., supra).* The slides are observed under a LEICA fluorescence microscope (DMRXA). Chromosomes are counterstained with propidium iodide and the fluorescent signal of the probe appears as two symmetrical yellow-green spots on both chromatids of the fluorescent R-band chromosome (red). Thus, a particular 5'EST (or cDNA or genomic DNA obtainable therefrom) may be localized to a particular cytogenetic R-band on a given chromosome.

Once the 5'EST (or cDNA or genomic DNA obtainable therefrom) have been assigned to particular chromosomes using the techniques described in Examples 52-54 above, they may be utilized to construct a high resolution map of the chromosomes on which they are located or to identify the chromosomes in a sample.

### EXAMPLE 55

### Use of 5'EST to Construct or Expand Chromosome Maps

Chromosome mapping involves assigning a given unique sequence to a particular chromosome as described above. Once the unique sequence has been mapped to a given chromosome, it is ordered relative to other unique sequences located on the same chromosome. One approach to chromosome mapping utilizes a series of yeast artificial chromosomes (YACs) bearing several thousand long inserts derived from the chromosomes of the organism from which the extended cDNAs (or genomic DNAs obtainable therefrom) are obtained. This approach is described in Nagaraja *et al., Genome Research* **7**:210-222, 1997. Briefly, in this approach each chromosome is broken into overlapping pieces which are inserted into the YAC vector. The YAC inserts are screened using PCR or other methods to determine whether they include the 5'EST (or cDNA or genomic DNA obtainable therefrom) whose position is to be determined. Once an insert has been found which includes the 5'EST (or cDNA or genomic DNA obtainable therefrom), the insert can be analyzed by PCR or other methods to determine whether the insert also contains other sequences known to be on the chromosome or in the region from which the 5'EST (or cDNA or genomic DNA obtainable therefrom) was derived. This process can be repeated for each insert in the YAC library to determine the location of each of the extended cDNAs (or genomic DNAs obtainable therefrom) relative to one another and to other known chromosomal markers. In this way, a high resolution map of the distribution of numerous unique markers along each of the organisms chromosomes may be obtained.

As described in Example 56 below extended cDNAs (or genomic DNAs obtainable therefrom) may also be used to identify genes associated with a particular phenotype, such as hereditary disease or drug response.

### 3. Use of 5'ESTs or Sequences Obtained Therefrom or Fragments Thereof in Gene Identification

### EXAMPLE 56

### Identification of genes associated with hereditary diseases or drug response

This example illustrates an approach useful for the association of 5'ESTs (or cDNA or genomic DNA obtainable therefrom) with particular phenotypic characteristics. In this example, a particular 5'EST (or cDNA or genomic DNA obtainable therefrom) is used as a test probe to associate that 5'EST (or cDNA or genomic DNA obtainable therefrom) with a particular phenotypic characteristic.

5'ESTs (or cDNA or genomic DNA obtainable therefrom) are mapped to a particular location on a human chromosome using techniques such as those described in Examples 52 and 53 or other techniques known in the art. A search of Mendelian Inheritance in Man (McKusick in *Mendelian Inheritance in Man* (available on line through Johns Hopkins University Welch Medical Library) reveals the region of the human chromosome which contains the 5'EST (or cDNA or genomic DNA obtainable therefrom) to be a very gene rich region containing several known genes and several diseases or phenotypes for which genes have not been identified. The gene corresponding to this 5'EST (or cDNA or genomic DNA obtainable therefrom) thus becomes an immediate candidate for each of these genetic diseases.

Cells from patients with these diseases or phenotypes are isolated and expanded in culture. PCR primers from the 5'EST (or cDNA or genomic DNA obtainable therefrom) are used to screen genomic DNA, mRNA or cDNA obtained from the patients. 5'ESTs (or cDNA or genomic DNA obtainable therefrom) that are not amplified in the patients can be positively associated with a particular disease by further analysis. Alternatively, the PCR analysis may yield fragments of different lengths when the samples are derived from an individual having the phenotype associated with the disease than when the sample is derived from a healthy individual, indicating that the gene containing the 5'EST may be responsible for the genetic disease.

### VI. Use of 5'EST (or cDNA or Genomic DNA Obtainable Therefrom) to Construct Vectors

The present 5'ESTs (or cDNA or genomic DNA obtainable therefrom) may also be used to construct secretion vectors capable of directing the secretion of the proteins encoded by genes therein. Such secretion vectors may facilitate the purification or enrichment of the proteins encoded by genes inserted therein by reducing the number of background proteins from which the desired protein must be purified or enriched. Exemplary secretion vectors are described in Example 57 below.

### 1. Construction of Secretion Vectors

### EXAMPLE 57

### Construction of Secretion Vectors

The secretion vectors include a promoter capable of directing gene expression in the host cell, tissue, or organism of interest. Such promoters include the Rous Sarcoma Virus promoter, the SV40 promoter, the human cytomegalovirus promoter, and other promoters familiar to those skilled in the art.

A signal sequence from a 5' EST (or cDNAs or genomic DNAs obtainable therefrom) is operably linked to the promoter such that the mRNA transcribed from the promoter will direct the translation of the signal peptide. The host cell, tissue, or organism may be any cell, tissue, or organism which recognizes the signal peptide encoded by the signal sequence in the 5' EST (or cDNA or genomic DNA obtainable therefrom). Suitable hosts include mammalian cells, tissues or organisms, avian cells, tissues, or organisms, insect cells, tissues or organisms, or yeast.

In addition, the secretion vector contains cloning sites for inserting genes encoding the proteins which are to be secreted. The cloning sites facilitate the cloning of the insert gene in frame with the signal sequence such that a fusion protein in which the signal peptide is fused to the protein encoded by the inserted gene is expressed from the mRNA transcribed from the promoter. The signal peptide directs the extracellular secretion of the fusion protein.

The secretion vector may be DNA or RNA and may integrate into the chromosome of the host, be stably maintained as an extrachromosomal replicon in the host, be an artificial chromosome, or be transiently present in the host. Many nucleic acid backbones suitable for use as secretion vectors are known to those skilled in the art, including retroviral vectors, SV40 vectors, Bovine Papilloma Virus vectors, yeast integrating plasmids, yeast episomal plasmids, yeast artificial chromosomes, human artificial chromosomes, P element vectors, baculovirus vectors, or bacterial plasmids capable of being transiently introduced into the host.

The secretion vector may also contain a polyA signal such that the polyA signal is located downstream of the gene inserted into the secretion vector.

After the gene encoding the protein for which secretion is desired is inserted into the secretion vector, the secretion vector is introduced into the host cell, tissue, or organism using calcium phosphate precipitation, DEAE-Dextran, electroporation, liposome-mediated transfection, viral particles or as naked DNA. The protein encoded by the inserted gene is then purified or enriched from the supernatant using conventional techniques such as ammonium sulfate precipitation, immunoprecipitation, immunochromatography, size exclusion chromatography, ion exchange chromatography, and HPLC. Alternatively, the secreted protein may be in a sufficiently enriched or pure state in the supernatant or growth media of the host to permit it to be used for its intended purpose without further enrichment.

The signal sequences may also be inserted into vectors designed for gene therapy. In such vectors, the signal sequence is operably linked to a promoter such that mRNA transcribed from the promoter encodes the signal peptide. A cloning site is located downstream of the signal sequence such that a gene encoding a protein whose secretion is desired may readily be inserted into the vector and fused to the signal sequence. The vector is introduced into an appropriate host cell The protein expressed from the promoter is secreted extracellularly, thereby producing a therapeutic effect.

The 5' ESTs may also be used to clone sequences located upstream of the 5' ESTs which are capable of regulating gene expression, including promoter sequences, enhancer sequences, and other upstream sequences which influence transcription or translation levels. Once identified and cloned, these upstream regulatory sequences may be used in expression vectors designed to direct the expression of an inserted gene in a desired spatial, temporal, developmental, or quantitative fashion. Example 58 describes a method for cloning sequences upstream of the extended cDNAs or 5' ESTs.

### 2. Identification of Upstream Sequences With Promoting or Regulatory Activities

### EXAMPLE 58

### Use of Extended cDNAs or 5' ESTs to Clone Upstream Sequences from Genomic DNA

Sequences derived from extended cDNAs or 5' ESTs may be used to isolate the promoters of the corresponding genes using chromosome walking techniques. In one chromosome walking technique, which utilizes the GenomeWalker^{™} kit available from Clontech, five complete genomic DNA samples are each digested with a different restriction enzyme which has a 6 base recognition site and leaves a blunt end. Following digestion, oligonucleotide adapters are ligated to each end of the resulting genomic DNA fragments.

For each of the five genomic DNA libraries, a first PCR reaction is performed according to the manufacturer's instructions using an outer adaptor primer provided in the kit and an outer gene specific primer. The gene specific primer should be selected to be specific for the extended cDNA or 5' EST of interest and should have a melting temperature, length, and location in the extended cDNA or 5'EST which is consistent with its use in PCR reactions. Each first PCR reaction contains 5 ng of genomic DNA, 5 µl of 10X Tth reaction buffer, 0.2 mM of each dNTP, 0.2 µM each of outer adaptor primer and outer gene specific primer, 1.1 mM of Mg(OAc)₂, and 1 µl of the Tth polymerase 50X mix in a total volume of 50 µl. The reaction cycle for the first PCR reaction is as follows: 1 min - 94°C / 2 sec - 94°C, 3 min - 72°C (7 cycles) / 2 sec - 94°C, 3 min - 67°C (32 cycles) / 5 min - 67°C._{.}

The product of the first PCR reaction is diluted and used as a template for a second PCR reaction according to the manufacturer's instructions using a pair of nested primers which are located internally on the amplicon resulting from the first PCR reaction. For example, 5 µl of the reaction product of the first PCR reaction mixture may be diluted 180 times. Reactions are made in a 50 µl volume having a composition identical to that of the first PCR reaction except the nested primers are used. The first nested primer is specific for the adaptor, and is provided with the GenomeWalker^{™} kit. The second nested primer is specific for the particular extended cDNA or 5' EST for which the promoter is to be cloned and should have a melting temperature, length, and location in the extended cDNA or 5' EST which is consistent with its use in PCR reactions. The reaction parameters of the second PCR reaction are as follows: 1 min - 94°C / 2 sec - 94°C, 3 min - 72°C (6 cycles) / 2 sec - 94°C, 3 min - 67°C (25 cycles) / 5 min - 67°C. The product of the second PCR reaction is purified, cloned, and sequenced using standard techniques.

Alternatively, two or more human genomic DNA libraries can be constructed by using two or more restriction enzymes. The digested genomic DNA is cloned into vectors which can be converted into single stranded, circular, or linear DNA. A biotinylated oligonucleotide comprising at least 15 nucleotides from the extended cDNA or 5' EST sequence is hybridized to the single stranded DNA. Hybrids between the biotinylated oligonucleotide and the single stranded DNA containing the extended cDNA or EST sequence are isolated as described in Example 29 above. Thereafter, the single stranded DNA containing the extended cDNA or EST sequence is released from the beads and converted into double stranded DNA using a primer specific for the extended cDNA or 5' EST sequence or a primer corresponding to a sequence included in the cloning vector. The resulting double stranded DNA is transformed into bacteria. DNAs containing the 5' EST or extended cDNA sequences are identified by colony PCR or colony hybridization.

Once the upstream genomic sequences have been cloned and sequenced as described above, prospective promoters and transcription start sites within the upstream sequences may be identified by comparing the sequences upstream of the extended cDNAs or 5' ESTs with databases containing known transcription start sites, transcription factor binding sites, or promoter sequences.

In addition, promoters in the upstream sequences may be identified using promoter reporter vectors as described in Example.

### EXAMPLE 59

### Identification of Promoters in Cloned Upstream Sequences

The genomic sequences upstream of the extended cDNAs or 5' ESTs are cloned into a suitable promoter reporter vector, such as the pSEAP-Basic, pSEAP-Enhancer, pβgal-Basic, pβgal-Enhancer, or pEGFP-1 Promoter Reporter vectors available from Clontech. Briefly, each of these promoter reporter vectors include multiple cloning sites positioned upstream of a reporter gene encoding a readily assayable protein such as secreted alkaline phosphatase, β galactosidase, or green fluorescent protein. The sequences upstream of the extended cDNAs or 5' ESTs are inserted into the cloning sites upstream of the reporter gene in both orientations and introduced into an appropriate host cell. The level of reporter protein is assayed and compared to the level obtained from a vector which lacks an insert in the cloning site. The presence of an elevated expression level in the vector containing the insert with respect to the control vector indicates the presence of a promoter in the insert. If necessary, the upstream sequences can be cloned into vectors which contain an enhancer for augmenting transcription levels from weak promoter sequences. A significant level of expression above that observed with the vector lacking an insert indicates that a promoter sequence is present in the inserted upstream sequence.

Appropriate host cells for the promoter reporter vectors may be chosen based on the results of the above described determination of expression patterns of the extended cDNAs and ESTs. For example, if the expression pattern analysis indicates that the mRNA corresponding to a particular extended cDNA or 5' EST is expressed in fibroblasts, the promoter reporter vector may be introduced into a human fibroblast cell line.

Promoter sequences within the upstream genomic DNA may be further defined by constructing nested deletions in the upstream DNA using conventional techniques such as Exonuclease III digestion. The resulting deletion fragments can be inserted into the promoter reporter vector to determine whether the deletion has reduced or obliterated promoter activity. In this way, the boundaries of the promoters may be defined. If desired, potential individual regulatory sites within the promoter may be identified using site directed mutagenesis or linker scanning to obliterate potential transcription factor binding sites within the promoter individually or in combination. The effects of these mutations on transcription levels may be determined by inserting the mutations into the cloning sites in the promoter reporter vectors.

### EXAMPLE 60

### Cloning and Identification of Promoters

Using the method described in Example 58 above with 5' ESTs, sequences upstream of several genes were obtained. Using the primer pairs GGG AAG ATG GAG ATA GTA TTG CCT G (SEQ ID NO:29) and CTG CCA TGT ACA TGA TAG AGA GAT TC (SEQ ID NO:30), the promoter having the internal designation PI3H2 (SEQ ID NO:31) was obtained.

Using the primer pairs GTA CCA GGGG ACT GTG ACC ATT GC (SEQ ID NO:32) and CTG TGA CCA TTG CTC CCA AGA GAG (SEQ ID NO:33), the promoter having the internal designation P15B4 (SEQ ID NO:34) was obtained.

Using the primer pairs CTG GGA TGG AAG GCA CGG TA (SEQ ID NO:35) and GAG ACC ACA CAG CTA GAC AA (SEQ ID NO:36), the promoter having the internal designation P29B6 (SEQ ID NO:37) was obtained.

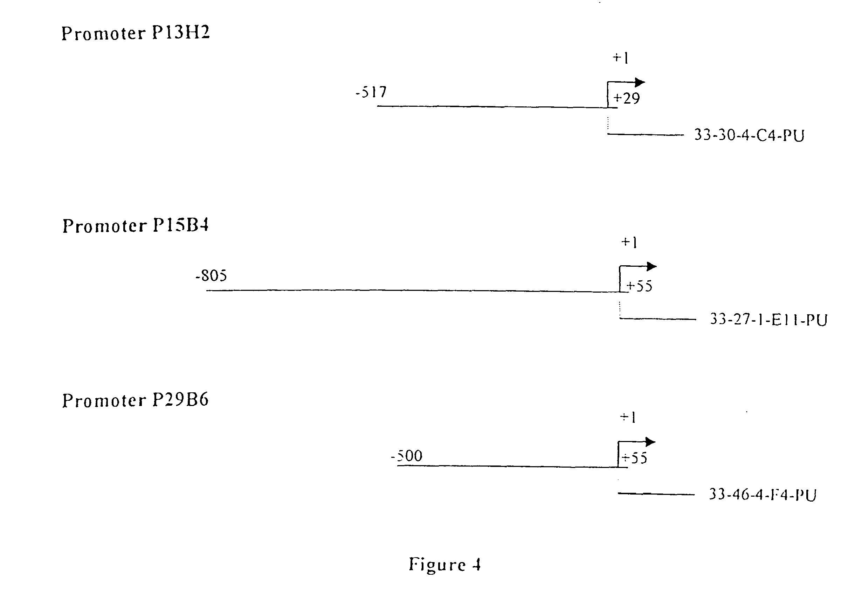

Figure 4 provides a schematic description of the promoters isolated and the way they are assembled with the corresponding 5' tags. The upstream sequences were screened for the presence of motifs resembling transcription factor binding sites or known transcription start sites using the computer program MatInspector release 2.0, August 1996.

Table VII describes the transcription factor binding sites present in each of these promoters. The columns labeled matrice provides the name of the MatInspector matrix used. The column labeled position provides the 5' position of the promoter site. Numeration of the sequence starts from the transcription site as determined by matching the genomic sequence with the 5' EST sequence. The column labeled "orientation" indicates the DNA strand on which the site is found, with the + strand being the coding strand as determined by matching the genomic sequence with the sequence of the 5' EST. The column labeled "score" provides the Matlnspector score found for this site. The column labeled "length" provides the length of the site in nucleotides. The column labeled "sequence" provides the sequence of the site found.

Bacterial clones containing plasmids containing the promoter sequences described above are presently stored in the inventor's laboratories under the internal identification numbers provided above. The inserts may be recovered from the deposited materials by growing an aliquot of the appropriate bacterial clone in the appropriate medium. The plasmid DNA can then be isolated using plasmid isolation procedures familiar to those skilled in the art such as alkaline lysis minipreps or large scale alkaline lysis plasmid isolation procedures. If desired the plasmid DNA may be further enriched by centrifugation on a cesium chloride gradient, size exclusion chromatography, or anion exchange chromatography. The plasmid DNA obtained using these procedures may then be manipulated using standard cloning techniques familiar to those skilled in the art. Alternatively, a PCR can be done with primers designed at both ends of the EST insertion. The PCR product which corresponds to the 5' EST can then be manipulated using standard cloning techniques familiar to those skilled in the art.

The promoters and other regulatory sequences located upstream of the extended cDNAs or 5' ESTs may be used to design expression vectors capable of directing the expression of an inserted gene in a desired spatial, temporal, developmental, or quantitative manner. A promoter capable of directing the desired spatial, temporal, developmental, and quantitative patterns may be selected using the results of the expression analysis described in Example 26 above. For example, if a promoter which confers a high level of expression in muscle is desired, the promoter sequence upstream of an extended cDNA or 5' EST derived from an mRNA which is expressed at a high level in muscle, as determined by the method of Example 26, may be used in the expression vector.

Preferably, the desired promoter is placed near multiple restriction sites to facilitate the cloning of the desired insert downstream of the promoter, such that the promoter is able to drive expression of the inserted gene. The promoter may be inserted in conventional nucleic acid backbones designed for extrachromosomal replication, integration into the host chromosomes or transient expression. Suitable backbones for the present expression vectors include retroviral backbones, backbones from eukaryotic episomes such as SV40 or Bovine Papilloma Virus, backbones from bacterial episomes, or artificial chromosomes.

Preferably, the expression vectors also include a poly A signal downstream of the multiple restriction sites for directing the polyadenylation of mRNA transcribed from the gene inserted into the expression vector.

Following the identification of promoter sequences using the procedures of Examples 58-60, proteins which interact with the promoter may be identified as described in Example 61 below.

### EXAMPLE 61

### Identification of Proteins Which Interact with Promoter Sequences. Upstream Regulatory Sequences, or mRNA

Sequences within the promoter region which are likely to bind transcription factors may be identified by homology to known transcription factor binding sites or through conventional mutagenesis or deletion analyses of reporter plasmids containing the promoter sequence. For example, deletions may be made in a reporter plasmid containing the promoter sequence of interest operably linked to an assayable reporter gene. The reporter plasmids carrying various deletions within the promoter region are transfected into an appropriate host cell and the effects of the deletions on expression levels is assessed. Transcription factor binding sites within the regions in which deletions reduce expression levels may be further localized using site directed mutagenesis, linker scanning analysis, or other techniques familiar to those skilled in the art.

Nucleic acids encoding proteins which interact with sequences in the promoter may be identified using one-hybrid systems such as those described in the manual accompanying the Matchmaker One-Hybrid System kit available from Clontech (Catalog No. K1603-1). Briefly, the Matchmaker One-hybrid system is used as follows. The target sequence for which it is desired to identify binding proteins is cloned upstream of a selectable reporter gene and integrated into the yeast genome. Preferably, multiple copies of the target sequences are inserted into the reporter plasmid in tandem. A library comprised of fusions between cDNAs to be evaluated for the ability to bind to the promoter and the activation domain of a yeast transcription factor, such as GAL4, is transformed into the yeast strain containing the integrated reporter sequence. The yeast are plated on selective media to select cells expressing the selectable marker linked to the promoter sequence. The colonies which grow on the selective media contain genes encoding proteins which bind the target sequence. The inserts in the genes encoding the fusion proteins are further characterized by sequencing. In addition, the inserts may be inserted into expression vectors or *in vitro* transcription vectors. Binding of the polypeptides encoded by the inserts to the promoter DNA may be confirmed by techniques familiar to those skilled in the art, such as gel shift analysis or DNAse protection analysis.

### VII. Use of 5' ESTs (or cDNAs or Genomic DNAs Obtainable Therefrom) in Gene Therapy

5'ESTs (or cDNA or genomic DNA obtainable therefrom) may also be used in gene therapy strategies, including antisense and triple helix strategies as described in Examples 62 and 63 below. In antisense approaches, nucleic acid sequences complementary to an mRNA are hybridized to the mRNA intracellularly, thereby blocking the expression of the protein encoded by the mRNA .The antisense sequences may prevent gene expression through a variety of mechanisms. For example, the antisense sequences may inhibit the ability of ribosomes to translate the mRNA Alternatively, the antisense sequences may block transport of the mRNA from the nucleus to the cytoplasm, thereby limiting the amount of mRNA available for translation. Another mechanism through which antisense sequences may inhibit gene expression is by interfering with mRNA splicing. In yet another strategy, the antisense nucleic acid may be incorporated in a ribozyme capable of specifically cleaving the target mRNA.

### EXAMPLE 62

### Preparation and Use of Antisense Oligonucleotides

The antisense nucleic acid molecules to be used in gene therapy may be either DNA or RNA sequences. They may comprise a sequence complementary to the sequence of the 5'EST (or cDNA or genomic DNA obtainable therefrom). The antisense nucleic acids should have a length and melting temperature sufficient to permit formation of an intracellular duplex with sufficient stability to inhibit the expression of the mRNA in the duplex. Strategies for designing antisense nucleic acids suitable for use in gene therapy are disclosed in Green *et al., Ann. Rev. Biochem.* **55**:569-597, 1986; and Izant and Weintraub, *Cell* **36**:1007-1015, 1984.

In some strategies, antisense molecules are obtained from a nucleotide sequence encoding a protein by reversing the orientation of the coding region with respect to a promoter so as to transcribe the opposite strand from that which is normally transcribed in the cell. The antisense molecules may be transcribed using *in vitro* transcription systems such as those which employ T7 or SP6 polymerase to generate the transcript. Another approach involves transcription of the antisense nucleic acids *in vivo* by operably linking DNA containing the antisense sequence to a promoter in an expression vector.

Alternatively, oligonucleotides which are complementary to the strand normally transcribed in the cell may be synthesized *in vitro.* Thus, the antisense nucleic acids are complementary to the corresponding mRNA and are capable of hybridizing to the mRNA to create a duplex. In some embodiments, the antisense sequences may contain modified sugar phosphate backbones to increase stability and make them less sensitive to RNase activity. Examples of modifications suitable for use in antisense strategies are described by Rossi *et al., Pharmacol. Ther.* **50(2)**:245-254, 1991.

Various types of antisense oligonucleotides complementary to the sequence of the 5'EST (or cDNA or genomic DNA obtainable therefrom) may be used. In one preferred embodiment, stable and semi-stable antisense oligonucleotides described in International Application No. PCT WO94/23026 are used. In these molecules, the 3' end or both the 3' and 5' ends are engaged in intramolecular hydrogen bonding between complementary base pairs. These molecules are better able to withstand exonuclease attacks and exhibit increased stability compared to conventional antisense oligonucleotides.

In another preferred embodiment, the antisense oligodeoxynucleotides against herpes simplex virus types 1 and 2 described in International Application No. WO 95/04141 are used.

In yet another preferred embodiment, the covalently cross-linked antisense oligonucleotides described in International Application No. WO 96/31523 are used. These double- or single-stranded oligonucleotides comprise one or more, respectively, inter- or intra-oligonucleotide covalent cross-linkages, wherein the linkage consists of an amide bond between a primary amine group of one strand and a carboxyl group of the other strand or of the same strand, respectively, the primary amine group being directly substituted in the 2' position of the strand nucleotide monosaccharide ring, and the carboxyl group being carried by an aliphatic spacer group substituted on a nucleotide or nucleotide analog of the other strand or the same strand, respectively.

The antisense oligodeoxynucleotides and oligonucleotides disclosed in International Application No. WO 92/18522 may also be used. These molecules are stable to degradation and contain at least one transcription control recognition sequence which binds to control proteins and are effective as decoys therefore. These molecules may contain "hairpin" structures, "dumbbell" structures, "modified dumbbell" structures, "cross-linked" decoy structures and "loop" structures.

In another preferred embodiment, the cyclic double-stranded oligonucleotides described in European Patent Application No. 0 572 287 A2 are used. These ligated oligonucleotide "dumbbells" contain the binding site for a transcription factor and inhibit expression of the gene under control of the transcription factor by sequestering the factor.

Use of the closed antisense oligonucleotides disclosed in International Application No. WO 92/19732 is also contemplated. Because these molecules have no free ends, they are more resistant to degradation by exonucleases than are conventional oligonucleotides. These oligonucleotides may be multifunctional, interacting with several regions which are not adjacent to the target mRNA.

The appropriate level of antisense nucleic acids required to inhibit gene expression may be determined using *in vitro* expression analysis. The antisense molecule may be introduced into the cells by diffusion, injection, infection, transfection or h-region-mediated import using procedures known in the art. For example, the antisense nucleic acids can be introduced into the body as a bare or naked oligonucleotide, oligonucleotide encapsulated in lipid, oligonucleotide sequence encapsidated by viral protein, or as an oligonucleotide operably linked to a promoter contained in an expression vector. The expression vector may be any of a variety of expression vectors known in the art, including retroviral or viral vectors, vectors capable of extrachromosomal replication, or integrating vectors. The vectors may be DNA or RNA.

The antisense molecules are introduced onto cell samples at a number of different concentrations preferably between 1x10⁻¹⁰M to 1x10⁻⁴M. Once the minimum concentration that can adequately control gene expression is identified, the optimized dose is translated into a dosage suitable for use *in* vivo. For example, an inhibiting concentration in culture of 1x10⁻⁷ translates into a dose of approximately 0.6 mg/kg bodyweight. Levels of oligonucleotide approaching 100 mg/kg bodyweight or higher may be possible after testing the toxicity of the oligonucleotide in laboratory animals. It is additionally contemplated that cells from the vertebrate are removed, treated with the antisense oligonucleotide, and reintroduced into the vertebrate.

It is further contemplated that the antisense oligonucleotide sequence is incorporated into a ribozyme sequence to enable the antisense to specifically bind and cleave its target mRNA. For technical applications of ribozyme and antisense oligonucleotides see Rossi *et al., supra.*

In a preferred application of this invention, the polypeptide encoded by the gene is first identified, so that the effectiveness of antisense inhibition on translation can be monitored using techniques that include but are not limited to antibody-mediated tests such as RIAs and ELISA, functional assays, or radiolabeling.

The 5' ESTs of the present invention (or cDNAs or genomic DNAs obtainable therefrom) may also be used in gene therapy approaches based on intracellular triple helix formation. Triple helix oligonucleotides are used to inhibit transcription from a genome. They are particularly useful for studying alterations in cell activity as it is associated with a particular gene. The 5' EST sequences (or cDNAs or genomic DNAs obtainable therefrom) of the present invention or, more preferably, a portion of those sequences, can be used to inhibit gene expression in individuals having diseases associated with expression of a particular gene. Similarly, a portion of 5' EST sequences (or cDNAs or genomic DNAs obtainable therefrom) can be used to study the effect of inhibiting transcription of a particular gene within a cell. Traditionally, homopurine sequences were considered the most useful for triple helix strategies. However, homopyrimidine sequences can also inhibit gene expression. Such homopyrimidine oligonucleotides bind to the major groove at homopurine:homopyrimidine sequences. Thus, both types of sequences from the 5'EST or from the gene corresponding to the 5'EST are contemplated within the scope of this invention.

### EXAMPLE 63

### Preparation and Use of Triple Helix Probes

The sequences of the 5' ESTs (or cDNAs or genomic DNAs obtainable therefrom) are scanned to identify 10-mer to 20-mer homopyrimidine or homopurine stretches which could be used in triple-helix based strategies for inhibiting gene expression. Following identification of candidate homopyrimidine or homopurine stretches, their efficiency in inhibiting gene expression is assessed by introducing varying amounts of oligonucleotides containing the candidate sequences into tissue culture cells which normally express the target gene. The oligonucleotides may be prepared on an oligonucleotide synthesizer or they may be purchased commercially from a company specializing in custom oligonucleotide synthesis, such as GENSET, Paris, France.

The oligonucleotides may be introduced into the cells using a variety of methods known to those skilled in the art, including but not limited to calcium phosphate precipitation, DEAE-Dextran, electroporation, liposome-mediated transfection or native uptake.

Treated cells are monitored for altered cell function or reduced gene expression using techniques such as Northern blotting, RNase protection assays, or PCR based strategies to monitor the transcription levels of the target gene in cells which have been treated with the oligonucleotide. The cell functions to be monitored are predicted based upon the homologies of the target gene corresponding to the extended cDNA from which the oligonucleotide was derived with known gene sequences that have been associated with a particular function. The cell functions can also be predicted based on the presence of abnormal physiologies within cells derived from individuals with a particular inherited disease, particularly when the extended cDNA is associated with the disease using techniques described in Example 56.

The oligonucleotides which are effective in inhibiting gene expression in tissue culture cells may then be introduced *in vivo* using the techniques described above and in Example 62 at a dosage calculated based on the *in vitro* results, as described in Example 62.

In some embodiments, the natural (beta) anomers of the oligonucleotide units can be replaced with alpha anomers to render the oligonucleotide more resistant to nucleases. Further, an intercalating agent such as ethidium bromide, or the like, can be attached to the 3' end of the alpha oligonucleotide to stabilize the triple helix. For information on the generation of oligonucleotides suitable for triple helix formation see Griffin *et al., Science* **245**:967-971, 1989.

### EXAMPLE 64

### Use of cDNAs Obtained Using the 5' ESTs to Express an Encoded Protein in a Host Organism

The cDNAs obtained as described above using the 5' ESTs of the present invention may also be used to express an encoded protein in a host organism to produce a beneficial effect. In such procedures, the encoded protein may be transiently expressed in the host organism or stably expressed in the host organism. The encoded protein may have any of the activities described above. The encoded protein may be a protein which the host organism lacks or, alternatively, the encoded protein may augment the existing levels of the protein in the host organism.

A full length extended cDNA encoding the signal peptide and the mature protein, or an extended cDNA encoding only the mature protein is introduced into the host organism The extended cDNA may be introduced into the host organism using a variety of techniques known to those of skill in the art. For example, the extended cDNA may be injected into the host organism as naked DNA such that the encoded protein is expressed in the host organism, thereby producing a beneficial effect.

Alternatively, the extended cDNA may be cloned into an expression vector downstream of a promoter which is active in the host organism. The expression vector may be any of the expression vectors designed for use in gene therapy, including viral or retroviral vectors. The expression vector may be directly introduced into the host organism such that the encoded protein is expressed in the host organism to produce a beneficial effect. In another approach, the expression vector may be introduced into cells *in vitro.* Cells containing the expression vector are thereafter selected and introduced into the host organism, where they express the encoded protein to produce a beneficial effect.

### EXAMPLE 65

### Use of Signal Peptides Encoded by 5' ESTs or Sequences obtained Therefrom to Import Proteins Into Cells

The short core hydrophobic region (h) of signal peptides encoded by the 5'ESTS or extended cDNAs derived from SEQ ID NOs: 38-291 may also be used as a carrier to import a peptide or a protein of interest, so-called cargo, into tissue culture cells (Lin *et al., J. Biol. Chem.,* **270:** 14225-14258, 1995; *Du et al., J. Peptide Res.,* **51:** 235-243, 1998; Rojas *et al., Nature Biotech.,* **16:** 370-375, 1998).

When cell permeable peptides of limited size (approximately up to 25 amino acids) are to be translocated across cell membrane, chemical synthesis may be used in order to add the h region to either the C-terminus or the N-terminus to the cargo peptide of interest. Alternatively, when longer peptides or proteins are to be imported into cells, nucleic acids can be genetically engineered, using techniques familiar to those skilled in the art, in order to link the extended cDNA sequence encoding the h region to the 5' or the 3' end of a DNA sequence coding for a cargo polypeptide. Such genetically engineered nucleic acids are then translated either *in vitro* or *in vivo* after transfection into appropriate cells, using conventional techniques to produce the resulting cell permeable polypeptide. Suitable hosts cells are then simply incubated with the cell permeable polypeptide which is then translocated across the membrane.

This method may be applied to study diverse intracellular functions and cellular processes. For instance, it has been used to probe functionally relevant domains of intracellular proteins and to examine protein-protein interactions involved in signal transduction pathways (Lin *et al., supra;* Lin *et al., J. Biol. Chem.,* **271:** 5305-5308, 1996; Rojas *et al., J. Biol. Chem.,* **271:** 27456-27461, 1996; Liu *et al., Proc. Natl. Acad* Sci. *USA,* **93:** 11819-11824, 1996; *Rojas et al., Bioch. Biophys. Res. Commun.,* **234:** 675-680, 1997).

Such techniques may be used in cellular therapy to import proteins producing therapeutic effects. For instance, cells isolated from a patient may be treated with imported therapeutic proteins and then re-introduced into the host organism.

Alternatively, the h region of signal peptides of the present invention could be used in combination with a nuclear localization signal to deliver nucleic acids into cell nucleus. Such oligonucleotides may be antisense oligonucleotides or oligonucleotides designed to form triple helixes, as described in examples 62 and 63 respectively, in order to inhibit processing and/or maturation of a target cellular RNA.

As discussed above, cDNAs or portions thereof obtained using the 5' ESTs of the present invention can be used for various purposes. The polynucleotides can be used to express recombinant protein for analysis, characterization or therapeutic use; as markers for tissues in which the corresponding protein is preferentially expressed (either constitutively or at a particular stage of tissue differentiation or development or in disease states); as molecular weight markers on Southern gels; as chromosome markers or tags (when labeled) to identify chromosomes or to map related gene positions; to compare with endogenous DNA sequences in patients to identify potential genetic disorders; as probes to hybridize and thus discover novel, related DNA sequences; as a source of information to derive PCR primers for genetic fingerprinting; for selecting and making oligomers for attachment to a "gene chip" or other support, including for examination for expression patterns; to raise anti-protein antibodies using DNA immunization techniques; and as an antigen to raise anti-DNA antibodies or elicit another immune response. Where the polynucleotide encodes a protein which binds or potentially binds to another protein (such as, for example, in a receptor-ligand interaction), the polynucleotide can also be used in interaction trap assays (such as, for example, that described in Gyuris *et al*., *Cell* 75:791-803, 1993) to identify polynucleotides encoding the other protein with which binding occurs or to identify inhibitors of the binding interaction.

The proteins or polypeptides provided by the present invention can similarly be used in assays to determine biological activity, including in a panel of multiple proteins for high-throughput screening; to raise antibodies or to elicit another immune response; as a reagent (including the labeled reagent) in assays designed to quantitatively determine levels of the protein (or its receptor) in biological fluids; as markers for tissues in which the corresponding protein is preferentially expressed (either constitutively or at a particular stage of tissue differentiation or development or in a disease state); and, of course, to isolate correlative receptors or ligands. Where the protein binds or potentially binds to another protein (such as, for example, in a receptor-ligand interaction), the protein can be used to identify the other protein with which binding occurs or to identify inhibitors of the binding interaction. Proteins involved in these binding interactions can also be used to screen for peptide or small molecule inhibitors or agonists of the binding interaction.

Any or all of these research utilities are capable of being developed into reagent grade or kit format for commercialization as research products.

Methods for performing the uses listed above are well known to those skilled in the art. References disclosing such methods include without limitation *Molecular Cloning; A Laboratory Manual.* 2d ed., Cold Spring Harbor Laboratory Press, Sambrook,. Fritsch and Maniatis eds., 1989, and *Methods in Enzymology; Guide to Molecular Cloning Techniques,* Academic Press, Berger and Kimmel eds., 1987.

Polynucleotides and proteins of the present invention can also be used as nutritional sources or supplements. Such uses include without limitation use as a protein or amino acid supplement, use as a carbon source, use as a nitrogen source and use as a source of carbohydrate. In such cases the protein or polynucleotide of the invention can be added to the feed of a particular organism or can be administered as a separate solid or liquid preparation, such as in the form of powder, pills, solutions, suspensions or capsules. In the case of microorganisms, the protein or polynucleotide of the invention can be added to the medium in or on which the microorganism is cultured.

**Table 1: Parameters used for each step of EST analysis**

| | **Search characteristic** | | **Selection Characteristics** | | |
|---|---|---|---|---|---|
| **Step** | **Program** | **Strand** | **Parameters** | **Identity (%)** | **Length (bp)** |
| miscellanaeous | blastn | both | S=61 X=16 | 90 | 17 |
| tRNA | fasta | both | - | 80 | 60 |
| rRNA | blastn | both | S=108 | 80 | 40 |
| mtRNA | blastn | both | S=108 | 80 | 40 |
| Procaryotic | blastn | both | S=144 | 90 | 40 |
| Fungal | blastn | both | S=144 | 90 | 40 |
| Alu | fasta* | both | - | 70 | 40 |
| L1 | blastn | both | S=72 | 70 | 40 |
| Repeats | blastn | both | S=72 | 70 | 40 |
| Promoters | blastn | top | S=54 X=16 | 90 | 15† |
| Vertebrate | fasta* | both | S=108 | 90 | 30 |
| ESTs | blastn | both | S=108 X=16 | 90 | 30 |
| Proteins | blastx^{¤} | top | E = 0.001 | - | - |

| | | | | | |
|---|---|---|---|---|---|
| * use "Quick Fast" Database scanner | | | | | |
| † alignement further constrained to begin closer than 10bp to EST\5' end | | | | | |
| ^{¤} using BLOSUM62 substitution matrix | | | | | |

**TABLE II**

| SEQ. ID NO. | CATEGORY | VON HEIJNE SCORE | TISSUE SOURCE | INTERNAL DESIGNATION |
|---|---|---|---|---|
| ID38 | new | 15 | Liver | 22-6-1-A10-PU |
| | | | Fetal liver | |
| ID39 | new | 13.2 | Ovary | 77-16-3-B7-PU |
| | | | Hypertrophic prostate | |
| | | | Brain | |
| ID40 | new | 13.1 | Fetal brain | 47-47-1-F2-PU |
| | | | Substantia nigra | |
| ID41 | new | 11.6 | Fetal kidney | 58-12-2-E11-PU |
| | | | Cancerous prostate | |
| ID42 | new | 10.7 | Liver | 21-4-2-D1-PU |
| | | | Kidney | |
| ID43 | new | 9.6 | Hypertrophic prostate | 77-38-4-B2-PU |
| | | | Cancerous prostate | |
| | | | Large intestine | |
| ID44 | new | 9.4 | Fetal kidney | 76-10-2-B7-PU |
| | | | Cancerous prostate | |
| ID45 | new | 9.4 | Prostate | 33-99-2-G8-PU |
| | | | Brain | |
| ID46 | new | 9.1 | Hypertrophic prostate | 78-32-2-C2-PU |
| | | | Normal prostate | |
| | | | Brain | |
| ID47 | new | 9.1 | Ovary | 26-40-3-D6-PU |
| | | | Brain | |
| ID48 | new | 8 | Fetal kidney | 33-106-2-F10-PU |
| | | | Brain | |
| ID49 | new | 7.8 | Fetal kidney | 58-38-1-A2-PU |
| | | | Lung (cells) | |
| ID50 | new | 7.4 | Lymph ganglia | 62-10-3-A11-PU |
| | | | Surrenals | |
| ID51 | new | 7.4 | Hypertrophic prostate | 76-45-1-F5-PU |
| | | | Cancerous prostate | |
| ID52 | new | 7.1 | Fetal kidney | 37-10-3-D7-PU |
| | | | Lung (cells) | |
| | | | Umbilical cord | |
| | | | Hypertrophic prostate | |
| | | | Cancerous prostate | |
| | | | Substantia nigra | |
| ID53 | new | 6.9 | Hypertrophic prostate | 78-16-2-B12-PU |
| | | | Normal prostate | |
| | | | Lymph ganglia | |
| | | | Spleen | |
| ID54 | new | 6.8 | Fetal brain | 33-38-2-A4-PU |
| | | | Brain | |
| ID55 | new | 6.7 | Heart | 47-25-4-A2-PU |
| | | | Spleen | |
| | | | Substantia nigra | |
| ID56 | new | 6.3 | Fetal brain | 20-10-3-D9-PU |
| | | | Spleen | |
| ID57 | new | 6.3 | Hypertrophic prostate | 84-5-1-C9-PU |
| | | | Thyroid | |
| ID58 | new | 6.3 | Prostate | 76-40-1-A8-PU |
| | | | Hypertrophic prostate | |
| | | | Normal prostate | |
| | | | Cancerous prostate | |
| ID59 | new | 6.3 | Fetal kidney | 76-5-1-F4-PU |
| | | | Normal prostate | |
| | | | Hypertrophic prostate | |
| | | | Cancerous prostate | |
| ID60 | new | 6.3 | Fetal kidney | 77-25-3-H5-PU |
| | | | Hypertrophic prostate | |
| | | | Kidney | |
| ID61 | new | 5.7 | Prostate | 42-1-4-H1-PU |
| | | | Lymph ganglia | |
| | | | Lung | |
| ID62 | new | 5.6 | Brain | 33-804-E4-PU |
| | | | Lymph ganglia | |
| | | | Pancreas | |
| ID63 | new | 5.6 | Fetal kidney | 58-47-2-E11-PU |
| | | | Normal prostate | |
| ID64 | new | 5.6 | Muscle | 33-56-4-F4-PU |
| | | | Brain | |
| ID65 | new | 5.5 | Placenta | 23-1-4-F6-PU |
| | | | Lung (cells) | |
| | | | Colon | |
| | | | Cancerous prostate | |
| ID66 | new | 5.3 | Normal prostate | 76-44-2-F7-PU |
| | | | Cancerous prostate | |
| ID67 | new | 5.2 | Hypertrophic prostate | 76-19-1-E9-PU |
| | | | Cancerous prostate | |
| ID68 | new | 5.1 | Colon | 78-31-1-D12-PU |
| | | | Normal prostate | |
| | | | Kidney | |
| ID69 | new | 4.9 | Prostate | 20-1-4-H6-PU |
| | | | Spleen | |
| ID70 | new | 4.9 | Lymphocytes | 2.1-3-4-C4-PU |
| | | | Cancerous prostate | |
| ID71 | new | 4.7 | Kidney | 33-102-2-C9-PU |
| | | | Brain | |
| ID72 | new | 4.7 | Colon | 48-47-3-A5-PU |
| | | | Lymph ganglia | |
| ID73 | new | 4.6 | Placenta | 77-2-3-D1-PU |
| | | | Hypertrophic prostate | |
| ID74 | new | 4.6 | Normal prostate | 76-3-3-C7-PU |
| | | | Thyroid | |
| | | | Cancerous prostate | |
| | | | Substantia nigra | |
| ID75 | new | 4.5 | Fetal kidney | 83-1-3-H6-PU |
| | | | Large intestine | |
| ID76 | new | 4.4 | Fetal brain | 33-7-2-D11-PU |
| | | | Brain | |
| ID77 | new | 4 | Normal prostate | 78-28-2-G12-PU |
| | | | Substantia nigra | |
| ID78 | new | 3.9 | Normal prostate | 76-23-3-D8-PU |
| | | | Cancerous prostate | |
| ID79 | new | 3.9 | Heart | 48-3-3-H9-PU |
| | | | Lymph ganglia | |
| ID80 | new | 3.8 | Brain | 42-2-4-B8-PU |
| | | | Lung | |
| ID81 | new | 3.8 | Normal prostate | 77-37-2-H1-PU |
| | | | Hypertrophic prostate | |
| ID82 | new | 3.8 | Lung (cells) | 51-37-4-B1-PU |
| | | | Testis | |
| | | | Lung | |
| ID83 | new | 3.7 | Ovary | 23-9-4-G9-PU |
| | | | Lung (cells) | |
| | | | Colon | |
| | | | Normal prostate | |
| ID84 | new | 3.5 | Ovary | 27-3-2-B6-PU |
| | | | Muscle | |
| | | | Hypertrophic prostate | |
| ID85 | new | 3.5 | Normal prostate | 76-30-3-B7-PU |
| | | | Hypertrophic prostate | |
| | | | Cancerous prostate | |
| ID86 | ext-est-not-vrt | 13.4 | Ovary | 76-9-4-G9-PU |
| | | | Prostate | |
| | | | Cancerous prostate | |
| ID87 | ext-est-not-vrt | 12.6 | Normal prostate | 78-25-4-HI-PU |
| | | | Hypertrophic prostate | |
| ID88 | ext-est-not-vrt | 11.8 | Fetal kidney | 77-1-4-D10-PU |
| | | | Hypertrophic prostate | |
| ID89 | ext-est-not-vrt | 11.2 | Lung (cells) | 78-37-1-A12-PU |
| | | | Normal prostate | |
| | | | Cancerous prostate | |
| ID90 | ext-est-not-vrt | 10.3 | Umbilical cord | 37-10-2-C10-PU |
| | | | Hypertrophic prostate | |
| ID91 | ext-est-not-vrt | 10.1 | Brain | 76-16-1-H5-PU |
| | | | Cancerous prostate | |
| ID92 | ext-est-not-vrt | 9.8 | Lymphocytes | 24-1-4-G11-PU |
| | | | Lung (cells) | |
| | | | Umbilical cord | |
| | | | Normal prostate | |
| ID93 | ext-est-not-vrt | 9.3 | Thyroid | 48-51-2-C10-PU |
| | | | Heart | |
| | | | Lymph ganglia | |
| ID94 | ext-est-not-vrt | 8.4 | Lung Lung | 33-97-4-G8-PU |
| ID95 | ext-est-not-vrt | 7.8 | Fetal brain | 33-22-1-F9-PU |
| | | | Brain | |
| ID96 | ext-est-not-vrt | 7.4 | Ovary | 37-7-4-E7-PU |
| | | | Liver | |
| | | | Umbilical cord | |
| | | | Kidney | |
| | | | Surrenals | |
| ID97 | ext-est-not-vrt | 7.2 | Muscle | 27-12-3-HS-PU |
| | | | Liver | |
| | | | Dystrophic muscle | |
| | | | Normal prostate | |
| | | | Testis | |
| | | | Cancerous prostate | |
| | | | Lymph ganglia | |
| | | | Large intestine | |
| ID98 | ext-est-not-vrt | 7.1 | Fetal kidney | 58-23-4-G9-PU |
| | | | Ovary | |
| ID99 | ext-est-not-vrt | 6.9 | Placenta | 58-34-2-H8-PU |
| | | | Fetal kidney | |
| ID100 | ext-est-not-vrt | 6.7 | Fetal kidney | 37-9-1-D4-PU |
| | | | Fetal brain | |
| | | | Umbilical cord | |
| | | | Heart | |
| | | | Fetal liver | |
| ID101 | ext-est-not-vrt | 6.6 | Fetal kidney | 58-5-3-A8-PU |
| | | | Liver | |
| | | | Thyroid | |
| | | | Kidney | |
| | | | Cancerous prostate | |
| | | | Lung (cells) | |
| | | | Normal prostate | |
| | | | Lymph ganglia | |
| ID102 | ext-est-not-vrt | 6.6 | Cancerous prostate | 76-35-1-A11-PU |
| | | | Normal prostate | |
| ID103 | ext-est-not-vrt | 5.4 | Hypertrophic prostate | 77-35-2-E10-PU |
| | | | Lung (cells) | |
| ID104 | ext-est-not-vrt | 5.4 | Fetal kidney | 58-52-4-D8-PU |
| | | | Fetal brain | |
| | | | Normal prostate | |
| ID105 | ext-est-not-vrt | 5.3 | Cancerous prostate | 47-26-3-D2-PU |
| | | | Substantia nigra | |
| ID106 | ext-est-not-vrt | 5.1 | Cancerous prostate | 30-9-1-G8-PU |
| | | | Fetal brain | |
| | | | Lung (cells) | |
| | | | Brain | |
| ID107 | ext-est-not-vrt | 4.9 | Lung | 33-98-1-C6-PU |
| | | | Brain | |
| ID108 | ext-est-not-vrt | 4.5 | Ovary | 78-26-1-B12-PU |
| | | | Prostate | |
| | | | Normal prostate | |
| | | | Brain | |
| ID109 | ext-est-not-vrt | 4.2 | Fetal kidney | 58-7-2-F8-PU |
| | | | Cancerous prostate | |
| | | | Normal prostate | |
| ID110 | ext-est-not-vrt | 3.7 | Fetal kidney | 58-33-1-F9-PU |
| | | | Ovary | |
| | | | Prostate | |
| | | | Normal prostate | |
| ID111 | ext-est-not-vrt | 3.6 | Brain | 33-19-1-FI-PU |
| | | | Lymph ganglia | |
| ID112 | ext-est-not-vrt | 3.5 | Fetal kidney | 58-14-2-D3-PU |
| | | | Liver | |
| | | | Kidney | |
| | | | Brain | |
| ID113 | ext-est-not-vrt | 3.5 | Ovary | 26-40-2-B2-PU |
| | | | Hypertrophic prostate | |
| ID114 | est-not-ext | 13.9 | Fetal kidney | 58-52-4-F10-PU |
| | | | Cancerous prostate | |
| | | | Normal prostate | |
| ID115 | est-not-ext | 13.9 | Fetal kidney | 58-15-1-H6-PU |
| | | | Lung (cells) | |
| ID116 | est-not-ext | 11.6 | Ovary | 51-29-2-B2-PU |
| | | | Dystrophic muscle | |
| | | | Cancerous prostate | |
| | | | Uterus | |
| | | | Testis | |
| | | | Lymph ganglia | |
| | | | Surrenals | |
| ID117 | est-not-ext | 11.6 | Lymph ganglia | 48-7-1-F2-PU |
| | | | Large intestine | |
| ID118 | est-not-ext | 11.6 | Umbilical cord | 37-6-1-E12-PU |
| | | | Pancreas | |
| ID119 | est-not-ext | 11.4 | Heart | 67-3-4-G7-PU |
| | | | Brain | |
| ID120 | est-not-ext | 11.2 | Dystrophic muscle | 33-35-4-F4-PU |
| | | | Brain | |
| ID121 | est-not-ext | 11 | Ovary | 48-14-1-All-PU |
| | | | Heart | |
| | | | Kidney. | |
| | | | Cancerous prostate | |
| | | | Lymph ganglia | |
| LD122 | est-not-ext | 10.5 | Lung | 37-11-1-G2-PU |
| | | | Umbilical cord | |
| | | | Normal prostate | |
| ID123 | est-not-ext | 10 | Fetal kidney | 58-3-4-G2-PU |
| | | | Cancerous prostate | |
| | | | Normal prostate | |
| | | | Brain | |
| ID124 | est-not-ext | 9.5 | Fetal kidney | 76-18-1-F6-PU |
| | | | Cancerous prostate | |
| | | | Umbilical cord | |
| | | | Normal prostate | |
| ID125 | est-not-ext | 9.5 | Placenta | 47-24-2-C1-PU |
| | | | Muscle | |
| | | | Substantia nigra | |
| ID126 | est-not-ext | 9.3 | Ovary | 37-11-4-H11-PU |
| | | | Cancerous prostate | |
| | | | Umbilical cord | |
| | | | Colon | |
| | | | Normal prostate | |
| | | | Testis | |
| ID127 | est-not-ext | 9.3 | Cancerous prostate | 47-37-2-E3-PU |
| | | | Normal prostate | |
| | | | Substantia nigra | |
| ID128 | est-not-ext | 9.3 | Spleen | 27-16-1-E4-PU |
| | | | Muscle | |
| ID129 | est-not-ext | 9.3 | Colon | 47-5-1-G3-PU |
| | | | Substantia nigra | |
| ID130 | est-not-ext | 9.2 | Ovary | 57-2-4-E11-PU |
| | | | Hypertrophic prostate | |
| | | | Fetal brain | |
| ID131 | est-not-ext | 9 | Cancerous prostate | 76-32-1-G12-PU |
| | | | Normal prostate | |
| ID132 | est-not-ext | 8.9 | Fetal kidney | 77-25-1-C6-PU |
| | | | Hypertrophic prostate | |
| | | | Placenta | |
| | | | Normal prostate | |
| | | | Brain | |
| ID133 | est-not-ext | 8.8 | Dystrophic muscle | 37-7-2-B11-PU |
| | | | Umbilical cord | |
| | | | Brain | |
| ID134 | est-not-ext | 8.8 | Fetal kidney | 77-7-3-C8-PU |
| | | | Dystrophic muscle | |
| | | | Hypertrophic prostate | |
| | | | Thyroid | |
| | | | Cancerous prostate | |
| | | | Fetal brain | |
| | | | Muscle | |
| | | | Lung (cells) | |
| | | | Normal prostate | |
| | | | Brain | |
| | | | Lymph ganglia | |
| | | | Large intestine | |
| ID135 | est-not-ext | 8.7 | Fetal kidney | 48-7-3-G5-PU |
| | | | Prostate | |
| | | | Hypertrophic prostate | |
| | | | Spleen | |
| | | | Lung (cells) | |
| | | | Umbilical cord | |
| | | | Testis | |
| | | | Brain | |
| | | | Lymph ganglia | |
| ID136 | est-not-ext | 8.6 | Fetal kidney | 78-17-2-E5-PU |
| | | | Normal prostate | |
| ID137 | est-not-ext | 8.6 | Placenta | 33-10-4-E2-PU |
| | | | Brain | |
| ID138 | est-not-ext | 8.5 | Umbilical cord | 37-1 1-1-C7-PU |
| | | | Normal prostate | |
| ID139 | est-not-ext | 8.5 | Fetal kidney | 26-48-1-H10-PU |
| | | | Lymphocytes | |
| | | | Ovary | |
| | | | Hypertrophic prostate | |
| ID140 | est-not-ext | 8.3 | Prostate | 60-13-3-F6-PU |
| | | | Cancerous prostate | |
| | | | Spleen | |
| | | | Normal prostate | |
| | | | Brain | |
| | | | Lymph ganglia | |
| | | | Large intestine | |
| ID141 | est-not-ext | 8.3 | Cancerous prostate | 78-22-4-A12-PU |
| | | | Normal prostate | |
| ID142 | est-not-ext | 8.1 | Fetal kidney | 57-28-4-B11-PU |
| | | | Ovary | |
| | | | Dystrophic muscle | |
| | | | Hypertrophic prostate | |
| | | | Cancerous prostate | |
| | | | Lung | |
| | | | Spleen | |
| | | | Placenta | |
| | | | Fetal brain | |
| | | | Normal prostate | |
| | | | Colon | |
| | | | Brain | |
| | | | Substantia nigra | |
| ID143 | est-not-ext | 8 | Cancerous prostate | 33-106-3-D8-PU |
| | | | Uterus | |
| | | | Lung (cells) | |
| | | | Colon | |
| | | | Brain | |
| | | | Substantia nigra | |
| ID144 | est-not-ext | 7.9 | Normal prostate | 23-8-3-F5-PU |
| | | | Colon | |
| ID145 | est-not-ext | 7.8 | Placenta | 17-1-3-H5 |
| | | | Brain | |
| ID146 | est-not-ext | 7.6 | Lung | 33-37-2-G9-PU |
| | | | Normal prostate | |
| | | | Brain | |
| | | | Substantia nigra | |
| ID147 | est-not-ext | 7.6 | Brain | 51-16-4-H4-PU |
| | | | Testis | |
| ID148 | est-not-ext | 7.6 | Hypertrophic prostate | 33-32-3-G1-PU |
| | | | Cancerous prostate | |
| | | | Fetal brain | |
| | | | Muscle | |
| | | | Brain | |
| | | | Lymph ganglia | |
| | | | Large intestine | |
| | | | Surrenals | |
| ID149 | est-not-ext | 7.6 | Fetal kidney | 47-10-4-F3-PU |
| | | | Hypertrophic prostate | |
| | | | Cancerous prostate | |
| | | | Lung (cells) | |
| | | | Umbilical cord | |
| | | | Normal prostate | |
| | | | Brain | |
| | | | Surrenals | |
| | | | Substantia nigra | |
| ID150 | est-not-ext | 7.4 | Heart | 51-1-3-G10-PU |
| | | | Cancerous prostate | |
| | | | Testis | |
| ID151 | est-not-ext | 7.4 | Umbilical cord | 33-39-4-B2-PU |
| | | | Brain | |
| | | | Lymph ganglia | |
| ID152 | est-not-ext | 7.4 | Normal prostate | 47-14-3-A3-PU |
| | | | Brain | |
| | | | Substantia nigra | |
| ID153 | est-not-ext | 7.4 | Liver | 48-53-3-H11-PU |
| | | | Lymph ganglia | |
| ID154 | est-not-ext | 7.4 | Cerebellum | 33-63-1-C3-PU |
| | | | Dystrophic muscle | |
| | | | Hypertrophic prostate | |
| | | | Heart | |
| | | | Uterus | |
| | | | Umbilical cord | |
| | | | Brain | |
| ID155 | est-not-ext | 7.3 | Fetal kidney | 53-3-4-F11-PU |
| | | | Ovary | |
| | | | Hypertrophic prostate | |
| | | | Spleen | |
| | | | Lung (cells) | |
| | | | Umbilical cord | |
| | | | Normal prostate | |
| | | | Brain | |
| | | | Substantia nigra | |
| ID136 | est-not-ext | 7.2 | Fetal kidney | 48-5-4-E8-PU |
| | | | Fetal brain | |
| | | | Uterus | |
| | | | Muscle | |
| | | | Umbilical cord | |
| | | | Lung (cells) | |
| | | | Colon | |
| | | | Normal prostate | |
| | | | Brain | |
| | | | Lymph ganglia | |
| | | | Fetal liver | |
| | | | Substantia nigra | |
| | | | Surrenals | |
| ID157 | est-not-ext | 7.1 | Cancerous prostate | 48-54-3-D2-PU |
| | | | Lymph ganglia | |
| | | | Large intestine | |
| | | | Surrenals | |
| ID158 | est-not-ext | 7.1 | Prostate | 78-18-3-C8-PU |
| | | | Hypertrophic prostate | |
| | | | Cancerous prostate | |
| | | | Normal prostate | |
| ID159 | est-not-ext | 7.1 | Normal prostate | 51-4-2-E10-PU |
| | | | Testis | |
| ID160 | est-not-ext | 7 | Fetal kidney | 24-11-1-E4-PU |
| | | | Lymphocytes | |
| | | | Umbilical cord | |
| ID161 | est-not-ext | 7 | Cancerous prostate | 76-1-2-B8-PU |
| | | | Brain | |
| ID162 | est-not-ext | 6.7 | Ovary | 51-11-3-G9-PU |
| | | | Thyroid | |
| | | | Cancerous prostate | |
| | | | Uterus | |
| | | | Muscle | |
| | | | Normal prostate | |
| | | | Testis | |
| | | | Lymph ganglia | |
| ID163 | est-not-ext | 6.7 | Hypertrophic prostate | 77-16-4-G3-PU |
| | | | Lung | |
| | | | Brain | |
| | | | Surrenals | |
| ID164 | est-not-ext | 6.6 | Fetal kidney | 77-38-2-D5-PU |
| | | | Hypertrophic prostate | |
| ID165 | est-not-ext | 6.6 | Fetal kidney | 58-3-3-C8-PU |
| | | | Cancerous prostate | |
| | | | Brain | |
| ID166 | est-not-ext | 6.5 | Brain | 51-1-4-Cl-PU |
| | | | Testis | |
| ID167 | est-not-ext | 6.5 | Fetal kidney | 58-9-2-A6-PU |
| | | | Brain | |
| | | | Lymph ganglia | |
| ID168 | est-not-ext | 6.3 | Fetal kidney | 30-4-1-E7-PU |
| | | | Cancerous prostate | |
| | | | Lung (cells) | |
| ID169 | est-not-ext | 6.3 | Normal prostate | 33-51-3-H4-PU |
| | | | Brain | |
| ID170 | est-not-ext | 6.3 | Cancerous prostate | 57-27-3-A11-PU |
| | | | Fetal brain | |
| ID171 | est-not-ext | 6.3 | Hypertrophic prostate | 57-5-4-G1-PU |
| | | | Fetal brain | |
| | | | Normal prostate | |
| | | | Brain | |
| ID172 | est-not-ext | 6.2 | Fetal kidney | 58-6-1-H4-PU |
| | | | Normal prostate | |
| | | | Testis | |
| ID173 | est-not-ext | 6.2 | Fetal kidney | 37-12-I-D7-PU |
| | | | Liver | |
| | | | Cancerous prostate | |
| | | | Umbilical cord | |
| ID174 | est-not-ext | 6.2 | Cancerous prostate | 78-13-1-H1-PU |
| | | | Normal prostate | |
| | | | Large intestine | |
| ID175 | est-not-ext | 6.2 | Brain | 33-18-3-G10-PU |
| | | | Substantia nigra | |
| ID176 | est-not-ext | 6.2 | Normal prostate | 78-39-4-B9-PU |
| | | | Substantia nigra | |
| ID177 | est-not-ext | 6.2 | Brain | 33-18-2-B1-PU |
| | | | Substantia nigra | |
| ID178 | est-not-ext | 6.1 | Fetal kidney | 37-4-3-D5-PU |
| | | | Umbilical cord | |
| | | | Normal prostate | |
| ID179 | est-not-ext | 6.1 | Cerebellum | 58-35-3-D12-PU |
| | | | Muscle | |
| | | | Brain | |
| | | | Substantia nigra | |
| | | | Fetal kidney | |
| | | | Prostate | |
| | | | Hypertrophic prostate | |
| | | | Cancerous prostate | |
| | | | Lung | |
| | | | Lung (cells) | |
| | | | Umbilical cord | |
| | | | Normal prostate | |
| | | | Testis | |
| | | | Lymph ganglia | |
| | | | Large intestine | |
| | | | Surrenals | |
| ID180 | est-not-ext | 6.1 | Fetal liver | 51-38-3-D10-PU |
| | | | Testis | |
| ID181 | est-not-ext | 6.1 | Uterus | 76-14-3-G2-PU |
| | | | Fetal liver | |
| | | | Substantia nigra | |
| | | | Ovary | |
| | | | Cancerous prostate | |
| | | | Fetal brain | |
| | | | Normal prostate | |
| | | | Lymph ganglia | |
| ID182 | est-not-ext | 6.1 | Cancerous prostate | 76-30-1-F7-PU |
| | | | Normal prostate | |
| ID183 | est-not-ext | 6 | Brain | 7643-3-E11-PU |
| | | | Cancerous prostate | |
| ID184 | est-not-ext | 6 | Thyroid | 78-H-2-H7-PU |
| | | | Pancreas | |
| | | | Fetal kidney | |
| | | | Normal prostate | |
| ID185 | est-not-ext | 5.9 | Liver | 59-8-1-B7-PU |
| | | | Lung | |
| ID186 | est-not-ext | 5.8 | Brain | 78-37-4-E6-PU |
| | | | Lung | |
| | | | Normal prostate | |
| ID187 | est-not-ext | 5.8 | Kidney | 59-1-2-E4-PU |
| | | | Cancerous prostate | |
| | | | Lung | |
| ID188 | est-not-ext | 5.7 | Umbilical cord | 78-38-4-G2-PU |
| | | | Normal prostate | |
| ID189 | est-not-ext | 5.7 | Lymphocytes | 20-1-3-G5-PU |
| | | | Spleen | |
| | | | Uterus | |
| | | | Substantia nigra | |
| | | | Fetal kidney | |
| | | | Hypertrophic prostate | |
| | | | Cancerous prostate | |
| | | | Normal prostate | |
| | | | Testis | |
| ID190 | est-not-ext | 5.7 | Brain | 58-37-3-E3-PU |
| | | | Fetal kidney | |
| ID191 | est-not-ext | 5.7 | Brain | 33-15-1-H3-PU |
| | | | Fetal brain | |
| ID192 | est-not-ext | 5.6 | Lymphocytes | 37-1-1-C2-PU |
| | | | Thyroid | |
| | | | Spleen | |
| | | | Uterus | |
| | | | Substantia nigra | |
| | | | Hypertrophic prostate | |
| | | | Umbilical cord | |
| | | | Normal prostate | |
| | | | Surrenals | |
| ID193 | est-not-ext | 5.6 | Fetal kidney | 48-10-1-A8-PU |
| | | | Umbilical cord | |
| | | | Lymph ganglia | |
| ID194 | est-not-ext | 5.6 | Surrenals | 62-1-2-D2-PU |
| ID195 | est-not-ext | 5.6 | Brain | 33-12-4-A7-PU |
| | | | Hypertrophic prostate | |
| ID196 | est-not-ext | 5.6 | Brain | 78-30-4-H3-PU |
| | | | Normal prostate | |
| ID197 | est-not-ext | 5.6 | Cerebellum | 47-8-4-C11-PU |
| | | | Brain | |
| | | | Substantia nigra | |
| | | | Fetal kidney | |
| | | | Hypertrophic prostate | |
| | | | Lung | |
| | | | Fetal brain | |
| | | | Normal prostate | |
| | | | Lymph ganglia | |
| ID198 | est-not-ext | 5.6 | Thyroid | 84-4-2-Cl-PU |
| | | | Brain | |
| ID199 | est-not-ext | 5.6 | Brain | 30-12-4-C2-PU |
| | | | Dystrophic muscle | |
| | | | Lung (cells) | |
| | | | Normal prostate | |
| | | | Testis | |
| ID200 | est-not-ext | 5.6 | Placenta | 1-32-0-D10 |
| | | | Lung | |
| ID201 | est-not-ext | 5.5 | Ovary | 30-1-2-E3-PU |
| | | | Lung (cells) | |
| ID202 | est-not-ext | 5.5 | Ovary | 60-11-I-F1-PU |
| | | | Prostate | |
| | | | Lymph ganglia | |
| ID203 | est-not-ext | 5.5 | Spleen | 33-105-2-C3-PU |
| | | | Brain | |
| | | | Fetal kidney | |
| | | | Prostate | |
| | | | Hypertrophic prostate | |
| | | | Lung (cells) | |
| | | | Umbilical cord | |
| | | | Testis | |
| | | | Lymph ganglia | |
| ID204 | est-not-ext | 5.5 | Cancerous prostate | 76-31-4-HI-PU |
| | | | Normal prostate | |
| ID205 | est-not-ext | 5.5 | Fetal kidney | 30-10-3-B10-PU |
| | | | Ovary | |
| | | | Cancerous prostate | |
| | | | Umbilical cord | |
| | | | Lung (cells) | |
| ID206 | est-not-ext | 5.4 | Muscle | 27-3-2-E11-PU |
| | | | Fetal kidney | |
| | | | Cancerous prostate | |
| | | | Lung | |
| | | | Lymph ganglia | |
| ID207 | est-not-ext | 5.3 | Placenta | 31-9-2-F9-PU |
| | | | Muscle | |
| | | | Brain | |
| | | | Substantia nigra | |
| | | | Cancerous prostate | |
| | | | Umbilical cord | |
| ID208 | est-not-ext | 5.3 | Brain | 47-40-3-D2-PU |
| | | | Substantia nigra | |
| | | | Fetal kidney | |
| ID209 | est-not-ext | 5.3 | Brain | 33-77-1-F10-PU |
| | | | Substantia nigra | |
| | | | Lung | |
| ID210 | est-not-ext | 5.2 | Cerebellum | 51-19-3-D6-PU |
| | | | Ovary | |
| | | | Umbilical cord | |
| | | | Testis | |
| ID211 | est-not-ext | 5.2 | Brain | 51-6-2-F10-PU |
| | | | Hypertrophic prostate | |
| | | | Colon | |
| | | | Testis | |
| ID212 | est-not-ext | 5.2 | Brain | 33-72-4-C5-PU |
| | | | Fetal kidney | |
| | | | Fetal brain | |
| | | | Umbilical cord | |
| | | | Normal prostate | |
| ID213 | est-not-ext | 5 | Brain | 33-18-3-E6-PU |
| | | | Normal prostate | |
| ID214 | est-not-ext | 5 | Brain | 33-5-2-E1-PU |
| | | | Substantia nigra | |
| | | | Fetal kidney | |
| | | | Umbilical cord | |
| | | | Lymph ganglia | |
| ID215 | est-not-ext | 5 | Liver | 76-22-3-E4-PU |
| | | | Uterus | |
| | | | Muscle | |
| | | | Heart | |
| | | | Cancerous prostate | |
| ID216 | est-not-ext | 5 | Fetal kidney | 51-15-2-H5-PU |
| | | | Testis | |
| ID217 | est-not-ext | 4.9 | Colon | 78-33-3-A9-PU |
| | | | Normal prostate | |
| ID218 | est-not-ext | 4.9 | Brain | 58-42-2-H11-PU |
| | | | Substantia nigra | |
| | | | Fetal kidney | |
| | | | Dystrophic muscle | |
| | | | Cancerous prostate | |
| | | | Lung | |
| | | | Lymph ganglia | |
| ID219 | est-not-ext | 4.9 | Brain | 33-111-3-F7-PU |
| | | | Substantia nigra | |
| ID220 | est-not-ext | 4.9 | Substantia nigra | 76-44-3-C5-PU |
| | | | Fetal kidney | |
| | | | Hypertrophic prostate | |
| | | | Cancerous prostate | |
| ID221 | est-not-ext | 4.9 | Substantia nigra | 78-40-4-B10-PU |
| | | | Normal prostate | |
| | | | Testis | |
| | | | Surrenals | |
| ID222 | est-not-ext | 4.9 | Fetal kidney | 78-6-3-F5-PU |
| | | | Normal prostate | |
| ID223 | est-not-ext | 4.9 | Thyroid | 58-48-4-E2-PU |
| | | | Brain | |
| | | | Fetal kidney | |
| ID224 | est-not-ext | 4.8 | Placenta | 77-38-1-F10-PU |
| | | | Hypertrophic prostate | |
| | | | Normal prostate | |
| ID225 | est-not-ext | 4.8 | Lung (cells) | 30-7-4-D6-PU |
| | | | Normal prostate | |
| ID226 | est-not-ext | 4.8 | Cancerous prostate | 48-4-2-H3-PU |
| | | | Lymph ganglia | |
| ID227 | est-not-ext | 4.8 | Brain | 33-77-4-E8-PU |
| | | | Dystrophic muscle | |
| | | | Normal prostate | |
| ID228 | est-not-ext | 4.8 | Brain | 33-111-2-B4-PU |
| | | | Substantia nigra | |
| ID229 | est-not-ext | 4.7 | Normal prostate | 62-8-1-A5-PU |
| | | | Surrenals | |
| ID230 | est-not-ext | 4.7 | Brain | 33-6-1-G11-PU |
| | | | Fetal kidney | |
| ID231 | est-not-ext | 4.7 | Fetal liver | 58-13-1-H2-PU |
| | | | Substantia nigra | |
| | | | Fetal kidney | |
| | | | Heart | |
| | | | Cancerous prostate | |
| | | | Umbilical cord | |
| | | | Normal prostate | |
| ID232 | est-not-ext | 4.7 | Liver | 58-40-2-H6-PU |
| | | | Brain | |
| | | | Substantia nigra | |
| | | | Fetal kidney | |
| | | | Lung (cells) | |
| | | | Testis | |
| | | | Large intestine | |
| ID233 | est-not-ext | 4.7 | Brain | 33-50-3-C3-PU |
| | | | Fetal brain | |
| ID234 | est-not-ext | 4.7 | Thyroid | 62-10-4-C5-PU |
| | | | Spleen | |
| | | | Placenta | |
| | | | Muscle | |
| | | | Brain | |
| | | | Substantia nigra | |
| | | | Fetal kidney | |
| | | | Ovary | |
| | | | Heart | |
| | | | Cancerous prostate | |
| | | | Lung | |
| | | | Fetal brain | |
| | | | Umbilical cord | |
| | | | Normal prostate | |
| | | | Colon | |
| | | | Testis | |
| | | | Lymph ganglia | |
| | | | Surrenals | |
| ID235 | est-not-ext | 4.6 | Prostate | 60-16-2-F2-PU |
| | | | Lung (cells) | |
| ID236 | est-not-ext | 4.6 | Muscle | 33-87-2-D2-PU |
| | | | Brain | |
| | | | Substantia nigra | |
| | | | Fetal brain | |
| | | | Testis | |
| ID237 | est-not-ext | 4.6 | Liver | 33-80-3-B8-PU |
| | | | Brain | |
| ID238 | est-not-ext | 4.5 | Liver | 22-12-3-D4-PU |
| | | | Cancerous prostate | |
| | | | Normal prostate | |
| ID239 | est-not-ext | 4.5 | Lymphocytes | 48-51-4-C11-PU |
| | | | Spleen | |
| | | | Uterus | |
| | | | Placenta | |
| | | | Muscle | |
| | | | Brain | |
| | | | Substantia nigra | |
| | | | Fetal kidney | |
| | | | Ovary | |
| | | | Prostate | |
| | | | Dystrophic muscle | |
| | | | Hypertrophic prostate | |
| | | | Heart | |
| | | | Cancerous prostate | |
| | | | Lung | |
| | | | Fetal brain | |
| | | | Lung (cells) | |
| | | | Umbilical cord | |
| | | | Normal prostate | |
| | | | Colon | |
| | | | Testis | |
| | | | Lymph ganglia | |
| | | | Surrenals | |
| ID240 | est-not-ext | 4.5 | Cerebellum | 47-15-1-H8-PU |
| | | | Substantia nigra | |
| | | | Normal prostate | |
| ID241 | est-not-ext | 4.4 | Hypertrophic prostate | 30-12-3-G5-PU |
| | | | Lung (cells) | |
| ID242 | est-not-ext | 4.4 | Brain | 58-4-4-D4-PU |
| | | | Fetal kidney | |
| | | | Cancerous prostate | |
| | | | Umbilical cord | |
| | | | Normal prostate | |
| ID243 | est-not-ext | 4.4 | Spleen | 53-3-2-D4-PU |
| ID244 | est-not-ext | 4.4 | Pancreas | 58-54-2-H8-PU |
| | | | Fetal kidney | |
| ID245 | est-not-ext | 4.4 | Thyroid | 27-17-2-C12-PU |
| | | | Kidney | |
| | | | Muscle | |
| | | | Brain | |
| | | | Ovary | |
| | | | Cancerous prostate | |
| | | | Umbilical cord | |
| | | | Normal prostate | |
| ID246 | est-not-ext | 4.4 | Liver | 48-5-3-A1-PU |
| | | | Placenta | |
| | | | Heart | |
| | | | Normal prostate | |
| | | | Lymph ganglia | |
| ID247 | est-not-ext | 4.4 | Placenta | 33-21-3-D 12-PU |
| | | | Brain | |
| ID248 | est-not-ext | 4.4 | Substantia nigra | 47-2-3-B3-PU |
| | | | Fetal kidney | |
| | | | Umbilical cord | |
| ID249 | est-not-ext | 4.3 | Muscle | 58-15-2-D7-PU |
| | | | Fetal kidney | |
| | | | Cancerous prostate | |
| | | | Lung (cells) | |
| ID250 | est-not-ext | 4.3 | Substantia nigra | 58-41-1-G7-PU |
| | | | Fetal kidney | |
| | | | Fetal brain | |
| ID251 | est-not-ext | 4.2 | Brain | 77-5-3-F3-PU |
| | | | Fetal kidney | |
| | | | Hypertrophic prostate | |
| | | | Normal prostate | |
| ID252 | est-not-ext | 4.2 | Brain | 33-106-2-B3-PU |
| | | | Fetal kidney | |
| ID253 | est-not-ext | 4.2 | | 58-3-3-B2-PU |
| ID254 | est-not-ext | 4.2 | Normal prostate | 48-46-2-G12-PU |
| | | | Lymph ganglia | |
| ID255 | est-not-ext | 4.1 | Brain | 58-44-2-B3-PU |
| | | | Substantia nigra | |
| | | | Fetal kidney | |
| | | | Hypertrophic prostate | |
| | | | Lung (cells) | |
| | | | Testis | |
| ID256 | est-not-ext | 4.1 | Cerebellum | 47-18-4-E3-PU |
| | | | Substantia nigra | |
| ID257 | est-not-ext | 4.1 | Muscle | 78-21-3-F8-PU |
| | | | Substantia nigra | |
| | | | Normal prostate | |
| ID258 | est-not-ext | 4.1 | Brain | 33-49-1-H4-PU |
| | | | Surrenals | |
| ID259 | est-not-ext | 4.1 | Brain | 23-11-1-E11-PU |
| | | | Fetal kidney | |
| | | | Fetal brain | |
| | | | Normal prostate | |
| | | | Colon | |
| ID260 | est-not-ext | 4 | Cerebellum | 33-5-2-H4-PU |
| | | | Brain | |
| | | | Heart | |
| | | | Fetal brain | |
| | | | Normal prostate | |
| ID261 | est-not-ext | 4 | Brain | 78-12-4-D9-PU |
| | | | Normal prostate | |
| ID262 | est-not-ext | 4 | Spleen | 33-103-1-D10-PU |
| | | | Brain | |
| | | | Hypertrophic prostate | |
| | | | Normal prostate | |
| ID263 | est-not-ext | 4 | Placenta | 33-100-4-B7-PU |
| | | | Brain | |
| | | | Substantia nigra | |
| | | | Hypertrophic prostate | |
| ID264 | est-not-ext | 3.9 | Dystrophic muscle | 29-11-2-D6-PU |
| | | | Umbilical cord | |
| ID265 | est-not-ext | 3.9 | Normal prostate | 78-27-3-D1-PU |
| ID266 | est-not-ext | 3.9 | Brain | 76-30-1-H7-PU |
| | | | Hypertrophic prostate | |
| | | | Cancerous prostate | |
| ID267 | est-not-ext | 3.9 | Uterus | 74-10-3-C9-PU |
| | | | Substantia nigra | |
| | | | Hypertrophic prostate | |
| ID268 | est-not-ext | 3.9 | Cancerous prostate | 76-19-1-A9-PU |
| ID269 | est-not-ext | 3.9 | Liver | 76-44-4-A6-PU |
| | | | Muscle | |
| | | | Brain | |
| | | | Cancerous prostate | |
| | | | Normal prostate | |
| ID270 | est-not-ext | 3.8 | Uterus | 74-2-1-H4-PU |
| | | | Brain | |
| | | | Substantia nigra | |
| ID271 | est-not-ext | 3.8 | Muscle | 27-21-1-H3-PU |
| | | | Lung (cells) | |
| ID272 | est-not-ext | 3.8 | Placenta | 33-13-3-E8-PU |
| | | | Brain | |
| ID273 | est-not-ext | 3.8 | Thyroid | 84-3-1-G10-PU |
| | | | Brain | |
| | | | Heart | |
| | | | Cancerous prostate | |
| | | | Fetal brain | |
| | | | Lung (cells) | |
| | | | Normal prostate | |
| | | | Testis | |
| | | | Lymph ganglia | |
| ID274 | est-not-ext | 3.7 | Uterus | 33-8-1-A3-PU |
| | | | Brain | |
| | | | Fetal kidney | |
| | | | Cancerous prostate | |
| ID275 | est-not-ext | 3.7 | Dystrophic muscle | 76-43-4-H1-PU |
| | | | Cancerous prostate | |
| ID276 | est-not-ext | 3.7 | Thyroid | 84-5-4-H7-PU |
| | | | Placenta | |
| ID277 | est-not-ext | 3.7 | Brain | 37-4-1-B2-PU |
| | | | Lung (cells) | |
| | | | Umbilical cord | |
| | | | Testis | |
| | | | Lymph ganglia | |
| ID278 | est-not-ext | 3.7 | Kidney | 74-11-4-A9-PU |
| | | | Placenta | |
| | | | Uterus | |
| | | | Hypertrophic prostate | |
| | | | Normal prostate | |
| | | | Lymph ganglia | |
| | | | Surrenals | |
| ID279 | est-not-ext | 3.7 | Substantia nigra | 77-2-2-B9-PU |
| | | | Hypertrophic prostate | |
| | | | Cancerous prostate | |
| ID280 | est-not-ext | 3.7 | Fetal kidney | 58-8-1-F2-PU |
| | | | Cancerous prostate | |
| | | | Lymph ganglia | |
| ID281 | est-not-ext | 3.7 | Uterus | 74-7-2-F2-PU |
| | | | Prostate | |
| | | | Normal prostate | |
| | | | Lymph ganglia | |
| ID282 | est-not-ext | 3.6 | Fetal kidney | 37-2-1-H11-PU |
| | | | Umbilical cord | |
| | | | Testis | |
| | | | Large intestine | |
| ID283 | est-not-ext | 3.5 | Lymphocytes | 58-6-1-F3-PU |
| | | | Brain | |
| | | | Fetal kidney | |
| | | | Normal prostate | |
| ID284 | est-not-ext | 3.5 | Muscle | 33-54-3-G1-PU |
| | | | Brain | |
| | | | Hypertrophic prostate | |
| ID285 | est-not-ext | 3.5 | Fetal liver | 47-39-2-H6-PU |
| | | | Substantia nigra | |
| ID286 | est-not-ext | 3.5 | Brain | 76-17-1-F5-PU |
| | | | Cancerous prostate | |
| | | | Surrenals | |
| ID287 | est-not-ext | 3.5 | Placenta | 27-7-3-D1-PU |
| | | | Muscle | |
| | | | Heart | |
| | | | Cancerous prostate | |
| | | | Lung (cells) | |
| | | | Umbilical cord | |
| | | | Colon | |
| ID288 | est-not-ext | 3.5 | Liver | 74-5-1-E4-PU |
| | | | Uterus | |
| | | | Muscle | |
| | | | Brain | |
| | | | Ovary | |
| | | | Dystrophic muscle | |
| | | | Cancerous prostate | |
| | | | Normal prostate | |
| | | | Colon | |
| | | | Large intestine | |
| ID289 | est-not-ext | 3.5 | Brain | 57-20-1-F6-PU |
| | | | Cancerous prostate | |
| | | | Fetal brain | |
| | | | Umbilical cord | |
| | | | Surrenals | |
| ID290 | ext-vrt-not-genomic | 7.4 | Spleen | 48-25-3-A3-PU |
| | | | Hypertrophic prostate | |
| | | | Lymph ganglia | |
| ID291 | ext-vrt-not-genomic | 7 | Brain | 46-1-3-F4-PU |
| | | | Pancreas | |
| | | | Hypertrophic prostate | |
| | | | Normal prostate | |

**TABLE III**

| SEQ. ID NO. | SIGNAL PEPTIDE |
|---|---|
| ID38 | MSSWSRQRPKSPGGIQPHVSRTLFLLLLLAASAWG |
| ID39 | MRVRIGLTLLLXAVLLSLASA |
| ID40 | MFSHLPFDCVLLLLLLLLTRS |
| ID41 | MGPVRLGILLFLFLAVDEAWA |
| ID42 | MKSLSLLLAVALGLATA |
| ID43 | MLLLLTLXLLGGPTWA |
| ID44 | MKIGILLSLLNSVISQTLMSCNWKQQMRRMKTILIILIXIWIWCLG |
| ID45 | MKASSGRCGLVRWLQVLLPFLLSLFPGALP |
| ID46 | MlVDCVSSHLKKTGDGAKTFIlFLCHLLRGLHA |
| ID47 | MAKALLFPSGRSVRVLYGAVNKERQXESVLNRACPPKANSKERRGRAVLGAELTQWSSPT TAGSCCSSCTLCARSSSXVIAPSPLVPFTSGLTSLSWLLXASCS |
| LD48 | MAASEAAWSSPSLKTDTSPVLETAGTVAAMAATPSARAAAAWAAAARTGSEARVS KAALATKLLSLSGVFA |
| ID49 | MKVGVLWLISFFTFRDG |
| ID50 | MEFGLSWIFLAAILKGVQC |
| ID51 | MAEPGHSHHLSARVRGRTERRIPRLWRLLLWAGTAFQ |
| ID52 | MTADPRKGRMGLQACLLGLFALILS |
| ID53 | MLVDGPSERPALCFLLLAVAMSFF |
| ED54 | MAAPLVLVLVVAVTVRA |
| ID55 | MTAAIRRQRELSILPKVTLEAMNTTVMQGFNRSERCPRDTRIVQLVFPALYTVVFLTGIL LNTLALWVFVHMSSSTFIIYLKNTLVADLXMTLMLPFKILS |
| ID56 | MSSVLAASHPLVLSSNAGTPGISEKDNRDPAGSSIGVLTLSHLISG |
| ID57 | MGLAMEHGGSYARAGGSSRGCWYYLRYFFLFVSLIQFLIILGLVLFMVYG |
| ID58 | MVEASLSVRHPEYNRPLLANDLMLIKLDESVSESDTIRSISIASQCPTAGNSCLVSGWGL LANG |
| ID59 | MGGKQRDEDDEAYGKPVKYDPSFRGPIKNRSCTDVICCVLFLLFILG |
| ID60 | MQKASVLLFLAWVCFLFY |
| ID61 | MSPVLHFYVRPSGHEGAASGHTRRKLQGKLPELQGVETELCYNVNWTAEALPSAEETKKL MWLFGCPYCWMMLLGSXGSFL |
| LD62 | MDVTPRESLSILVVAGSGGHTTEILRLLGSLSNAYS |
| ID63 | MMGVAKLTLLRVLNLPHNSIG |
| ID64 | MDVTPRESLSILVVAGSGGHTTEILRLLGSLSNAYS |
| ID65 | MVLLTMIARVADG |
| ID66 | MVPVENTEGPSLLNQKGTAVETEGXGSRHPPWARGCGMFTFLSSVXA |
| ID67 | METFLEPNNKKLLFPVGRSWSCFA |
| ID68 | MGFLWGLALPLFFFC |
| ID69 | MQSTSNHLWLLSDILGQGATA |
| ID70 | MVEICAGSVLPPYSNC |
| ID71 | MVAPVLETSHVFCCPNRVRGVLNWXSGPRGLLAFGTSCSVVXY |
| ID72 | MDSLRKMLISVAMLGAXAGVGYALLVIVTPGERRKQEMLKENIPLQDPRSREEAART QQLLLATLQEAATT |
| ID73 | MRQTLPCIYFWGGLLPFGMLCASSTT |
| ID74 | MADDLEQQSQGWLSSWLPTWRPTSMSQLKNVEARILQCLQNKFLARYVSLPNQNKI WTVTVSPEQNDRTPLVMVHGFGGGVGLWILNMDSLXARRTLHTXGLLGFGRXQG |
| ID75 | MKVTGITILFWPLSMILLSDKIQS |
| ID76 | MAAGRAQVPSSEQAWLEDAQVFIQKTLCPAVKEPNVQLTPLVIDCVKTVWLSQGRN QGSTLPLSYSFVSVQDLKTHQRLPCCSHLSWSSSAYQAWA |
| ID77 | MSTCCWCTPGGAST |
| ID78 | MPFAEDKTYKYICRNFSNFCXVDVVEILPYLPCLTA |
| ID79 | MAESEDRSLRIVLVGKTGSGKSATANTILGEEIFDSRIAAQAVTKNCQKASREWQGRDLL VVDTPGLFDTKESLXTICKEIXRCIISSCPGPHAIVLVLLLGRYTEE |
| ID80 | MAQKPLRLLACGDVEGKFDILFNRVQAIQKXSGNFDLLXCVGNFFGSTQ |
| ID81 | MESRKDITNQEELWKMKPRRNLEEDDYLHKDTGETSMLKRPVLLHLHQTAHA |
| ID82 | MESRKDITNQEEXWKMKPRRNLEEDDYLHKDTGETSMLKRPVLLHLHQTAHA |
| ID83 | MAATCEISNIFSNYFSAMYSSEDSTLASVPPAATFG |
| ID84 | MRDCPGVEXILDCSXRQKTEGCRLQAGKECVDSPVEGGQSEAPPSLVSFAVSSEGTEQ |
| ID85 | MERQSRVMSEKDEYQFQHQGAVELLVFNFLLILTILT |
| ID86 | MKMASSLAFLLLNFHVSLLLVQLLTPCSA |
| ID87 | MVFLPLKWSLATMSFLLSSLLALLTVSTPSWC |
| ID88 | MESAAALHFSRPASLLLLLLXCVHWS |
| ID89 | MEKIPVSAFLLLVALSYTLA |
| ID90 | MGPWGEPELLVWRPEAVASEPPVPVGLEVKLGALVLLLVLTLLCSL |
| ID91 | MAPLLLQLAVLGAALA |
| ID92 | MAMEGYWRFLXLLGSALLVGFLSVIFA |
| ID93 | MAQSLALSLLILVLAFG |
| ID94 | MEAMWLLCVALAVLA |
| ID95 | MAPITTSREEFDEIPTVVGIFSAFGLVFTVSLFAWICC |
| ID96 | MEGPRGWLVLCVLAISLA |
| ID97 | MTAWEAMAPHVNPTLKDKALSPQQXXXTSPAPCXSNHHNKKHLILAFCAGVLLTLLLIAF IFL |
| ID98 | MLCSLLLCECLLLXAGYA |
| ID99 | MGHAMGLVXSLPVHCLTFA |
| ID100 | MARCFSLVLLLTSIWT |
| ID101 | MLLTRKQTCQLGILLSIHRQHSKDLQDIVATLGPRSATHPHQPAIQVLAQLAFLSQISQ |
| ID102 | MWAFSELPMPLLINLIVSLLGFVATVTL |
| ID103 | MFKVIQRSVGPASLSLLTFKVYA |
| ID104 | MAKSLLKTASLSGRTKLLHQTGLSLYSTSHGFYEEEVKKTLQQFPGGSIDLQKEDNGIGI LTLNNPSRMNAFSGVMMILQLLEKVIELEWTEGKGLIVRGAKNTFSSGSDLNAVKSLGLQ RLPLISVALVQGWALG |
| ID105 | MTSFSTSAQCSTSDSACRISPGQINXVRPKLPLLKJLHAAGAQG |
| ID106 | MDTAEEDICRVCRSEGTPEKPLYHPCVCTGSKXVHQECLVQWLKHSRKEYCELCKHRFA FTPIYSPDMPSRLPIQDIFAGLVTSIGTAIRYWFHYTLVAFAWLGWPLTAC |
| ID107 | MLINILGIFFNVHS |
| ID108 | MGGLWRPGWRCVPFCGWRWIHPGSPTRAAERVEPFLRPEWSGTGGAERGLRWLGTWKR CSLRARHPALQPPRRPKSSNPFTRAXEEERRRXNKTTLTYVAAVAVGMLXASYA |
| ID109 | MAAQCVTKVALNVSCANLLDKDIGSKSDPLCVLFLNTSG |
| ID110 | MTGSNEFKLNQPPEDGISSVKFSPNTSQFLLVSSWDTSVRLYDVPANSMRLKYQHTGAVL DCAFYDPTHA |
| ID111 | MGKHLWYPGQASAHLCWCGSHCCST |
| ID112 | MLAVSLTVXLLGA |
| ID113 | MSSTLAKIAEIEAEMARTQKNKATAHHLGLLKARLAKLRRELITPKGGGGGGPGEGFDWP RQVMLELDLLVFHLWG |
| ID114 | MAAAVPKRMRGPAQAKLLPGSAIQALVGLARPLVLALLLVSAALS |
| ID115 | MTPQSLLQTTLFLLSLLFLVQGAHG |
| ID116 | MMVVGTGTSLALSSLLSLLLFAGMQIYSRQLASTEWLTIQGGLLGSGLFVFSLTAFNNLE NLVFGKGFQAKIFPEILLCLLLALFASG |
| ID117 | MDWTWRVFCLLAVAPGAHS |
| ID118 | MRIANRTRFSSPFLARGAGWTHGRGMMVVGTGTSLALXSLLSLLLFAGMQMYSRQLASTE WLTIQGGLLGSGLFVFSLTAFNNLENLVFGFGKGFQAKIFPEILLCLLLALFASG |
| ID119 | MTSVSTQLSLVLMSLLLVLPWEA |
| ID120 | MTPLLTLILVVLMGLPLAQA |
| ID121 | MALLLALSLLVLWTSP |
| ID122 | MGGLEPCSRLLLLPLLLAVSG |
| ID123 | MEVPPPAPRSFLCRALCLFPRVFA |
| ID124 | MDLRQFLMCLSLCTAFALS |
| ID125 | MAGGVRPLRGLRALCRVLLFLSQFCILSGG |
| ID126 | MAAAAWLQVLPVILLLLGAHP |
| ID127 | MRTLFNLLWLALACSPVHT |
| ID128 | MDVLFVAIFAVPLILG |
| ID129 | MAAAAWLQVLPVILLLLGAHP |
| ID130 | MRTLFNLLXLALACSPVHT |
| ID131 | MGSKVADLLYWKDTRTSGVVFTGLMVSLLCLLHFSIVSVA |
| ID132 | MAARWRFWCVSVTMWALLIVCDVPSASA |
| ID133 | MEGESTSAVLSGFVLGALA |
| ID134 | MFAPAVMRAFRKNKTLGYGVPMLLLIVGGSFG |
| ID135 | MAAAWXSGPSAPEAVTARLVGVLWFVSVTTGPWGAVATSAGGEESLKCEDLKVGQ YICKDPKINDATQEPVNCTNYTAHVSCFPAPNITCKDSSGNETHFTGNEVGFFKPISCRNV NGYSYKVAVALSLFLGWLGA |
| ID136 | MRTLFNLLWLALACSPVHT |
| ID137 | MDGQKKNWKDKVVDLLYWRDIKKTGVVFGASLFLLLSLTVFS |
| ID138 | MVAPGLVLGLVLPLILWA |
| ID139 | MSPSGRLCLLTIVGLILPTRG |
| ID140 | MRIANRTRFSLPFLARGAGWTHGRGMMWGTGTSLALSSLLSLLLFA |
| ID141 | MVLGGCPVSYLLLCGQAALLLGNLLLLHCVSRSHS |
| ID142 | MGSVLGLCSMASWIPCLCGSAPCLLCRCCPSGNNSTVTRLIYALFLLVGVCVA |
| ID143 | MVLLHVLFEHAVGYALLALKEVEEISLLQPQVEESVLNLGKFHSIVRLVAFCPFASS |
| ID144 | MSGGRAPAVLLGGVASLLLSFVWMPALLPVASRLLLLPRVLLTMASG |
| ID145 | MVAPVWYLVAAALLVGFILFLTRSRG |
| ID146 | MAVLAPLIALVYSVPRLSRWLAQPYYLLSALLSAAFLLVRKLPPLCHG |
| ID147 | MVGEAGRDLRRRRXXAVTAXKMAVLAPLIALVYSVPRLSRWLAQPYYLLSALLSAAFLLV RKLPPLCHG |
| ID148 | MEALGKLKQFDAYPKTLEDFRVKTCGGATVTIVSGLLMLLLFLSELQY |
| ID149 | MAVLAPLIALVYSVPRLSRWLAQPYYLLSALLSAAFLLVRKLPPLCHG |
| ID150 | MRCLTTPMLLRALAQAARA |
| ID151 | MRCLTTPMLLRALAQAARA |
| ID152 | MDFITSTAILPLLFGCLGVFG |
| ID153 | MHPAVFLSLPDLRCSLLLLVTWVFTPVTT |
| ID154 | MASLGHILVFCVGLLTMAKA |
| ID155 | MSGSSLPSALALSLLLVSGSLLP |
| ID156 | MAVHDLIFWRDVKKTGFVFGTTLIMLSLAAFSVIS |
| ID157 | MXGSVECTXGWGHCAPSPLLLWTLLLFAAPFG |
| ID158 | MQCFSFIKTMMILFNLLIFLCGAALLAVG |
| ID159 | MRGSVECTWGXGHCAPSPLLLWTLLLFAAPFG |
| ID160 | MALRLLKLAATSASA |
| ID161 | MPSAFSVSSFPVSIPAVLTQTDWTEPWLMGLATFHALCVLLTCLSSRSYRLQIGHFLCLV ILVYC |
| ID162 | MALPHQEPKPGDLIEIFRLGYEHWALYIXDGYVIHLAPPSEYPGAGSSSVFSVLSNSAEV KRERLEDWGGCCYRVNNSLDHEYQPRPVEVIISSAKEMVGQKMKYSIVSRNCEHFVTQL RYGKSRCKQVEKAKVEVGVATALGILWAGCSFA |
| ID163 | MAASTSMVPVAVTAAVAPVLSINSDFSDLREIKKQLLLIAGLTRERGLLHSSKWSAELAF SLPALPLAEL |
| ID164 | MEEGGNLGGLIKMVHLLVLSGAWG |
| ID165 | MAGPAAAFRRLGALSGAAALGFASYGAI-IGAXFPDAYGKELFDKANKHFLHSLALL GVPHCRKPLWAGLLLASGTTLFCTS |
| ID166 | MGHRFLRGLLTLLLPPPPLYT |
| ID167 | MELLQVTILFLLPSICSSNS |
| ID168 | MASSNTVLMRLVASAYSIA |
| ID169 | MRSSCVLLTALVALA |
| ID170 | MGIQTSPVLLASLGVGLVTLLGLAVG |
| ID171 | MTLQWAAVATFLYAE[GLELIFCLPFIPPQRWQKIFSFNVWGKIATFWNKAFLTIIILLI VLFLDAVRE |
| ID172 | MPSEGRCWETLKALRSSDKGRLCYYRDWLLRREVSGGPGGRRPFRPLATETFSLAVGTFC SREPVQSNNLHLFLDFCVYIPLSWG |
| ID173 | MTKLAQWLWGLAILGSTWVALTTG |
| ID174 | MLLAWVQAFLVSNMLLAEAYG |
| ID175 | MAMHFIFSDTAVLLFHFWSVHSPAGMALSVLVLLLLAVLYE |
| ID176 | MKQVHQCIERCHVPLAQAQALVTSELEKFQDRLARCTMHCNDKAKDSIDAGXKELQ VKQQLXVVXXSVLXTTCXS |
| ID177 | MQMSYAIRCAFYQLLLAALMLVAMLQL |
| ID178 | MMTQTCIILLIHTMQVCTT |
| ID179 | MXXHLQTRPLFLTCLFWPLAAL |
| ID180 | MAANYSSTXTRREHVKVKTSSQPGFLERLSETSGGMFVGLMAFLLSFYLIFT |
| ID181 | MRGAHLTALEMLTAFASHIRA |
| ID182 | MVHKPMMTQTCIELLIHTMQVCTT |
| ID183 | MAGIKALISLSFGGAIGLMFLMLGCALP |
| ID184 | MSLMPKMHLLFPLTLVRSFWS |
| ID185 | MMKRAAAAAVGGALAVGAVPVVLSAMGFTGAGIAASSIAAKMMSAAAIANGGGVSA GSLVATLQSVGAAGLSTSSNILLASVGSVLG |
| ID186 | MVTIILLLSCXFWA |
| ID187 | MXKRAAAAAVGGALAVGAVPVVLSAMGFTGAGIAASSIAAKMMSAAAJANGGGVSA GSLVATLQSVGAAGLSTSSNILLASVGSVSG |
| ID188 | MSQDGGXGELKHMVMSFRVSELQVLLGXXGRNKSGRKHELLAKALHLLKSSCAPSVQ MKIKELYRRRFPRKTLGPSDLSLLSLPPGTSP |
| ID189 | MPXLLPVASRLLLLPRVLLTMASG |
| ID190 | MVFSNNDEGLINKKLPKELLLRIFSFLDIVTLCRC |
| ID191 | MVFSNNDEGLINKKLPKELLLRIFSFLDIVTLCRC |
| ID192 | MASYFDEHDCEPSDPEQETRTNMLLELARSLFNRMDFEDLGLVVDWDHHLPPPAAKTVVE NLPRTVIRGSQAELKCPVCLLEFEEEETAIEMPCHHLFHSSCILPWLSKTNS |
| ID193 | MPLILSLQVCRPATL |
| ID194 | NILGITSCSDQQAKEGEGLEGSSTGSSSGNHGGSGGGNGHKPGCEKPGNEARGSGNLGFRT LRRLLGCLTLTLS |
| ID195 | MARKALKLASWTSMALA |
| ID196 | MAAAALPAWLSLQSRA |
| ID197 | MVKIAFNTPTAVQKEEARQDVEALLSRTVRTQILTGKELRVATQEKEGSSGRCMLTLXXL SFILA |
| ID198 | MIGSGLAGSGGAGGPSSTVTWCALXSNHVAATQASLLLSFVWMPALLP |
| ID199 | MSGAQLXGFLFXVIVLTS |
| ID200 | MSFFQLLMKRKELLPLVVFMTVAASGASS |
| ID201 | MELAHSLLLNEEALA |
| LD202 | MTSALTQGLERIPDQLGYLVLSEGAVLA |
| ID203 | MAAAWPSGPXAPEAVTARLVGVLWFVSVTTG |
| ID204 | NIVLLTMIARVADG |
| ID205 | MVLLTMIARVADG |
| ID206 | MTSQPVPNETIIVLPSNVINFSQAEKPEPTNQGQDSLKKHLHAEIKVIGTIQILCGMMVL SLGIXLASA |
| ID207 | MASVVLALRTRTAVTSLLSPTPATA |
| ID208 | MASVVLALRTRTAVTSLLSPTPATA |
| ID209 | MMPSRTNLATGIPSSKVKYSRLSSTDDGYIDLQFKKTPPKIPYKAIALATVLFLIGA |
| ID210 | MPLILSLQVCRPATL |
| ID211 | MPLILSLQVCRPATL |
| ID212 | MASSVGNVADSTEPTKRMLSFQGLAELAHREYQAGDFEAAERHCMQLWRQEPDNTG VLLLLSSIHFQC |
| ID213 | MFGSAPQRPVAMTTAQRDSLLWKLAGLLREXGDVVLSGCSTLSLLTPTLQQLNHVFELHL GPWGPGQTGFVALPSHPADSPVILQLQFLFDVLQ |
| ID214 | MSFIFEWIYNGFSSVLQFLGLYKKSGKLVFLGLDNAGKTTLLHMLKDDRLGQHVPTLHPT SEELTIAGMTLQLLILVGTSKHVAFG |
| ID215 | MDKPCGCPPGVCDHGTGDRRDPWYSTVGLLPPVRA |
| ID216 | MAAALKCLLTLGRWCPGLGVAPQARALAALVPGVTQ |
| ID217 | MVARVWSLMRFLIKGSVAGGAVYLVYDQELLGPSDKSQAALQKAGEVVPPAMXQFS QYVCQQTGLQIPQLPAPPKIYFPIRDSWXAGIMTVMSALSVAPSKA |
| ID218 | MVNELQNLXSLQGSQA |
| ID219 | MLYMSLKYIRAFFFSIQPFLPCSS |
| ID220 | MNLERVSNEEKLNLCRKYYLGGFAFLPFLWLVNIFWFFREAFLVPAYTEQSQIKGYVWRS AVGFLFWVIVLTSWITIFQ |
| ID221 | MAGELQGTQAPSLRGXGLTSQDSGVNPNNSXRGREAMASGSNWLSGVNWLVMAYG SLVFVLLFIFVKRQ |
| ID222 | MTGFLLPPASRGTRRSCSRSRKRQTRRRRNPSSFVASCPTLLPFACVPGASPTTLA |
| ID223 | MEEXSXPLVEFVKVLCTNQVLITARA |
| ID224 | MVRRLXXWAFVAPGES |
| ID225 | MAVPGVGLLTRLNLCARRRTRVQRPIVRLLSCPGTVA |
| ID226 | MMAAWPGLEPWNRVRIPKAGNRSAVTVQNPGAALDLCIAAVIKECHLVILSLKSQTLDA |
| ID227 | MASLDRVKVLVLGDSGVGKSSLVHLLCQNQVLG |
| ID228 | MVFPAKRFCLVPSMEGVRWAFSCGTWLPSRA |
| ID229 | MASKIGSRRWMLQLIMQLGSVLLTRC |
| ID230 | MLSKGLKRKREEEEEKEPLAVDSWWLDPGHA |
| ID231 | MDYSLAAALTLHGHWG |
| ID232 | MSYITSQEMKCILHWFANWSGPQRERFLEDLVAKAVPEKLQPXLDSLEQLSVSGADDHLL SLXASYIFGISG |
| ID233 | MPLLCQIEMEYLLLKWQMTMLQSMLCDLVSYPLLPLQQTKEANLDFPKIKVSSVTITPTR WFXLIVYLWVVSFIAS |
| ID234 | MWFEILPGLSVMGVCLLIPGLA |
| ID235 | MEFKLEAHRIVSISLGKIYNSRVQRGGIKLHKNLLVSLVLRXPAKS |
| ID236 | MAVLSKEYGFVLLTGAASFWIVAHLAINVSKARKKYKVEYPIMYSTDPENGHIFNCIQRA HQNTLEVYPXFLFFLAVGGVYHPRIASGLGLXLDCWT |
| ID237 | MDGHWSAAFSALTVTAMSSWARRRSSSSRRIPSLPGSPVCWA |
| ID238 | MAQRLLLRRFLASVIS |
| ID239 | MASLKPAFVNYFFLLLLEVSHLLLI |
| ID240 | MNLERVSNEEKLNLCRKYYLGGFAFLPFLWLVNTFWFFREAFLVPAYTEQSQIKGYVWRS AVGFLFWVIVLTSWITI |
| ID241 | MAQLGAVVAVASSFFCASLFS |
| ID242 | MSLRNLWRDYKVLVFMVPLVGLIHI. |
| ID243 | MGWDGCKCLGVFCLLISIPTPSA |
| ID244 | MAASQAVEEMRTAWFWGSLGFAMSILLTFPVTIPVMMPGTRXGFEXRXFRVDVVH MDENSLEFDMVGIDAAIANAFRRILLAEVPTMAVEKVLVYNNTSIVQDEILAHRLGLIPIHA |
| ID245 | MAASKVKQDMPPPGGYGPLDYKRNLPRRGLSGYSMLAIGIGTLIYGHWSIMKWNRERRRL QIEDFEARIALLPLLQA |
| ID246 | MSGFLEGLRCSECIDWGEKRNTIASIAAGVLFFTGWWIIIDA |
| ID247 | MMTQEPGIYTWPEKTRIICSACSSVPLPWTVLVFLTFLSIPSFV |
| ID248 | MFLTALLWRGRIPG |
| ID249 | MNQENPPPYPGPGPTAPYPPYPPQPMGPGXMGGPYPPPQGYPYQGYPQYGWQGGPQEPPK TTVYVVEDQRRDELGPSTCLTACWTALCCC |
| ID250 | MASLEVSRSPRRSRRELEVRSPRQNKHSVLLPTYNEREELPLIVWLLVKSFSES |
| ID251 | MCPTCLCAPSXXWG |
| ID252 | MAAATGAVAASAASGQAEG |
| ID253 | MAAMSLLXRVSVTAVAA |
| ID254 | MAGPLQGGGARALDLLRGLPRVSLA |
| ID255 | MATATEQWVLVEMVQALYEAPAYHLILEGILILWIIRLLFS |
| ID256 | MEDPNPEENMXQQDSPKERSPQSPGGNICHLGAPKCTRCLITFADSKXXERHMKREHPAD FVAQKLQGVLFICFTCARS |
| ID257 | MNVIDHVRDMAAAGLHSNVRLLSSLLLTMSNN |
| ID258 | MQNVINTVKGKALEVAEYLTPVLKESKFKETGVITPEEFVAAGDHLVHHCPTWQWATG |
| ID259 | MATLTFSLRKPLQRSLIRPSHLPLCCFDWRLSHYYRLPPAVRLHQQRGGRPGRSSADHWH SGVPTRILPPAHRLLCIQRLPWLLLCRG |
| ID260 | MEKPLFPLVPLHWFGFGYTALWSGGIVGYVKTGSVPSLAAGLLFGSXA |
| ID261 | MASTWAVGLTIAAAGFA |
| ID262 | MVIRVYIASSSGSTAIKKKQQDVLGFLEANKIGFEEKDIAANEENRKWMRENVPENSRPA VQGPHAFRYKAFSFSRLLSQCRP |
| ID263 | MSSRGHSTLPRTLMAPRMISEGDIGGIAQITSSLFLGRGSVA |
| ID264 | MAAPGPALCLFDVDGTLT |
| ID265 | MPLGARILFHGVFYAGGFA |
| ID266 | MLLSIGMLMLSAT |
| ID267 | MSLTSSSSVRVEWIAAVTIAAGTAA |
| ID268 | MSGSNGSKENSHNKARTSPYPGSKVERSQVPNEKVGWLVEWQDYKPVEYTAVSVLA GPRWA |
| ID269 | MAISLRSSGISVKCLSKLWMRWTVTSTTRA |
| ID270 | MSEVRLPPLRALDDFVLGSARLGGSGS |
| ID271 | MKLVSATAWLEECWW |
| ID272 | MKAISVSLLRLTKLLWFFSIVLYVPLLAVCCLHS |
| ID273 | MGSLSGLRLAAGSCFRLCERDVSXSLRLTRSSDLKRINGFCTKPQESPGAPSRTYNRVPL HKPTDWQKKILIWSGRFKKEXXIPETVSLEMLXXAKNKMRVKISYLMIALTVVGCIFM |
| ID274 | NETLYRVPFLVLECPNLKLKKPPWLHNTSAMTVYALVVVSYFLITGGIIYDVIVEPPSVG SMTDEHGHQRPVAFLAYRVNGQYIMEGLASSFLFTMGGLG |
| DD275 | MLVLRSGLTKALA |
| ID276 | MAAPLSVEVEFGGGAXSCLTVLRNIESLAWTGGTLG |
| ID277 | MTHLLEYDRHRKSRLSPLQHLYLLPADHSPNAAF-RFPGAWFQPPTVDSEASAFVGGLPVI FWSWA |
| ID278 | MAAAALGQIWARKLLSVPWLLC |
| ID279 | MAVESRVTQEEIKKEPEKPIDREKTCPLLLLVFTTNNG |
| ID280 | MRLKYQHTGAVLDCAFYDPTHA |
| ID281 | MALLFARSLRLCRWGAKRLGVASTEAQRGVSFKLXEKTAHSSLALFRDDTGVKYGL VGLEPTKVALNVERFREWAVVLADTAVTSG |
| ID282 | MAAAAAGTXTSQRFFQSFSDALIDEDPQAALEELTKALEQKPDDAQYYCQRAYCHILLGN YCVAVADA |
| ID283 | MAQLKYMENVGYAQEDRERMHRNIVSLAQNLLNFMIGSILDLWQCFLWFYIGSSLNGTRG |
| ID284 | MSPAFRAMDVEPRAKGSFWSPLSTRSGGTHA |
| ID285 | MADEELEALRRQRLAELQAKHGDPGDAAQQEAKHREAEMRNSILAQVLDQSARA |
| ID286 | MSAAGARGLRATYHRLLDKVELMLPEKLRPLYNHPAGPRTVFFWAPIMKWGLVCAGL ADMARP |
| ID287 | MSNYSVSLVGPAPWGFRLQGGKDFNMPLTISSLKDGGKAAQANVRIGDVVLSIDGINAQG MTHLEAQNKIKGCTGXLNMTLQRASA |
| ID288 | MANPKLLGLELSEAEAIG |
| ID289 | MJIPLLEILIIIVLNEVLLFDVNSVYKALLCTLLLHFQNI |
| ID290 | MDIQMANNFTPPSATPQGNDCDLYAHHSTARIVMPLHYSLVFIIGLVGNLLA |
| ID291 | MLTIVKSPQKSYLFPSSMIGIGSLPSCWA |

**TABLE IV**

| Minimum signal peptide score | false positive rate | false negative rate | proba(0.1) | proba(0.2) |
|---|---|---|---|---|
| 3.5 | 0.121 | 0.036 | 0.467 | 0.664 |
| 4 | 0.096 | 0.06 | 0.519 | 0.708 |
| 4.5 | 0.078 | 0.079 | 0.565 | 0.745 |
| 5 | 0.062 | 0.098 | 0.615 | 0.782 |
| 5.5 | 0.05 | 0.127 | 0.659 | 0.813 |
| 6 | 0.04 | 0.163 | 0.694 | 0.836 |
| 6.5 | 0.033 | 0.202 | 0.725 | 0.855 |
| 7 | 0.025 | 0.248 | 0.763 | 0.878 |
| 7.5 | 0.021 | 0.304 | 0.78 | 0.889 |
| 8 | 0.015 | 0.368 | 0.816 | 0.909 |
| 8.5 | 0.012 | 0.418 | 0.836 | 0.92 |
| 9 | 0.009 | 0.512 | 0.856 | 0.93 |
| 9.5 | 0.007 | 0.581 | 0.863 | 0.934 |
| 10 | 0.006 | 0.679 | 0.835 | 0.919 |

**TABLE V**

| Minimum signal peptide score | All ESTs | New ESTs | ESTs matching public EST closer than 40 bp from beginning | ESTs extending known mRNA more 40 bp | ESTs extending public EST more than40 bp |
|---|---|---|---|---|---|
| 3.5 | 2674 | 947 | 599 | 23 | 150 |
| 4 | 2278 | 784 | 499 | 23 | 126 |
| 4.5 | 1943 | 647 | 425 | 22 | 112 |
| 5 | 1657 | 523 | 353 | 21 | 96 |
| 5.5 | 1417 | 419 | 307 | 19 | 80 |
| 6 | 1190 | 340 | 238 | 18 | 68 |
| 6.5 | 1035 | 280 | 186 | 18 | 60 |
| 7 | 893 | 219 | 161 | 15 | 48 |
| 7.5 | 753 | 173 | 132 | 12 | 36 |
| 8 | 636 | 133 | 101 | 11 | 29 |
| 8.5 | 543 | 104 | 83 | 8 | 26 |
| 9 | 456 | 81 | 63 | 6 | 24 |
| 9.5 | 364 | 57 | 48 | 6 | 18 |
| 10 | 303 | 47 | 35 | 6 | 15 |

**TABLE VI**

| Tissue | All ESTs | New ESTs | ESTs matching public EST closer than 40 bp from beginning | ESTs extending known mRNA more than 40 bp | ESTs extending public EST more than 40 bp |
|---|---|---|---|---|---|
| Brain | 329 | 131 | 75 | 3 | 24 |
| Cancerous prostate | 134 | 40 | 37 | 1 | 6 |
| Cerebellum | 17 | 9 | 1 | 0 | 6 |
| Colon | 21 | 11 | 4 | 0 | 0 |
| Dystrophic muscle | 41 | 18 | 8 | 0 | 1 |
| Fetal brain | 70 | 37 | 16 | 0 | 1 |
| Fetal kidney | 227 | 116 | 46 | 1 | 19 |
| Fetal liver | 13 | 7 | 2 | 0 | 0 |
| Heart | 30 | 15 | 7 | 0 | 1 |
| Hypertrophic prostate | 86 | 23 | 22 | 2 | 2 |
| Kidney | 10 | 7 | 3 | 0 | 0 |
| Large intestine | 21 | 8 | 4 | 0 | 1 |
| Liver | 23 | 9 | 6 | 0 | 0 |
| Lung | 24 | 12 | 4 | 0 | 1 |
| Lung (cells) | 57 | 38 | 6 | 0 | 4 |
| Lymph ganglia | 163 | 60 | 23 | 2 | 12 |
| Lymphocytes | 23 | 6 | 4 | 0 | 2 |
| Muscle | 33 | 16 | 6 | 0 | 4 |
| Normal prostate | 181 | 61 | 45 | 7 | 11 |
| Ovary | 90 | 57 | 12 | 1 | 2 |
| Pancreas | 48 | 11 | 6 | 0 | 1 |
| Placenta | 24 | 5 | 1 | 0 | 0 |
| Prostate | 34 | 16 | 4 | 0 | 2 |
| Spleen | 56 | 28 | 10 | 0 | 1 |
| Substantia nigra | 108 | 47 | 27 | 1 | 6 |
| Surrenals | 15 | 3 | 3 | 1 | 0 |
| Testis | 131 | 68 | 25 | 1 | 8 |
| Thyroid | 17 | 8 | 2 | 0 | 2 |
| Umbilical cord | 55 | 17 | 12 | 1 | 3 |
| Uterus | 28 | 15 | 3 | 0 | 2 |
| Non tissue-specific | 568 | 48 | 177 | 2 | 28 |
| Total | 2677 | 947 | 601 | 23 | 150 |

**TABLE VII**

| **Description of Transcription Factor Binding Sites present on promoters isolated from SignalTag sequences** | | | | | |
|---|---|---|---|---|---|
| **Promoter sequence P13H2 (646 bp):** | | | | | |
| **Matrix** | **Position** | **Orientation** | **Score** | **Length** | **Sequence** |
| CMYB_01 | -532 | + | 0.983 | 9 | TGTCAGTTG |
| MYOD_Q6 | -501 | - | 0.961 | 10 | CCCAACTGAC |
| S8_01 | -444 | - | 0.960 | 11 | AATAGAATTAG |
| S8_01 | -425 | + | 0.968 | 11 | AACTAAATTAG |
| OELTAEF1_01 | -390 | - | 0.960 | 11 | GCACACCTCAG |
| GATA_C | -364 | - | 0.964 | 11 | AGATAAATCCA |
| CMYB_01 | -349 | + | 0.958 | 9 | CTTCAGTTG |
| GATA1_02 | -343 | + | 0.959 | 14 | TTGTAGATAGGACA |
| GATA_C | -339 | + | 0.953 | 11 | AGATAGGACAT |
| TAL1ALPHAE47_01 | -235 | + | 0.973 | 16 | CATAACAGATGGTAAG |
| TAL1BETAE47_01 | -235 | + | 0.983 | 16 | CATAACAGATGGTAAG |
| TAL18ETAITF2_01 | -235 | + | 0.978 | 16 | CATAACAGATGGTAAG |
| MYOD_Q6 | -232 | - | 0.954 | 10 | ACCATCTGTT |
| GATA1_04 | -217 | - | 0.953 | 13 | TCAAGATAAAGTA |
| IK1_01 | -126 | + | 0.963 | 13 | AGTTGGGAATTCC |
| IK2_01 | -126 | + | 0.985 | 12 | AGTTGGGAATTC |
| CREL_01 | -123 | + | 0.962 | 10 | TGGGAATTCC |
| GATA1_02 | -96 | + | 0.950 | 14 | TCAGTGATATGGCA |
| SRY_02 | -41 | - | 0.951 | 12 | TAAAACAAAACA |
| E2F_02 | -33 | + | 0.957 | 8 | TTTAGCGC |
| MZF1_01 | -5 | - | 0.975 | 8 | TGAGGGGA |

| **Promoter sequence P16B4 (861bp) :** | | | | | |
|---|---|---|---|---|---|
| **Matrix** | **Position** | **Orientation** | **Score** | **Length** | **Sequence** |
| NFY_Q6 | -748 | - | 0.956 | 11 | GGACCAATCAT |
| MZF1_01 | -738 | + | 0.962 | 8 | CCTGGGGA |
| CMYB_01 | -684 | + | 0.994 | 9 | TGACCGTTG |
| VMYB_02 | -682 | - | 0.985 | 9 | TCCAACGGT |
| STAT_01 | -673 | + | 0.968 | 9 | TTCCTGGAA |
| STAT_01 | -673 | - | 0.951 | 9 | TTCCAGGAA |
| MZF1_01 | -556 | - | 0.956 | 8 | TTGGGGGA |
| IK2_01 | -451 | + | 0.965 | 12 | GAATGGGATTTC |
| MZF1_01 | -424 | + | 0.986 | 8 | AGAGGGGA |
| SRY_02 | -398 | - | 0.955 | 12 | GAAAACAAAACA |
| MZF1_01 | -216 | + | 0.960 | 8 | GAAGGGGA |
| MYOD_Q6 | -190 | + | 0.981 | 10 | AGCATCTGCC |
| DELTAEF1_01 | -176 | + | 0.958 | 11 | TCCCACCTTCC |
| S8_01 | 5 | - | 0.992 | 11 | GAGGCAATTAT |
| MZF1_01 | 16 | - | 0.986 | 8 | AGAGGGGA |

| **Promoter sequence P29B6 (666 bp) :** | | | | | |
|---|---|---|---|---|---|
| **Matrix** | **Position** | **Orientation** | **Score** | **Length** | **Sequence** |
| ARNT_01 | -311 | + | 0.964 | 16 | GGACTCACGTGCTGCT |
| NMYC_01 | -309 | + | 0.965 | 12 | ACTCACGTGCTG |
| USF_01 | -309 | + | 0.985 | 12 | ACTCACGTGCTG |
| USF_01 | -309 | - | 0.985 | 12 | CAGCACGTGAGT |
| NMYC_01 | -309 | - | 0.956 | 12 | CAGCACGTGAGT |
| MYCMAX_02 | -309 | - | 0.972 | 12 | CAGCACGTGAGT |
| USF_C | -307 | + | 0.997 | 8 | TCACGTGC |
| USF_C | -307 | - | 0.991 | 8 | GCACGTGA |
| MZF1_01 | -292 | - | 0.968 | 8 | CATGGGGA |
| ELK1_02 | -105 | + | 0.963 | 14 | CTCTCCGGAAGCCT |
| CETS1P54_01 | -102 | + | 0.974 | 10 | TCCGGAAGCC |
| AP1_04 | -42 | - | 0.963 | 11 | AGTGACTGAAC |
| AP1FJ_Q2 | -42 | - | 0.961 | 11 | AGTGACTGAAC |
| PADS_C | 45 | + | 1.000 | 9 | TGTGGTCTC |

## Claims

1. A signal peptide having the sequence of amino acids -30 to -1 of SEQ ID No: 299.

2. A signal sequence encoding a signal peptide according to claim 1.

3. A signal sequence according to claim 2 having the sequence of nucleotides 115 to 204 of SEQ ID No: 45.

4. A purified and isolated nucleic acid encoding a human secreted polypeptide comprising the signal peptide of claim 1 at the 5'-end of the coding sequence.

5. A nucleic acid according to claim 4, wherein said nucleic acid comprises a signal sequence having the sequence of nucleotides 115 to 204 of SEQ ID No: 45.

6. A nucleic acid according to claim 5, wherein the amino terminus of said polypeptide is encoded by nucleotides 115 to 396 of SEQ ID No: 45.

7. A nucleic acid according to claim 4, wherein said signal sequence is fused in frame to the 5'-end of a sequence encoding a polypeptide that is heterologous to a polypeptide comprising amino acids 1 to 64 of SEQ ID No: 299.

8. An expression vector comprising a signal sequence according to claim 2 or 3 operably linked to a promoter.

9. An expression vector comprising a nucleic acid according to any one of claims 4 to 7 operably linked to a promoter.

10. An expression vector according to claim 8 or 9, wherein said vector is a secretion vector.

11. A polypeptide encoded by a polynucleotide according to any one of claims 4 to 6.

12. A polypeptide according to claim 11, wherein said polypeptide comprises amino acids 1 to 64 of SEQ ID No: 299.

13. A fusion protein encoded by a nucleic acid according to claim 7.

14. Use of a signal peptide according to claim 1 for directing the extracellular secretion of a polypeptide provided that said use is not applied to a human or animal body.

15. Use of a signal peptide according to claim 1 for simplifying protein purification of a desired polypeptide.

16. Use of a vector of any one according to any one of claims 8 to 10 for directing the extracellular secretion of a polypeptide provided that said use is not applied to a human or animal body.

17. Use of a vector according to any one of claims 8 to 10 for simplifying protein purification of a desired polypeptide.

18. Use according to any one of claims 14 to 17, wherein said polypeptide is a polypeptide according to any one of claims 11 to 13.

## Patentansprüche

1. Signalpeptid, welches die Sequenz der Aminosäuren -30 bis -1 der SEQ ID NO:299 aufweist.

2. Signalsequenz, welche für ein Signalpeptid nach Anspruch 1 codiert.

3. Signalsequenz nach Anspruch 2, welche die Sequenz der Nukleotide 115 bis 204 der SEQ ID NO:45 aufweist.

4. Gereinigte und isolierte Nukleinsäure, welche für ein humanes, sekretiertes Polypeptid codiert, welches das Signalpeptid nach Anspruch 1 am 5'-Ende der codierenden Sequenz umfasst.

5. Nukleinsäure nach Anspruch 4, worin die Nukleinsäure eine Signalsequenz umfasst, welche die Sequenz der Nukleotide 115 bis 204 der SEQ ID NO:45 aufweist.

6. Nukleinsäure nach Anspruch 5, worin der Aminoterminus des Polypeptids durch die Nukleotide 115 bis 396 der SEQ ID NO:45 codiert ist.

7. Nukleinsäure nach Anspruch 4, worin die Signalsequenz im Leserahmen mit dem 5'-Ende einer Sequenz verbunden ist, welche für ein Polypeptid codiert, welches heterolog ist im Bezug auf ein Polypeptid, welches die Aminosäuren 1 bis 64 der SEQ ID NO:299 umfasst.

8. Expressionsvektor, umfassend eine Signalsequenz nach Anspruch 2 oder 3, welche in funktionsfähiger Weise mit einem Promoter verbunden ist.

9. Expressionsvektor, umfassend eine Nukleinsäure nach einem der Ansprüche 4 bis 7, welche in funktionsfähiger Weise mit einem Promoter verburiden ist.

10. Expressionvektor nach Anspruch 8 oder 9, worin der Vektor ein Sekretionsvektor ist.

11. Polypeptid, welches von einem Polynukleotid nach einem der Ansprüch 4 bis 6 codiert ist.

12. Polypeptid nach Anspruch 11, worin das Polypeptid die Aminosäuren 1 bis 64 der SEQ ID NO:299 umfasst.

13. Fusionsprotein, welches durch eine Nukleinsäure nach Anspruch 7 codiert ist.

14. Verwendung eines Signalpeptids nach Anspruch 1 zum Steuern der extrazellulären Sekretion eines Polypeptids, mit der Maßgabe, dass die Verwendung nicht an einem menschlichen oder tierischen Körper eingesetzt wird.

15. Verwendung eines Signalpeptids nach Anspruch 1 zum Vereinfachen der Proteinreinigung eines gewünschten Polypeptids.

16. Verwendung eines Vektors nach einem der Ansprüche 8 bis 10 zum Steuern der extrazellulären Sekretion eines Polypeptids, mit der Maßgabe, dass die Verwendung nicht an einem menschlichen oder tierischen Körper eingesetzt wird.

17. Verwendung eines Vektors nach einem der Ansprüche 8 bis 10 zum Vereinfachen der Proteinreinigung eines gewünschten Polypeptids.

18. Verwendung nach einem der Ansprüche 14 bis 17, worin das Polypeptid ein Polypeptid nach einem der Ansprüche 11 bis 13 ist.

## Revendications

1. Peptide signal ayant la séquence en acides aminés de -30 à -1 de SEQ ID No : 299.

2. Séquence signal codant un peptide signal selon la revendication 1.

3. Séquence signal selon la revendication 2, ayant la séquence des nucléotides 115 à 204 de SEQ ID No : 45.

4. Acide nucléique purifié et isolé codant un polypeptide sécrété humain comprenant le peptide signal selon la revendication 1 à l'extrémité 5' de la séquence codante.

5. Acide nucléique selon la revendication 4, comprenant une séquence signal ayant la séquence des nucléotides 115 à 204 de SEQ ID No : 45.

6. Acide nucléique selon la revendication 5, dans lequel la partie amino-terminale dudit polypeptide est codée par les nucléotides 115 à 396 de SEQ ID No : 45.

7. Acide nucléique selon la revendication 4, dans lequel ladite séquence signal est fusionnée en phase à l'extrémité 5' d'une séquence codant un polypeptide qui est hétérologue à un polypeptide comprenant les acides aminés 1 à 64 de SEQ ID No :299.

8. Vecteur d'expression comprenant une séquence signal selon la revendication 2 ou 3, lié de façon opérationnelle à un promoteur.

9. Vecteur d'expression comprenant un acide nucléique selon l'une quelconque des revendications 4 à 7, lié de façon opérationnelle à un promoteur.

10. Vecteur d'expression selon la revendication 8 ou 9, ledit vecteur étant un vecteur de sécrétion.

11. Polypeptide codé par un polynucléotide selon l'une quelconque des revendications 4 à 6.

12. Polypeptide selon la revendication 11, comprenant les acides aminés 1 à 64 de SEQ ID No : 299.

13. Protéine de fusion codée par un acide nucléique selon la revendication 7.

14. Utilisation d'un peptide signal selon la revendication 1, pour diriger la sécrétion extracellulaire d'un polypeptide sachant que ladite utilisation n'est pas appliquée à un humain ou un animal.

15. Utilisation d'un peptide signal selon la revendication 1, pour simplifier la purification protéique d'un polypeptide désiré.

16. Utilisation d'un vecteur selon l'une quelconque des revendications 8 à 10, pour diriger la sécrétion extracellulaire d'un polypeptide sachant que ladite utilisation n'est pas appliquée à un humain ou un animal.

17. Utilisation d'un vecteur selon l'une quelconque des revendications 8 à 10, pour simplifier la purification protéique d'un peptide désiré.

18. Utilisation selon l'une quelconque des revendications 14 à 17, dans laquelle ledit polypeptide est un polypeptide selon l'une quelconque des revendications 11 à 13.
